# EUROPEAN PATENT SPECIFICATION

(11) **EP 1 604 632 B1**
(45) Date of publication and mention of the grant of the patent: **06.06.2007**
(21) Application number: 05105007.8
(22) Date of filing: 08.06.2005
(51) Int. Cl.: A61K 8/81, A61K 8/89, A61Q 1/02

(54) **Cosmetic comprising a specific terpolymer**
Kosmetische Zusammensetzung enthaltend ein spezifisches Terpolymer
Composition cosmétique comprenant un terpolymère spécifique

(30) Priority: 08.06.2004 JP 2004170207; 08.06.2004 JP 2004170209; 08.06.2004 JP 2004170210; 08.06.2004 JP 2004170211
(43) Date of publication of application: 14.12.2005
(73) Proprietor: Shiseido Co., Ltd., Tokyo 104-8010 (JP)
(72) Inventor: Yoshida, Kunihiko, Hiroshima 734-0005 (JP); Kakoki, Hiroyuki Shiseido Res.Center-Shin-Yokohama, 224-8558, Kanagawa (JP); Mori, Atsumi, c/o Shiseido Sales Co., Fukuoka-shi, Fukuoka 810-8670 (JP)
(74) Representative: Henkel, Feiler & Hänzel

(56) References cited:
- US-A1- 2002 076 390
- DATABASE WPI Section Ch, Week 199304 Derwent Publications Ltd., London, GB; Class A14, AN 1993-032169 XP002343896 & JP 04 359912 A (KAO CORP) 14 December 1992 (1992-12-14)
- DATABASE WPI Section Ch, Week 200464 Derwent Publications Ltd., London, GB; Class A14, AN 2004-656551 XP002343897 & JP 2004 250359 A (DAITO KASEI KOGYO KK) 9 September 2004 (2004-09-09)

## Description

### RELATED APPLICATIONS

This application claims a priority of Japanese Patent Application No. 2004-170207, filed on June 8, 2004, Japanese Patent Application No. 2004-170208, filed on June 8, 2004, Japanese Patent Application No. 2004-170210, filed on June 8, 2004, and Japanese Patent Application No. 2004-170211, filed on June 8, 2004, which is incorporated herein by reference.

### BACKGOUND OF THE INVENTION

### FIELD OF THE INVENTION

The invention relates to a cosmetic, in particular, relates to improvements of retaining performance and film-line feeling of makeup cosmetic for lips, eyelashes and skin, and improvements of emulsion stability and dispersibility of powder in water-in-oil emulsion cosmetic.

### PRIOR ART

Conventional oil-based lipsticks have been composed of various oils, waxes and color materials with good luster upon application on the lips, while it was a problem that the makeup retaining performance is poor for instance, the color is transferred to a coffee cup or clothes and the color faded out with the passage of time. Various researches have been carried out for improving such problems of makeup retaining performance, and lipsticks for example, using various silicones such as alkylmethyl polisiloxane, polyoxyalkylene-modified organopolysiloxane and trimethyl siloxysilicate and the like, and non-aqueous polymer dispersion as film-forming components are proposed. (see, for example, Japanese Unexamined Patent Publication (JP-A) Nos. 5-178722, 6-298623, 7-33622, 8-225432 and 10-59827, and Japanese Examined Patent Publication (JP-B) No. 61-12884).

However, these lipsticks, in which such film-forming components are blended, exhibit a luster function and makeup retaining function to some extent, while the feeling of use was of problem since film-like feeling remains on the lips due to film-forming components in the material. When the amount of blending of the film-forming components was reduced, on the other hand, an effect for improving makeup retaining performance was unsatisfactory. Accordingly, it was quite difficult to permit both of these functions to be compatible with each other.

Cosmetics for eyelashes represented by mascara are expected to have such effects as pretending eyelashes to be thick and long and affording eyelashes massive feeling, as well as a function for makeup retaining (water-resistance and skin fat resistance), a curling effect (a quick drying effect and an effect for curling the eyelashes upward), and a curl-retaining effect (sustained curling effect with time). Natural latexes and water-soluble polymers, and polymer emulsions have been blended as film-forming components, in order to improve the curling effect and makeup retaining performance of the cosmetics for eyelashes. However, water resistance of these film-forming components was so insufficient that they were readily removed by water, sweat and tears and the like. While solid oils such as solid paraffin, waxes and lanolin derivatives have been frequently blended, the blended component is excellent in water-resistance, but it is readily dissolved in oily fractions such as skin fat and other oil-based cosmetics.

For solving the problems described above, makeup cosmetics comprising blended organic silicone resins as the film-forming component have been developed in recent years (for example, see JP-A Nos. 61-18708, 61-65809 and 61-161211). Since the organic silicone resin is quite insoluble in the skin fat and oily fractions that are usually blended in cosmetics while the resin forms a tough film after drying, the cosmetics for eyelashes being excellent in both of the curling effect and makeup retaining performance may be obtained. However, the although cosmetics for eyelashes using the organic silicone resin has an excellent function with respect to the curling effect and makeup retaining performance to some extent, the feeling of use was of problem since the cosmetic gave a remarkable film-like feeling are to the organic silicone resin as the film-forming component. When the amount of blending of the organic silicone resin is reduced, on the other hand, the curling effect and makeup retaining effect cannot be sufficiently obtained although the film-like feeling has been improved. Accordingly, it was quite difficult to permit both of these functions to be compatible.

Meanwhile, it is desired to enhance a so-called makeup retaining effect by preventing makeup from coming off by the sweat and skin fat or with time, or to prevent the cosmetics from adhering on the cloths, in the development of the makeup cosmetics such as foundations. In various efforts for this purpose, a silicone resin is blended, for example, in the makeup cosmetics. Since the silicone resin is quite insoluble in water and oily components such as skin fat and forms a tough coating film after drying, blending the resin affords the makeup cosmetics excellent in makeup retaining performance. However, the feeling of use was not so good due to remarkable film-like feeling ascribed to the silicone resin film, although the makeup cosmetics using the silicone resin as the film-forming component has an excellent function with respect to makeup retaining performance.

Recently, makeup cosmetics using silicone derivatives of polysaccharides represented by siliconated pullulane as the film-forming component have been proposed (for example, see JP-A No. 10-29910). However, film-like feeling is caused on the skin with unfavorable feeling of use even by using siliconated polysaccharide as the film-forming component, when the compound of the amount sufficient for obtaining the makeup sustaining effect is blended in the base material of the cosmetics. While the film-like feeling is improved by reducing the amount of blending of the film-forming component, a sufficient effect for sustaining makeup cannot be attained. Accordingly, it was a quite difficult problem to permit both of these functions to be compatible.

On the other hand, a water-in-oil emulsion having an oil phase as an outer phase and a water phase as an inner phase has been used in various cosmetics. Such water-in-oil emulsion is superior to oil-in-water emulsions in protection of the skin, flexing effect on the skin and suppression of evaporation of water from the skin, and is considered to be a formulation suitable for an external use agent for the skin. Examples of emulsifying agents for forming the water-in-oil emulsion that have been used include hydrophobic emulsifying agents having a HLB value of 1 to 12 such as polyhydric alcohol fatty acid ester surfactants such as glycerin fatty acid esters and sorbitan fatty acid esters, and polyoxyalkylene modified organopolysiloxane surfactants.

However, since the water-in-oil emulsion using such emulsifying agent is poor in stability of the emulsion and the water phase is separated from the oil phase at high temperatures or upon long period of time, it was quite difficult to stabilize the formulation. While the formulation may be stabilized by blending a wax in the oil phase as the outer phase, the preparation is still unstable since the wax is melted or softened at high temperatures. Therefore, the formulation is not sufficiently stable with additional problems in use such as it is hardly extendable and becomes sticky on application.

Water-in-oil makeup external formulations for the skin such as the foundation and eye liner are required to blend a large amount of a pigment powder. However, it is difficult to uniformly disperse a powder using a conventionally used surfactant, when a large amount of the powder is blended in the base material of the water-in-oil emulsion. The feeling of use was remarkably impaired due to aggregation of the powder in the base material.

### SUMMARY OF THE INVENTION

Accordingly, this invention performed with reference to the foregoing problems, the first object of the invention is to provide a cosmetic having excellent retaining performance and film-like feeling. Also, the second object of the invention is to provide an oil-in-water emulsion cosmetic having excellent stability of the emulsion and dispersibility of powder.

The inventors of the invention have found, through intensive studies for solving the aforementioned problems, that the cosmetic become quite excellent in makeup retaining performance while film-like feeling is remarkably improved by blending a copolymer comprising a specific acrylic monomer, a specific polyoxyalkylene group containing monomer, and a specific organopolysiloxane containing monomer in a specified proportion as the film-forming component, as compared with cosmetics using conventional film-forming components,. The inventors of the invention have also found that the stability of the emulsion and the dispersibility of large amount of the powder blended in the water-in-oil cosmetic become excellent by blending a copolymer comprising a specific acrylic monomer, a specific polyoxyalkylene containing monomer and a specific organosiloxane containing monomer in a specified proportion as the emulsifying agent, as compared with the cosmetics using conventional water-in-oil emulsifying agent. The invention has been completed based on the discoveries as described above.

A first aspect of the invention is to provide a cosmetic comprising one or more of copolymer which comprises monomer (A) represented by formula (1), monomer (B) represented by formula (2) and monomer (C) represented by formula (3) as constituting monomers, wherein the content of monomer (A) is 20% by mass or more "the content of monomer (B) is 0.1 to 50% by mass, the content of monomer (C) is 30 to 80% by mass, each" relative to the total amount of the constituting monomers. 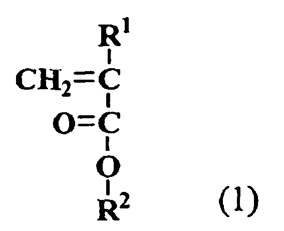 where, R¹ is hydrogen or a hydrocarbon group having 1 to 3 of carbon atoms, and R² is hydrogen or a hydrocarbon group having 1 to 24 of carbon atoms. 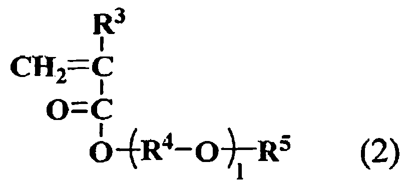 where, R³ is hydrogen or a hydrocarbon group having 1 to 3 of carbon atoms, R⁴ is a divalent hydrocarbon group having 1 to 4 of carbon atoms, R⁵ is a hydrocarbon group having 1 to 24 of carbon atoms, and 1 is an integer of 1 to 50. 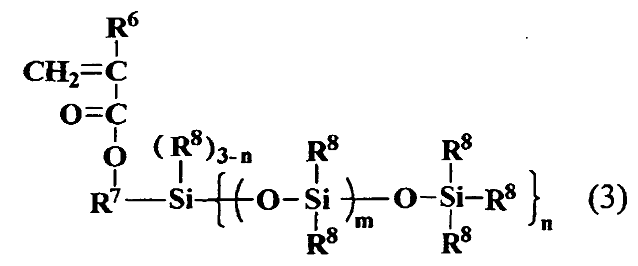 where, R⁶ is hydrogen or a hydrocarbon group having 1 to 3 of carbon atoms, R⁷ is a divalent hydrocarbon group having 1 to 4 of carbon atoms, R⁸ is a hydrocarbon group 1 to 6 of carbon atoms, m is an integers of 0 to 500, and n is an integers of 1 to 3.

Preferably, the cosmetic is a lip makeup cosmetic.

Preferably, the lip makeup cosmetic further comprises one or more of volatile oil components selected from a linear silicone oil represented by formula (4), a cyclic silicone oil represented by formula (5), an alkyl-modified silicone oil represented by formula (6), and isoparaffin. 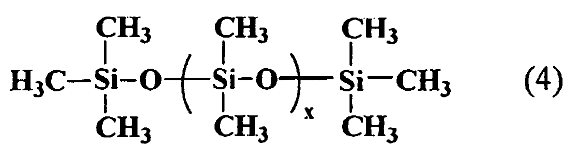 where, x is an integer of 0 to 3. 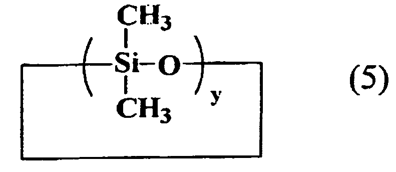 where, y is an integer of 3 to 6. 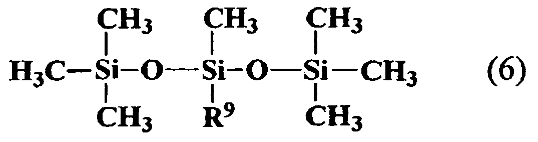 where, R⁹ is a hydrocarbon group having 2 to 8 of carbon atoms.

Preferably, the lip makeup cosmetic further comprises one or more of non-volatile component selected from a fluorine-modified dimethylsilicone represented by formula (7), a fluorine-modified phenylsilicone represented by formula (8), an alkoxy-modified silicone represented by formula (9), an alkyl-modified silicone represented by formula (10), tri(hydrogenated rosin-isostearic acid)glyceryl, and 2-ethylhexyl paramethoxycinnamate. 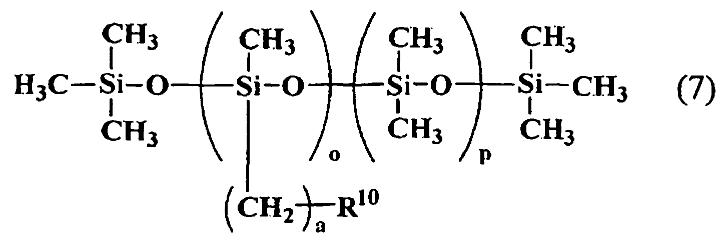 where, o and p are average values, o is an integer of 1 to 150, p is an integer of 0 to 150, the sum of o and p is 4 or more, a is an integer of 0 to 10, and R¹⁰ is a perfluoroalkyl group having 1 to 12 of carbon atoms. 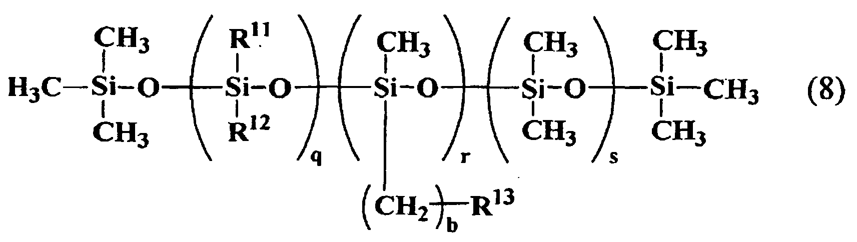 where, q, r and s are average values, q is an integer of 1 to 150, r is an integer of 1 to 150, s is an integer of 0 to 150, the sum of q, r and s is 4 or more, b is an integer of 0 to 10, R¹¹ and R¹² is a methyl or phenyl group with at least one of R¹¹ and R¹²is the phenyl group, and R¹³ is a perfluoroalkyl group having 1 to 12 of carbon atoms. 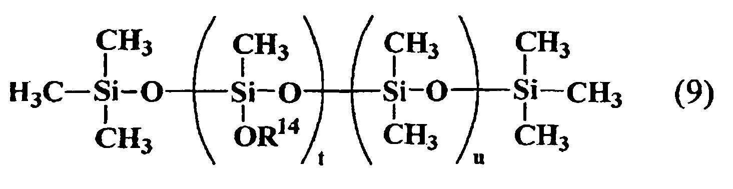 where, OR¹⁴ is an alkoxy group having 2 to 30 of carbon atoms, t and u are average values, t is an integer of 1 to 500, u is an integer of 0 to 500, and the sum of t and u is 4 or more. 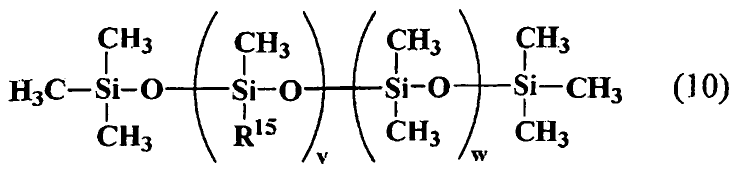 where, R¹⁵ is an alkyl group having 10 to 30 of carbon atoms, v and w are average values, v is an integer of 1 to 500, w is an integer of 0 to 500, and the sum of v and w is 4 or more.

Preferably, the lip makeup cosmetic further comprises water or a humectant.

Preferably, the cosmetic is an eyelashes makeup cosmetic.

Preferably, the eyelashes makeup cosmetic is a water-in-oil eyelashes makeup cosmetic comprising the copolymer and an oil ingredient in the outer phase and water in the inner phase.

Preferably, the water-in-oil eyelashes makeup cosmetic comprises 1 to 30% of the copolymer and volatile silicone oil and/or hydrocarbon oil in the outer phase, and water and a film-forming emulsion resin in the inner phase.

Preferably, the eyelashes makeup cosmetic is an oil-in-water eyelashes makeup cosmetic comprising the copolymer and an oil ingredient in the inner phase, and water in the outer phase.

Preferably, the oil-in-water eyelashes cosmetic comprises 1 to 30% of the copolymer and a volatile silicone oil and/or hydrocarbon oil in the inner phase, and water and a film-forming emulsion resin in the outer phase.

Preferably, the eyelashes makeup cosmetic is an oil-based eyelashes makeup cosmetic comprises the copolymer and a wax.

Preferably, the oil-based eyelashes makeup cosmetic comprises 1 to 30% of the polymer, a wax, a volatile silicone oil and/or hydrocarbon oil and a viscosity improving agent.

Preferably, the oil-based eyelashes makeup cosmetic further comprises a hollow particle.

Preferably, the makeup cosmetic is a skin makeup cosmetic.

Preferably, the skin makeup cosmetic further comprises siliconated polysaccharide represented by formula (11). 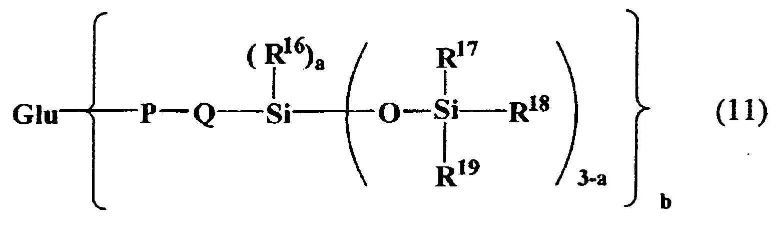 where, Glu is a sugar residue of the polysaccharide, P is a divalent binding group, Q represents a divalent aliphatic group, R¹⁶ is a hydrocarbon group having 1 to 8 of carbon atoms, and R¹⁷, R¹⁸ and R¹⁹ is hydrocarbon groups having 1 to 8 of carbon atoms or siloxy groups resented by -OSiR²⁰R²¹R²², wherein R²⁰, R²¹ and R²² are hydrocarbon group having 1 to 8 of carbon atoms, a is an integer of 0 to 2, and b is a positive integer.

Preferably, the siliconated polysaccharide is siliconated pullulane represented by formula (12). 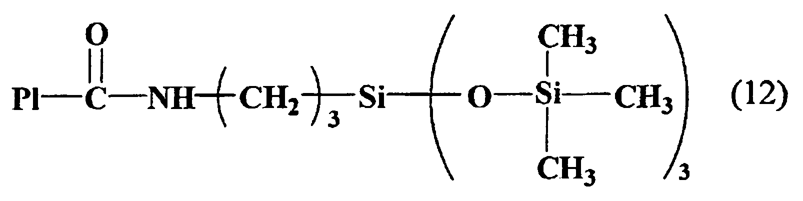 where, Pl is a glucose residue of pullulane.

Preferably, the cosmetic is a water-in-oil emulsion cosmetic.

Preferably, the water-in-oil emulsion cosmetic further comprises a water-swelling clay mineral and quaternary ammonium cation surfactant.

Preferably, the water-in-oil emulsion cosmetic further comprises powder.

Preferably, the water-in-oil emulsion cosmetic further comprises a nonionic surfactant.

Preferably, the water-in-oil emulsion cosmetic further comprises volatile silicone.

Preferably, the water-in-oil emulsion cosmetic further comprises a UV absorbing agent and/or a UV scattering agent.

### DESCRIPTION OF THE PREFERRED EMBODIMENTS

Preferable embodiments of the invention will be described in detail hereinafter.

The copolymer used in the cosmetic according to the invention comprises monomer (A) represented by formula (1), monomer (B) represented by formula (2) and monomer (C) represented by formula (3) as constituting monomers, wherein the content of monomer (A) is 20% by mass or more "the content of monomer (B) is 0.1 to 50% by mass, the content of monomer (C) is 30 to 80% by mass, each" relative to the total amount of the constituting monomers. 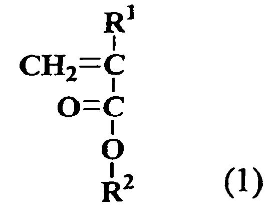 where, R¹ is hydrogen or a hydrocarbon group having 1 to 3 of carbon atoms, and R² is hydrogen or a hydrocarbon group having 1 to 24 of carbon atoms. 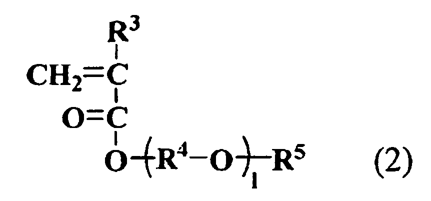 where, R³ is hydrogen or a hydrocarbon group having 1 to 3 of carbon atoms, R⁴ is a divalent hydrocarbon group having 1 to 4 of carbon atoms, R⁵ is a hydrocarbon group having 1 to 24 of carbon atoms, and 1 is an integer of 1 to 50. 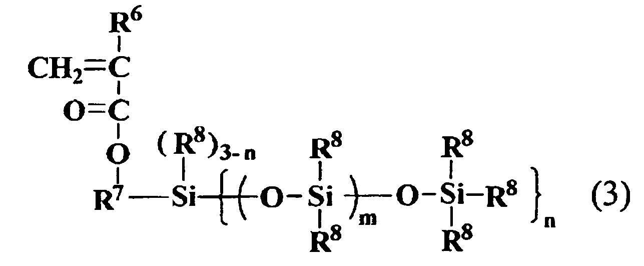 where, R⁶ is hydrogen or a hydrocarbon group having 1 to 3 of carbon atoms, R⁷ is a divalent hydrocarbon group having 1 to 4 of carbon atoms, R⁸ is a hydrocarbon group 1 to 6 of carbon atoms, m is an integers of 0 to 500, and n is an integers of 1 to 3.

Monomer (A) represented by formula (1) is alkyl-substituted acrylic acid such as acrylic acid or methacrylic acid or an alkyl ester thereof. R¹ as the substituent of α-carbon of acrylic acid in formula (1) is hydrogen or a hydrocarbon group having 1 to 3 of carbon atoms. Examples of the hydrocarbon group include an alkyl group and alkenyl group and the like, which may be either linear or branched. Hydrogen and methyl group are particularly preferable as R¹, R² as a terminal substituent in formula (1) is hydrogen or a hydrocarbon group with a having 1 to 24 of carbon atoms. Examples of the hydrocarbon group include an alkyl group and an alkenyl group and the like, which may be either linear or branched, and a part of hydroxyl groups or fluorine atoms may be substituted. Examples of R² include a hydrogen atom, a methyl group, an ethyl group, a propyl group, a butyl group, a hexyl group, a 2-ethylhexyl group, a decyl group, a tridecyl group, a stearyl group, an isostearyl group, a 2-hydroxyethyl group, a 2-hydroxypropyl group, a trifluoromethyl group and a trifluoroethyl group. The hydrogen atom, methyl group, butyl group, 2-ethylhexyl group, tridecyl group, stearyl group and isostearyl group are particularly preferable as R².

Examples of monomer (A) used in the invention include acrylic acid; alkyl esters of acrylic acid such as methyl acrylate, ethyl acrylate, butyl acrylate, hexyl acrylate, 2-ethylhexyl acrylate, octyl acrylate, decyl acrylate, lauryl acrylate and stearyl acrylate; methacrylic acid; and alkyl esters of methacrylic acid such as methyl methacrylate, ethyl methacrylate, butyl methacrylate, hexyl methacrylate, 2-ethylhexyl methacrylate, octyl methacrylate, decyl methacrylate, dodecyl methacrylate, tridecyl methacrylate, stearyl methacrylate and isostearyl methacrylate and the like..

One or more of monomer (A) may be used as the constituting monomer of the copolymer used in the invention.

The content of monomer (A) in the copolymer used in the invention is 20% by mass or more relative to the total content of the constituting monomers. When the content of monomer (A) is less than 20% by mass of the total content of the constituting monomers, the film-forming ability of the copolymer may be reduced in case of the copolymer is used as the film-forming agent, and the stability of the emulsion may be poor in case of the copolymer is used as the water-in-oil emulsifying agent. The content of monomer (A) is preferably 20 to 60% by mass, particularly 25 to 50% by mass.

Monomer (B) represented by formula (2) is a compound having an oxyalkylene moiety in acrylic acid or an alkyl-substituted acrylic acid such as methacrylic acid. For example, the compound is an alkyleneglycol ester acrylic acid or alkyl-substituted acrylic acid or alkyl ether thereof. R³ as a substituent of α-carbon of acrylic acid in formula (2) is hydrogen or a hydrocarbon group having 1 to 3 of carbon atoms. Examples of the hydrocarbon group include an alkyl group or alkenyl group and the like, which may be either linear or branched. Hydrogen or a methyl group is particularly preferable as R³. R⁴ as a hydrocarbon moiety in the oxyalkylene group in formula (2) is a divalent hydrocarbon group having 1 to 4 of carbon atoms. Examples of the divalent hydrocarbon group include an alkylene group, which may be either linear or branched. R⁴ may be the same or different, for example the oxyalkylene moiety may have a block or randomly repeating structure comprising two or more oxyalkylene groups includinig R⁴ having different carbon numbers. However, an ethylene group is particularly preferable as R⁴. R⁵ in formula (2) as a terminal substituent of the oxyalkylene group is hydrogen or a hydrocarbon group having 1 to 24 of carbon atoms. Examples of the hydrocarbon group include an alkyl group or alkenyl group and the like, which may be either linear or branched. The letter 1 representing the repeating units of oxyalkylene group in formula (2) is an integer of 1 to 50. Hydrogen or a methyl group is particularly preferable as R⁵. In particular, the letter 1 preferably is 1 to 20.

Examples of monomer (B) used in the invention include,
CH₂=C(CH₃)COO(C₂H₄O)₆H, CH₂=C(CH₃)COO(C₂H₄O)₈H, CH₂=C(CH₃)COO(C₂H₄O)₁₀H,
CH₂=C(CH₃)COO(C₂H₄O)₁₅H, CH₂=C(CH₃)COO(C₂H₄O)₆CH₃,
CH₂=C(CH₃)COO(C₂H₄O)₈CH₃, CH₂=C(CH₃)COO(C₂H₄O)₁₀CH₃,
CH₂=C(CH₃)COO(C₂H₄O)₁₅C₄H₉, CH₂=CHCOO(C₂H₄O)₆H, CH₂=CHCOO(C₂H₄O)₈H,
CH₂=CHCOO(C₂H₄O)₁₀H, CH₂=CHCOO(C₂H₄O)₁₅H, CH₂=CHCOO(C₂H₄O)₆CH₃,
CH₂=CHCOO(C₂H₄O)₈CH₃, CH₂=CHCOO(C₂H₄O)₁₀CH₃, CH₂=CHCOO(C₂H₄O)₁₅C₄H₉,
CH₂=CHCOO(C₂H₄O)₁₀(C₃H₆O)₁₀C₁₂H₂₅, and CH₂=°C(CH₃)COO(C₂H₄O)₃₀(C₃H₆O)₁₀C₁₈H₃₇ and the like.

One or more of monomer (B) may be used as the constituting monomer in the copolymer used in the invention.

The content of monomer (B) in the copolymer used in the invention is in a range of from 0.1 to 50% by mass, preferably 1 to 40% by mass relative to the total content of the constituting monomers when the copolymer is used as a film-coating agent. The film-like feeling may be deteriorated when the content of monomer (B) is less than 0.1 % by mass of the total content of the constituting monomers, while the film-forming ability may be lowered when the content is more than 50% by mass. The content of monomer (B) is preferably 2 to 30% by mass, particularly 5 to 20% by mass, relative to the total content of the constituting monomers when the copolymer is used as a water-in-oil emulsifying agent. An emulsion may not be obtained when the content of monomer (B) is less than 2% by mass of the total content of the constituting monomers, while the emulsion may be an oil-in-water emulsion composition when the content is more than 30% by mass.

Monomer (C) represented by formula (3) is a compound having an organopolysiloxane moiety in acrylic acid or an alkyl-substituted acrylic acid such as methacrylic acid, for example an ester prepared by linking organopolysiloxane to acrylic acid or alkyl-substituted acrylic acid via a divalent hydrocarbon group. R⁶ representing the substituent at α-carbon of acrylic acid in formula (3) is hydrogen or a hydrocarbon group having 1 to 3 of carbon atoms. Examples of the hydrocarbon group include the alkyl group and alkenyl group and the like, which may be either linear or branched. In particular, R⁶ is preferably hydrocarbon and methyl group. R⁷, which is a hydrogen group for linking the acrylic acid moiety to the organopolysiloxane moiety, in formula (3) is a divalent hydrocarbon group having 1 to 4 of carbon atoms. Examples of the divalent hydrocarbon group include an alkylene group, which may be either linear or branched. Particularly, R⁷ is preferably a propylene group. R⁸, which is a side chain substituent of organopolysiloxane group, in formula (3) is a hydrocarbon group having 1 to 6 of carbon atoms. Examples of the hydrocarbon group include the alkyl group, alkenyl group or fluoroalkyl group and the like, which may be either linear or branched. R⁸ may be the same or different, and may be, for example, a hydrocarbon group in which only a part of terminal R⁸ has a different carbon number. In particular, R⁸ is preferably a methyl group or a phenyl group or a trifuloropropyl group. The letter m representing the repeating unit of the organosiloxane group in formula (3) is an integer of 0 to 500. In particular, m preferably is 0 to 200. The letter n representing the number of substituents of organosiloxane group in formula (3) is an integer of 1 to 3.

Examples of monomer (C• used in the invention include
CH₂=C(CH₃)COOC₃H₆Si[OSi(CH₃)₃]₃, CH₂=C(CH₃)COOC₃H₆[Si(CH₃)₂O]₂₀Si(CH₃)₃,
CH₂=C(CH₃)COOC₃H₆[Si(CH₃)₂O]₄₀Si(CH₃)₂C₄H₉,
CH₂=C(CH₃)COOC₃H₆[Si(CH₃)₂O]₁₀₀Si(CH₃)₂C₄H₉,
CH₂=C(CH₃)COOC₃H₆[Si(CH₃)₂O]₁₀₀[Si(C₆H₅)₂O]₂₀Si(CH₃)₂C₄H₉, and
CH₂=C(CH₃)COOC₃H₆[Si(CH₃)₂O]₁₅₀[Si(CH₃)(C₂H₄CF₃)O]₅₀Si(CH₃)₂C₄H₉ and the like..

One or more of monomer (C) may be used as constituting monomers in the copolymer used in the invention.

The content of monomer (C) is in the copolymer used in the invention, in a range of from 30 to 80% by mass relative to the total content of constituting monomers. Compatibility of the copolymer with silicone components may be poor in the preparation when the content of monomer (C) is less than 30% by mass of the total amount of the constituting monomers. When the contents is more than 80% by mass, the film-forming ability may be lowered in case of the copolymer is used as a film-forming material, and the stability of the emulsion may be poor in case of the copolymer is used as an emulsifying agent. It is particularly preferable that the content of monomer (C) is in the range of 40 to 70% by mass.

Monomers other than monomers (A) to (C) may be added as constituting monomers in a range not impairing the effect of the invention. The content of other monomers may be in the range of 50% by mass or less, for example in the range of 0.0001 to 10% by mass, of the total amount of the constituting monomers. Examples of such monomers include ethylene, propylene, butadiene, styrene, vinyl chloride, vinylidene chloride, vinyl alcohol, vinyl acetate, maleic anhydride, acrylonitrile, acrylamide, methacrylamide, methyl acrylamide, methyl methacrylamide, dimethyl methacrylamide, ethyl acrylamide, ethyl methacrylamide, diethyl methacrylamide, N-isopropyl acrylamide, N-vinyl pyrrolidone, ε-caprolactam, N,N'-dimethylaminoethyl methacrylic acid, diallyldimethyl ammonium chloride and 2-acrylamide-2-methylpropane sodium sulfonate.

The copolymer used in the invention can be obtained by a well-known polymerization method using monomers (A) to (C). Examples of the polymerization method available include homogeneous solution polymerization, heterogeneous solution polymerization, emulsion polymerization, reversed phase emulsion polymerization, bulk polymerization, suspension polymerization and precipitation polymerization. In the case of homogeneous solution polymerization, for example, monomers (A) to (C) are dissolved in a solvent with an appropriate monomer proportion, and the solution is stirred with heating under a nitrogen atmosphere by adding a radical polymerization initiator to obtain the copolymer of the invention. The copolymer may be also obtained by a post modification method using polyacrylic acid in which appropriate functional groups are added after polymerization.

Any solvents may be used for polymerization so long as the solvent is able to dissolve or suspend the monomers. Examples of the solvent include alcohol solvents such as methanol, ethanol, propanol, isopropanol and butanol; hydrocarbon solvents such as hexane, haptane, octane, isooctane, decane and liquid paraffin; ether solvents such as dimethylether, diethylether and tetrahydrofuran; ketone solvents such as acetone and methylethyl ketone; ester solvents such as methyl acetate, ethyl acetate and butyl acetate; chloride solvents such as methylene chloride, chloroform and carbon tetrachloride as well as dimethylformamide, diethylformamide, dimethylsulfoxide and dioxane. Two or more of these may be used by mixing. It is preferable to select the solvents having a higher boiling point than the polymerization initiation temperature of the polymerization initiator used.

The polymerization initiator is not particularly restricted so long as it has ability for initiating radical polymerization. Examples of the polymerization initiator include peroxide such as benzoyl peroxde, azo compounds such as azobisisobutylonitrile (AIBN) and 2,2'-azobis(isobutylic acid)dimethyl, as well as persulfate polymerization initiators such as potassium persulfate and ammonium persulfate. It is possible to polymerize by photochemical reactions and radiation without depending on these polymerization initiators. The polymerization temperature is above the polymerization initiation temperature of the polymerization initiator. For example, the polymerization temperature is usually about 70°C when the peroxide polymerization initiator is used.

While the polymerization time is not particularly restricted, it is usually 2 to 24 hours. The reaction time is desirably one day or more when a polymer having a relatively high molecular weight is to be obtained. Unreacted monomer may be left behind when the reaction time is too short while the molecular weight is reduced. While the average molecular weight of the copolymer used in the invention is not particularly restricted, an average molecular weight of 10,000 to 200,000 is particularly preferable. The order of addition of monomers (A) to (C) is not particularly defined in the copolymer molecule used in the invention, and addition of the monomers may be block-wise or random. However, the copolymer obtained comprises randomly added monomers (A) to (C).

A representative example of the copolymer used in the invention is shown by formula (13) as below:

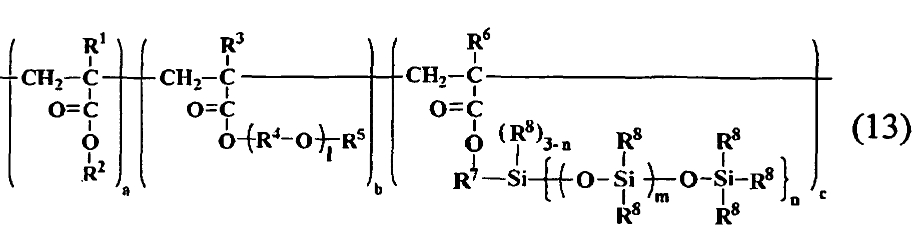

R¹ to R⁸,1, m and n in formula (13) have been defined above. a, b and c represent the proportion by mass of monomers (A), (B) and (C) in the total amount of the constituting monomers, respectively. In formula (13), the proportion by mass of monomer (A) represented by a is adjusted to be 20% by mass or more relative to the total amount of the constituting monomers.

The amount of the copolymer in the cosmetic according to the invention is not particularly restricted, and the copolymer may be used by appropriately controlling the amount depending on the object of use. However, the amount is preferably 0.0 1 to 30% by mass, more preferably 0.2 to 25% by mass, relative to the total amount of the composition. The effect by blending the copolymer may not be obtained when the amount of the copolymer is less than 0.01% by mass, while usability of the cosmetic become worse when the amount is more than 30% by mass.

Film-like feeling by the cosmetic of the invention may be improved while makeup retaining performance is improved by blending the copolymer produced as described above in the cosmetic as a film-forming component.

### Lip makeup cosmetic

Film-like feeling on the lip makeup cosmetic may be improved while makeup retaining performance on the lips is improved, by particularly blending the copolymer produced as described above with the lip makeup cosmetic.

The amount of blending of the copolymer in the lip makeup cosmetic according to the invention is not particularly restricted, and it may be used by preparing in an appropriate amount of blending depending on the object of use. However, the amount is preferably 0.1 to 20% by mass, more preferably 0.5 to 15% by mass, relative to the total amount of the composition. The effect for improving retaining performance may not be exhibited when the amount of blending of the copolymer is less than 0. 1% by mass, while film-like feeling on the lips becomes evident when the amount is more than 20% by mass.

Components usually used for cosmetic and medicines may be blended in the lip makeup cosmetic according to the invention in a range not impairing the effect of the invention, in addition to the copolymer that is an essential component.

Volatile oil components may be blended with the copolymer in the lip makeup cosmetic according to the invention. Examples of the volatile oil component used in the invention include a linear silicone oil represented by formula (4) below, a cyclic silicone oil represented by formula (5) below, an alkyl-modified silicone oil represented by formula (6) below, and isoparaffin. 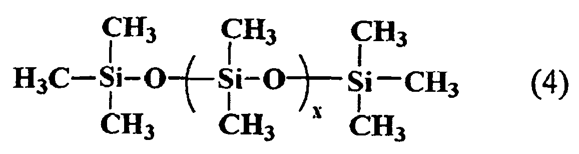 where, x is an integer of 0 to 3. 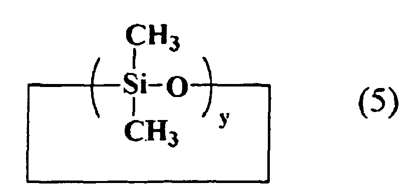 where, y is an integer of 3 to 6. 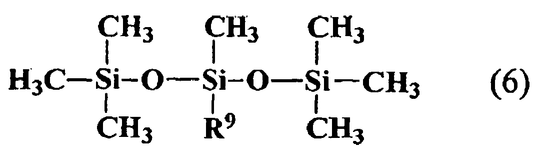 where, R⁹ is a hydrocarbon group having 2 to 8 of carbon atoms.

One or more of the volatile oil components above may be selected for blending with the lip makeup cosmetic according to the invention. While the amount of blending of the volatile oil component is not particularly restricted, it is preferably 10 to 90% by mass, more preferably 20 to 80% by mass, in the composition. Makeup retaining performance may be decreased when the amount of blending of the volatile oil component is too small, while stability of the shape of the lipstick may be deteriorated when the amount is too large.

Non-volatile oil components may be favorably blended in the lip makeup cosmetic according to the invention. Examples of the non-volatile oil component used in the invention include fluorine-modified dimethyl silicone represented by formula (7) below, fluorine-modified phenyl silicone represented by formula (8) below, alkoxy-modified silicone represented by formula (9) below, alkyl-modified silicone represented by formula (10) below, tri(Hydrogenated rosin-isostearic acid)glyceryl, and 2-ethylhexyl paramethoxycinnamate. 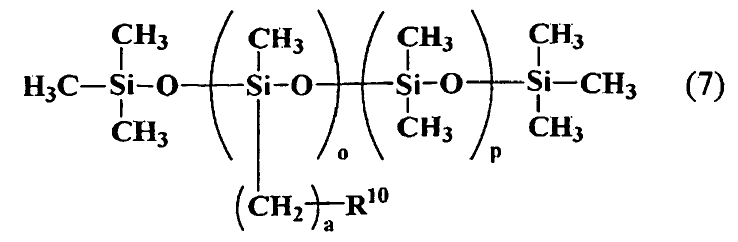 where, o and p are average values, o is an integer of 1 to 150, p is an integer of 0 to 150, the sum of o and p is 4 or more, a is an integer of 0 to 10, and R¹⁰ is a perfluoroalkyl group having 1 to 12 of carbon atoms. 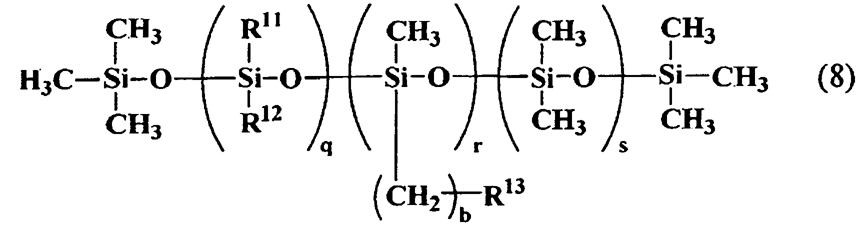 where, q, r and s are average values, q is an integer of 1 to 150, r is an integer of 1 to 150, s is an integer of 0 to 150, the sum of q, r and s is 4 or more, b is an integer of 0 to 10, R¹¹ and R¹² is a methyl or phenyl group with at least one of R¹¹ and R¹²is the phenyl group, and R¹³ is a perfluoroalkyl group having 1 to 12 of carbon atoms. 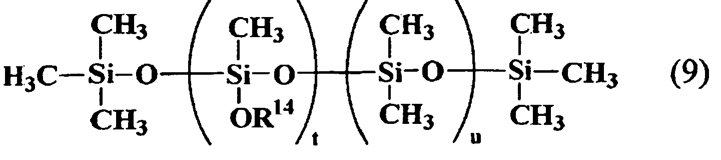 where, OR¹⁴ is an alkoxy group having 2 to 30 of carbon atoms, t and u are average values, t is an integer of 1 to 500, u is an integer of 0 to 500, and the sum of t and u is 4 or more. 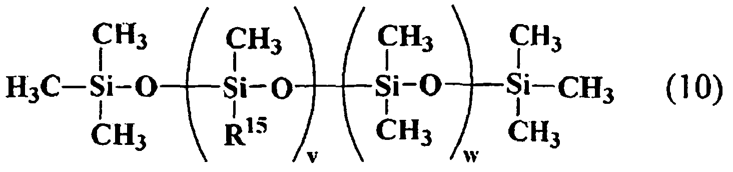 where, R¹⁵ is an alkyl group having 10 to 30 of carbon atoms, v and w are average values, v is an integer of 1 to 500, w is an integer of 0 to 500, and the sum of v and w is 4 or more.

One or more of the non-volatile oil component may be selected for blending in the lip makeup cosmetic according to the invention. The amount of blending of the non-volatile oil component is preferably 0.1 to 50% by mass, more preferably 0.5 to 40% by mass, in the total composition. Feeling of use may be poor when the amount of blending of the non-volatile oil component is too small, while makeup retaining performance may be deteriorated when the amount is too large.

Water or humectants may be favorably blended in the lip makeup cosmetic according to the invention. Examples of the humectant used in the invention include ethyleneglycol, diethyleneglycol, 1,3-butyleneglycol, glycerin, hexamethyleneglycol, isopreneglycol, polyethyleneglycol, hyaluronic acid, chondroitin sulfate, chitin, chitosane, xylitol, sorbitol, multitol, mucoitin sulfate, charonin sulfate, atherocollagen, ehoresteryl-12-hydroxy stearate, sodium lactate, dl-pyrrolidone carbonate, short chain soluble collagen, diglycerin (EO)PO adduct, Chestnut Rose extract, Achillea millefolium extract, merillot extract and dipropylene glycol.

One or more of the humectants may be selected for blending in the lip makeup cosmetic according to the invention. The amount of blending of water or humectant is preferably 0.01 to 20% by mass, more preferably 0.1 to 10% by mass in the total composition. Moisturizing effect may be poor when the amount of water or humectant is too small, while the preparation will be unstable when the amount is too large.

The lip makeup cosmetic of the invention is not particularly restricted when the cosmetic are makeup cosmetic applied for use for the lips such as lipsticks and lip glosses, and any configurations thereof may be available including stick, pencil, ointment, liquid and gel forms. The lip makeup cosmetic of the invention may be produced by melting a mixture containing the essential ingredients as described above with heating, and by flowing into a stick or dish followed by cooling for solidification. The lip makeup cosmetic of the invention can be used as lipsticks and lip glosses as well as lip creams without blending with color materials.

### Eyelashes makeup cosmetic

The copolymer produced as described above can be particularly blended in the eyelashes makeup cosmetic in the invention in order to improve film-like feeling as well as curling effect and retaining performance of the eyelashes makeup cosmetic.

The amount of blending of the copolymer in the eyelashes makeup cosmetic according to the invention is not particularly restricted, and the amount may be appropriately adjusted depending on the object of use. However, the amount is preferably 1.0 to 30% by mass, more preferably 3.0 to 25.0% by mass, relative to the total amount of the eyelashes makeup cosmetic. The curling effect and retaining performance improving effect may not be exhibited when the amount of blending of the copolymer is less than 1.0% by mass, while film-like feeling becomes evident when the amount is more than 30.0% by mass.

Components usually used in cosmetic and pharmaceuticals may be blended in the eyelashes makeup cosmetic of the invention in a range not impairing the effect of the invention in addition to the copolymer as an essential ingredient of the cosmetic.

While conventional eyelashes makeup cosmetic are formulated as water-in-oil or oil-in-water emulsion base materials mainly comprising liquid oil components and water or oil-based materials mainly comprising solid oil components and volatile liquid oil components (not blended with water), the eyelashes makeup cosmetic of the invention are not limited thereto, and formulation type may be appropriately selected depending on the application field. The eyelashes makeup cosmetic of the invention may be formulated as either a water-in-oil emulsion base materials, an oil-in-water emulsion base material or an oil-based material.

For example, the water-in-oil eyelashes makeup cosmetic of the invention comprises the copolymer and oils in the oil phase as an outer phase and water in the aqueous phase as an inner phase. Alternatively, the oil-in-water eyelashes makeup cosmetic according to the invention comprises the copolymer and oils in the oil phase as an inner phase and water in the aqueous phase as an outer phase.

Preferably, the water-in-oil or oil-in-water eyelashes makeup cosmetic of the invention comprises volatile silicone oil or hydrocarbon oil blended in the oil phase as the inner phase or outer phase. Examples of the volatile silicone oil used in the invention include chain polysiloxane such as dimethyl polysiloxane, methylphenyl polysiloxane and methyl hydrogene polysiloxane; and cyclic polysiloxane such as octamethyl cyclotetrasiloxane, decamethyl cyclopentasiloxane, dodecamethyl cyclohexasiloxane, and tetramethyl tetrahydrogene cyclotetrasiloxane. Examples of the commercially available silicone oils include KF96A-0.65, KF96A-1, KF96A-1.5, KF994, KF995 and KF9937 (manufactured by Shin-Etsu Chemical Co. Ltd.), SH200-1cs, SH200-1.5cs and SH200-2cs (manufactured by Toray-Dow Coming Silicone Co. Ltd.), and TSF404, TSF405 and TSF4045 (manufactured by GE Toshiba Silicones).

One or more of the volatile silicone oil may be selected for blending in the water-in-oil or oil-in-water eyelashes makeup cosmetic according to the invention. The amount of blending of the volatile silicone oil is preferably 1.0 to 40.0% by mass, more preferably 5.0 to 30.0% by mass, relative to the total amount of the eyelashes makeup cosmetic. The cosmetic can be hardly applied to the eyelashes due to too rapid drying of the mascara liquid when the amount of blending of the volatile silicone oil is too small, while the mascara liquid tends to adhere around the eyes since the drying speed of the mascara liquid is retarded when the amount is too large.

Either linear or branched hydrocarbon oils may be used for the volatile hydrocarbon oil used in the invention. Examples of commercially available volatile hydrocarbon oil include Isopar A, C, D, E, G, H, K, L and M (trade names, manufactured by Exon Co.), Shellsol (trade name, manufactured by Shell Co.), Soltrol 100, 130 and 220 (trade names, manufactured by Philips Co.), Isosol (trade name, manufactured by Nippon Petroleum Chemical Co.), Pearl Ream 4 (trade name, Manufactured by Nippon Oil & Fat Co.), IP Solvent 1620 and 2028 (trade names, manufactured by Idemitsu PetroChemical Co. Ltd.), isohexadecane and tetraisobutane 90 (manufactured by Bayer Yakuhin), and Permethyl 99A, 101A and 102A (trade names, manufactured by Press Perse Co.).

One or more of the volatile hydrocarbon oils may be selected for blending in the water-in-oil or oil-in-water eyelashes makeup cosmetic according to the invention. The amount of blending of the volatile hydrocarbon oil is preferably 1.0 to 40.0% by mass, more preferably 5.0 to 30% by mass, relative to the total amount of the eyelashes makeup cosmetic. The cosmetic can be hardly applied to the eyelashes due to too rapid drying of the mascara liquid when the amount of blending of the volatile hydrocarbon oil is too small, while the mascara liquid is liable to adhere around the eyes before drying when the drying speed of the mascara liquid is too rapid when the amount is too large.

Preferably, an emulsion resin having film-forming ability may be blended in the aqueous phase as the inner or outer phase in the water-in-oil or oil-in-water eyelashes makeup cosmetic according to the invention. The emulsion resin having film-forming ability used in the invention may be obtained, for example, by soap-free polymerization taking advantage of a reactive emulsifying agent, heterogeneous polymerization in water containing no emulsifying agent, or polymerization using an aqueous resin solution as an emulsifying agent in which a mixture comprising polymerizable monomers is polymerized as an emulsion in the presence of a radical polymerization initiator.

Specific examples of the monomer constituting the emulsion resin having film-forming ability include acrylic and methacrylic monomers such as methyl (meth)acrylate, ethyl (meth)acrylate, propyl (meth)acrylate, butyl (meth)acrylate, isobutyl (meth)acrylate, t-butyl (meth)acrylate, benzyl (meth)acrylate, hexyl (meth)acrylate, octyl (meth)acrylate, 2-ethylhexyl (meth)acrylate, lauryl (meth)aerylate, stearyl (meth)acrylate, 2-hydroxylethyl (meth)acrylate, methoxy (meth)acrylate, ethoxy (meth)acrylate, butoxy (meth)acrylate, (meth)acrylic acid, acryl(meth)amide, styrene or α-styrene, styrene sulfonic acid vinyl acetate, vinylethers, maleic anhydride, crotonic acid, itaconic acid, cinnamic acid, polydimethylsiloxane methacrylate, polydimethylsiloxane acrylate, fluoroalkyl (meth)acrylate, and unsaturated monomers of alkoxysilane.

Examples of the radical polymerization initiator include water soluble types including persulfates such as potassium persulfate and ammonium persulfate, hydrogen peroxide, t-butyl hydroperoxide, and azobisamidinopropane hydrochloride; and oil soluble types such as benzoyl peroxide, cumene hydroperoxide, dibutyl peroxide, diisopropyl peroxydicarbonate, cumyl peroxyneodecanoate, and azobisisobutylonitrile. Reducing agents such as L-ascorbic acid, acid potassium sulfite, rongalite, sugars and amines may be used.

One or more of the emulsion resins having film-forming ability may be selected for blending with the water-in-oil or oil-in-water eyelashes makeup cosmetic according to the invention. The amount of blending of the emulsion resin having the film-forming ability is preferably 1.0 to 30.0% by mass, more preferably 5.0 to 20.0% by mass, relative to the total amount of the eyelashes makeup cosmetic. Makeup retaining performance may be poor when the amount of blending of the emulsion resin having film-forming ability is too small, while finish of makeup may be rather stiff when the amount is too large.

The oil-based eyelashes makeup cosmetic according to the invention comprises the copolymer and wax. The wax as used in the invention means solid oils at room temperature, and examples thereof include beeswax, candelilla wax, cotton wax, carnauba wax, batberry wax, ibota wax, spermaceti wax, montan wax, rice bran wax, lanolin, kapok wax, vegetable wax, lanolin acetate, liquid lanolin, sugar cane wax, lanolin fatty acid isopropyl, hexyl laurate, reduced lanolin, jojoba wax, rigid lanolin, shellac wax, bees wax, microcrystalline wax, paraffin wax, POE lanolin alcohol ether, POE lanolin alcohol acetate, POE cholesterol ether, lanolin fatty acid polyethyleneglycol, fatty acid glyceride, rigid castor oil, vaseline and POE hydrogenated lanolin alcohol ether.

One or more of the waxes may be selected for blending with the oil-based eyelashes makeup cosmetic according to the invention. The amount of blending of the wax is preferably 0.1 to 25% by mass, more preferably 1.0 to 20.0% by mass, relative to the total amount of the eyelashes makeup cosmetic. A volume effect may be impaired when the amount of blending of the wax is too small, while finish of makeup may be poor when the amount is too large.

Preferably, the volatile silicone oil or hydrocarbon oil is blended with the oil-based eyelashes makeup cosmetic of the invention. Specific examples and favorable amount of blending of the volatile silicone oil and hydrocarbon oil are as described previously.

A viscosity improving agent is preferably blended with the oil-based eyelashes makeup cosmetic according to the invention. Examples of the viscosity improving agent include gum Arabic, carrageenan, karaya gum, gum tragacanth, carob gum, quince seed (marmelo), casein, dextrin, gelatin, sodium pectinate, sodium arginate, methyl cellulose, ethyl cellulose, carboxymethyl cellulose, hydroxyethyl cellulose, hydroxypropyl cellulose, polyvinyl alcohol, polyvinylmethyl ether, polyvinyl pyrrolidone, sodium polyacrylate, carboxyvinyl polymer, Locust bean gum, guar gum, tamalint gum, dialkyldimethylammonium sulfate cellulose, xanthan gum, aluminum magnesium silicate, bentonite and hectorite.

One or more of the viscosity improving agent may be selected for blending with the oil-based eyelashes makeup cosmetic according to the invention. The amount of blending of the viscosity improving agent is preferably 0.1 to 20% by mass, more preferably 1.0 to 18.0% by mass* relative to the total amount of the eyelashes makeup cosmetic. Application may be difficult or volume effect may be impaired when the amount of blending of the viscosity improving agent is too small, while application may become difficult and finish of makeup may be poor when the mount is too large.

Preferably, a hollow powder is blended to the oil-based eyelashes makeup cosmetic according to the invention. Examples of the hollow powder include a hollow resin powder and a hollow inorganic powder.

Basically, the hollow resin powder is prepared by allowing a thermoplastic resin, which contains a volatile foaming agent that is vaporized mainly by heating, to swell or foam by heating. Examples of the resin that forms an outer shell of the hollow foaming resin powder include homopolymers or copolymers comprising one or more monomer selected from vinyl monomers such as vinyl chloride, vinyl acetate and methylvinyl ether; acrylic monomers such as acrylic acid, acrylic acid esters, methacrylic acid, methacrylic acid esters, acrylonitrile and methacrylonitrile; and styrene, vinylidene chloride, divinylbenzene and ethyleneglycol dimethacrylate. The resin is preferably a copolymer comprising two or more monomers selected from acrylic acid or methacrylic acid or esters thereof, vinylidene chloride, acrylonitrile and methacrylonitrile. These polymers may be cross-linked with a cross-linking agent such as divinylbenzene, ethyleneglycol dimethacrylate and triacryl formal.

While the volatile foaming agent is not particularly restricted, examples available include low boiling point compounds such as hydrocarbons including methane, ethane, propane, butane, isobutane, isobutene, pentane, isopentane, neopentane, hexane, neohexane, haptane and acetylene; halogenated hydrocarbons including trichlorofluoromethane and dichlorodifluorometane; and tetraalkylsilane.

The hollow resin powder is produced by allowing a thermoplastic resin powder containing a volatile foaming agent to foam by heating, for example, as disclosed in JP-B No. 59-53290. The hollow resin powder is commercially available, and examples thereof include Matsumoto microsphere MFL series [MFL-50STI (particle diameter 10 to 30 µm, absolute specific gravity 0.20), MFL-50SCA (particle diameter 10 to 30 µm, absolute specific gravity 0.29), MFL-80GCA(partiele diameter 10 to 30 µm, absolute specific gravity 0.20), MFL-80CA (particle diameter 90 to 110 µm, absolute specific gravity 0.13), MFL-100SCA (particle diameter 20 to 40 µm, absolute specific gravity 0.20), MFL-100CA (particle diameter 90 to 110 µm, absolute specific gravity 0.13), MFL-30STI(particle diameter 10 to 30 µm, absolute specific gravity 0.20)] and Matsumoto Microsphere F-80ED (particle diameter 90 to 110 µm, absolute specific gravity 0.020 to 0.030) manufactured by Matsumoto Yushi-Seiyaku Co., Ltd.; EXPANCEL microsphere 551 DE 40 d42 (particle diameter 30 to 50 µm, true density 42 kg/m³), 551DE 40 d60 (particle diameter 15 to 25 µm, true density 60 kg/m³), 551 DE 80 d42 (particle diameter 50 to 80 µm, true density 42 kg/m³), 461 DE 40 d60 (particle diameter 20 to 40 µm, true density 60 kg/m³), 461 DE 20 d70 (particle diameter 15 to 25 µm, true density 70 kg/m³), 051 DE 40 d60 (particle diameter 20 to 40 µm, true density 60 kg/m³), 091 DE 40 d30 (particle diameter 35 to 55 µm, true density 30 kg/m³), 091 DE 80 d30 (particle diameter 60 to 90 µm, true density 30 kg/m³), 092 DE 40 d30 (particle diameter 35 to 55 µm, true density 30 kg/m³) and 092 DE 80 d30 (particle diameter 60 to 90 µm, true density 32 kg/m³) manufactured by Expancel; and Ganz pearl GMH-0850 (particle diameter 8 µm, absolute specific gravity 0.65) manufactured by Ganz Chemical Co., Ltd.

The hollow inorganic powder is prepared by allowing inorganic materials such as a glass including a volatile foaming agent that is vaporized mainly by heating to swell or foam by heating, or from fly ash formed in a combustion process of coal (fine coal powder). The hollow inorganic powder is commercially available, and examples thereof include Scotch Light Glass Bubble series [K1 (true density 0.125 g/cm³), K15 (true density 0.150 g/cm³), K20 (true density 0.200 g/cm³), K25 (true density 0.250 g/cm³), K37 (true density 0.370 g/cm³), K46 (true density 0.460 g/cm³),] and S series [S22 (true density 0.220 g/cm³), S38 (true density 0.380 g/cm³), S60 (true density 0.600 g/cm³)], and floated series [A16 (true density 0.16 g/cm³), A20 (true density 0.20 g/cm³), D32 (true density 0.2 g/cm³)] manufactured by Sumitomo 3M Co., Ltd.; CEL-Star Z-20 (mean particle diameter 67 µm, true density 0.17 to 0.23), Z-25 (mean particle diameter 65 µm, true density 0.22 to 0.28), Z-27 (mean particle diameter 63 µm, true density 0.24 to 0.30), Z-31T (mean particle diameter 60 µm, true density 0.28 to 0.34), Z-36 (mean particle diameter 56 µm, true density 0.33 to 0.39), SX-39 (mean particle diameter 40 µm, true density 0.36 to 0.42), Z-45 (mean particle diameter 52 µm, true density 0.42 to 0.48) and PZ-6000 (mean particle diameter 40 µm, true density 0.70 to 0.80), and Metasphere #52 as fly ash balloon (mean particle diameter 128 µm, true density 0.6 to 0.8) and Metasphere #100 (mean particle diameter 72 µm, true density 0.6 to 0.8) manufactured by Tokai Kogyo Co., Ltd.

The surface of the hollow powder may be coated with an inorganic substance. As disclosed in JP-A No. 4-9319, the thermoplastic resin including the volatile foaming agent is mixed with the inorganic powder before foaming or during foaming, and the mixture is heated to obtain the hollow powder coated with the inorganic powder. Otherwise, the hollow powder is coated with the inorganic powder by a wet method, in which a dispersion solution of the inorganic powder in water or in an organic solvent and the hollow resin powder are mixed followed by drying, or the dispersion solution of the inorganic powder is sprayed onto the hollow resin powder followed by drying, or the hollow powder and inorganic powder are complexified by a physical force such as a high impact force.

While the inorganic powder to be coated on the surface of the hollow resin powder is not particularly restricted, it is selected depending on desired effects. Examples of the inorganic powder include talc, sericite, mica, calcium carbonate, magnesium carbonate, kaolin, boron nitride, titanium oxide, zinc oxide, iron oxide, cerium oxide, zirconium oxide and silica. The particle shape of these inorganic powders is not particularly restricted, and it may be granular, spherical, plate-like or needle-like. While the average particle diameter is not restricted, it is preferably 0.001 to 20 µm. The mass ratio between the hollow resin powder and inorganic powder is preferably 5:95 to 50:50.

The hollow powder used in the invention is preferably the hollow resin powder. The resin forming the outer shell is preferably resins of vinyl chloride, vinylidene chloride and methyl methacrylate, while hydrocarbon gases are favorably used as the volatile liquid foaming agents. While commercially available foaming agents include MFL-50SCA (trade name, manufactured by Matsumoto Yushi-Seiyaku Co.) and GHM-0850 (trade name, manufactured by Ganz Chemical Co., Ltd.), they are not restricted thereto.

One or more of the hollow powder may be selected for blending with the oil-based eyelashes makeup cosmetic of the invention. The amount of blending of the hollow powder is preferably 0.001 to 10.0% by mass, more preferably 0.1 to 8.0% by mass, relative to the total amount of the eyelashes makeup cosmetic. The curling effect and volume effect are lowered when the amount of blending of the hollow powder is too small, while finish of makeup becomes poor when the amount is too large.

The eyelashes makeup cosmetic is not particularly restricted in the invention so long as the cosmetic are used for applying on the eyelashes such as mascara, and are used irrespective of their configurations. The eyelashes makeup cosmetic may be used as mascara as well as transparent mascara in which no color materials are blended.

### Skin makeup cosmetic

The copolymer produced as described above is blended in the skin makeup cosmetic in the invention for improving film-like feeling as well as retaining performance of the skin makeup cosmetic.

While the amount of blending of the copolymer is not particularly restricted in the skin makeup cosmetic according to the invention, and the copolymer may be used by appropriately controlling the amount of blending depending on the object of use. The amount of blending is preferably 0.01 to 20% by mass, more preferably 0.2 to 10% by mass, relative to the total amount of cosmetic. The effect for improving retaining performance cannot be exhibited when the amount of blending of the copolymer is less than 0.01% by mass, while film-like feeling may be evident when the amount is more than 20% by mass.

Preferably, the skin makeup cosmetic according to the invention further comprises a siliconated polysaccharide represented by following formula (11). 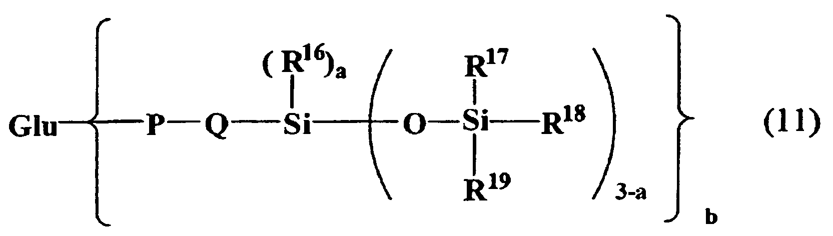 where, Glu is a sugar residue of the polysaccharide, P is a divalent binding group, Q represents a divalent aliphatic group, R¹⁶ is a hydrocarbon group having 1 to 8 of carbon atoms, and R¹⁷, R¹⁸ and R¹⁹ is hydrocarbon groups having 1 to 8 of carbon atoms or siloxy groups resented by -OSiR²⁰R²¹R²², wherein R²⁰, R²¹ and R²² are hydrocarbon group having 1 to 8 of carbon atoms, a is an integer of 0 to 2, and b is a positive integer.

In the siliconated polysaccharide represented by formula (11), Glu is a sugar residue of the polysaccharide compound. Various known polysaccharide compounds may be used for polysaccharide compound, and examples of them include cellulose, hemicellulose, gum Arabic, tragacanth gum, tamarind gum, pectin• starch, mannan, guar gum, locust bean gum, quince seed gum, alginic acid, carrageenan, agar, xanthan gum, dextran, pullulane, chitin, chitosane, hyaluronic acid and chondroitin sulfate, as well as derivatives of these polysaccharide compounds, for example carboxymethylated, sulfated, phosphated, methylated, ethylated polysaccharide compounds, adducts of alkylene oxide such as ethylene oxide and propylene oxide, acylated or cationated polysaccharide compounds, and degraded polysaccharide derivatives. Ethyl cellulose and pullulane are preferable among these compounds, and pullulane is particularly preferable. While the average molecular weight of the polysaccharide compound differs depending on the kind of the polysaccharide compound in the invention, it is preferably 1,000 to 5,000,000.

These polysaccharide compounds comprise at least one or more reactive functional group such as a hydroxyl group or carboxyl group depending on the kind of the polysaccharide compound. The divalent linking group represented by P is derived from A by formed by allowing the reactive functional group of the polysaccharide compound to react with the silicone compound represented by formula (14) below.

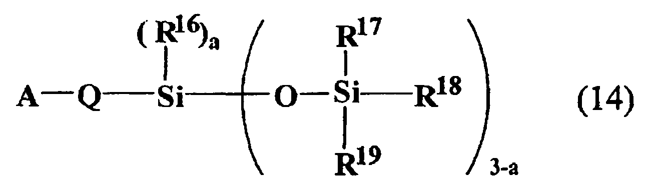

Q, R¹⁶, R¹⁷, R¹⁸, R¹⁹ and a in formula (14) are defined as those in formula (11). A represents a functional group capable of reacting with the reactive functional group of the polysaccharide compound, and examples thereof include an isocyanate group, an epoxy group, a vinyl group, an acryloyl group, a methacryloyl group, an amino group, an imino group, a hydroxyl group, a carboxyl group and a mercapto group.

Known methods, for example, the method described in JP-A No. 8-134103, may be used for the reaction of these silicone compounds with polysaccharide.

While examples ofP include a carbamoyl group, -CH₂CH(OH)-, carbonyl group, amino group and ether group, the carbamoyl group (-CONH-) that is formed by allowing a compound represented by formula (11), in which A is an isocyanate group (O=°C=N-), to react with the hydroxyl group of the polysaccharide compound is preferable from the view point of reactivity. The sugar residue of the polysaccharide compound in this case corresponds to a remaining portion of the polysaccharide compound except hydrogen atoms that react with the isocyanate group. The sugar residue of the polysaccharide compound also has the same meaning in other reactions.

Examples of the divalent aliphatic group represented by Q include an alkylene group, an alkylene group in the main chain having oxygen, nitrogen or sulfur atoms in the main chain, an alkylene group having an arylene group such as a phenylene group, and an alkylene group having a carbonyloxy group or oxycarbonyl group in the main chain. These divalent aliphatic group may comprise substituents such as hydroxyl, alkoxy and alkyl groups, and terminal atoms of the aliphatic group may be hetero atoms such as oxygen, nitrogen and sulfur atoms. While examples of Q include -(CH₂)₂-, -(CH₂)₃-, -(CH₂)₄-, -(CH₂)₆-, -(CH₂)₈-, -[(CH₂)CH(CH₃)]-, -(CH₂)₂O(CH₂)₃- and -CH₂CH(OH)-CH₂-, the propylene group represented by -(CH₂)₃- is preferable.

Examples of the monovalent organic group having a 1 to 8of carbon atoms represented by R¹⁶, R¹⁷, R¹⁸, R¹⁹, R²⁰ and R²¹ in formula (11) include alkyl groups such as methyl group, ethyl group, propyl group and butyl group; cycloalkyl groups such as cyclopentyl group and cyclohexyl group; aryl groups such as phenyl group; aralkyl groups such as benzyl groups; alkenyl groups such as vinyl group and allyl group; and fluorinated alkyl groups such as 3,3,3-trifuloropropyl group. The alkyl group is most preferable among these organic groups, and the methyl group is even more preferable.

R¹⁷, R¹⁸ and R¹⁹ each may be a siloxy group represented by -OsiR²⁰R²¹R²². Examples of such siloxy group include trimethyl siloxy group, ethyldimethyl siloxy group, phenyldimethyl siloxy group, vinyldimethyl siloxy group and 3,3,3-trifluoropropyldirnethyl siloxy group. R¹⁶, R¹⁷, R¹⁸, R¹⁹, R²⁰, R²¹ and R²² may be the same or different with each other. In siliconated polysaccharide used in the invention, a = 0, and R¹⁷, R¹⁸ and R¹⁹ are preferably methyl groups.

The degree of substitution of siliconated polysaccharide in the invention refers to an average number of bonds of the silicone compound per one unit of the constituting sugar of polysaccharide. Specifically, it is preferable that the average number of bonds of the silicone compound per one unit of the constituting sugar of polysaccharide is 0.1 to 2.0, although it depends on the kinds.

The particularly preferable siliconated polysaccharide in the invention is siliconated pullulane represented by formula (12). 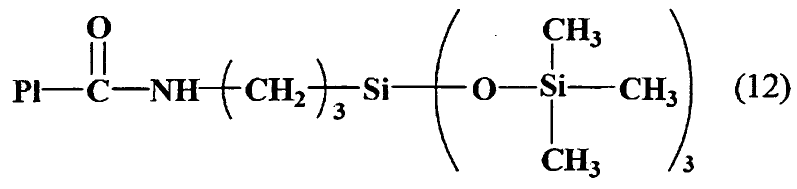 where, P1 is a hydroxyl group of a glucose residue of pullulane with a degree of substitution per one unit of the constituting sugar of 0.1 to 2.0.

For example, the degree of substitution of siliconated pullulane above denotes an average value of the substituent -CONH(CH₂)₃Si[OSi(CH₃)₃] on the basic unit of pullulane represented by formula (15) below.

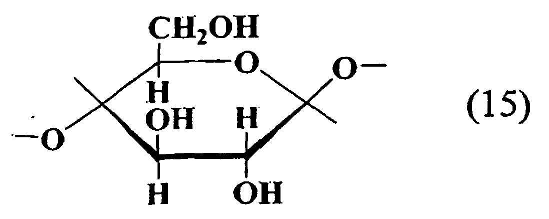

Siliconated pullulane is produced by using pullulane as one of water-soluble polysaccharide as a starting material. Pullulane is an extra-cellular polysaccharide biologically produced by Aureobasidium pullulans, which is water-soluble polysaccharide composed of D-glucopyranose residues and is a neutral linear glean. While the molecular weight of the pullulane molecule is different depending on culture conditions of the microorganisms and the strain of the microorganisms, the molecular weight of pullulane used for the starting material of siliconated pullulane blended in the skin makeup cosmetic of the invention is desirably 50,000 to 10,000,000.

Siliconated pullulane can be produced, for example, by the method described in JP-A No. 8-134103, i.e. by allowing the hydroxyl group of pullulane to react with oregano-polysiloxane having isocyanate groups. The degree of substitution of siliconated pullulane is 0.1 to 2.0, preferably 1.5 to 1.9.

While the amount of blending of siliconated polysaccharide is not particularly restricted in the skin makeup cosmetic according to the invention, it is usually 0.01 to 20% by weight, preferably 0.2 to 10% by weight. A sufficient effect cannot be obtained when the amount of blending of siliconated polysaccharide is too small, while the cosmetic become sticky with heavy feeling of use when the amount is too large.

The blending ratio of the copolymer to siliconated polysaccharide is preferably 5:95 to 95:5, more preferably 10:90 to 90:10 in mass ratio in the skin makeup cosmetic according to the invention. No improvement of retaining performance and freeness from film-like feeling cannot be attained when the blending ratio between the copolymer and siliconated polysaccharide is out of the range described above.

Components usually used in conventional cosmetic and pharmaceuticals other than the components as described above may be blended in the skin makeup cosmetic according to the invention in the range not impairing the effect of the invention.

While the use of the skin makeup cosmetic according to the invention is not particularly restricted so long as the copolymer as an essential ingredient is used in the skin makeup cosmetic, it can be used for various products such as creams, foundations, eye shadows, eye liners and body makeup preparations.

### Water-in-oil emulsion cosmetic

Stability of the emulsion and dispersibility of the powder may be improved by blending the copolymer produced as described above as an emulsifying agent in the water-in-oil emulsion cosmetic.

The amount of blending of the copolymer in the water-in-oil cosmetic according to the invention is not particularly restricted, and the copolymer may be used by appropriately adjusting the amount of blending depending on the object of use. The amount of blending of the copolymer is 0.1 to 10.0% by mass, preferably 0.5 to 5.0% by mass, relative to the total amount of the composition. Stability of the emulsion may be deteriorated when the amount of blending of the copolymer is less than 0.1% by mass, while the effect of blending is not improved any more when the amount is more than 10.0% by mass.

It is favorable that the water-in-oil emulsion cosmetic according to the invention further comprises water-swellable clay minerals and quaternary ammonium salt type cationic surfactants.

Examples of the water-swellable clay mineral used in the water-in-oil emulsion cosmetic according to the invention include layered silicate minerals belonging to smectite minerals. Specific examples of these layered silicate minerals of smectite minerals available in the invention include montmorillonite, beidellite, nontronite, saponite and hectorite, which may be either natural or synthetic. Examples of commercially available water-swellable clay mineral include Kunipia and Smecton (trade name, manufactured by Kunimine Industries, Co., Ltd.), Beagum (trade name, manufactured by Bunder Built Co.), Laponite (trade name, LaPorte Co.) and fluorine tetrasilicate mica (trade name, manufactured by Topy Industries, Ltd.). One or more of the water-swellable clay mineral may be selected for use in the water-in-oil emulsion cosmetic according to the invention.

The amount of blending of the water-swellable clay mineral in the water-in-oil emulsion cosmetic according to the invention is not particularly restricted, and may be appropriately adjusted depending on the object of use. The amount of blending is preferably 0.1 to 10.0% by mass, more preferably 0.2 to 5.0% by mass, relative to the total amount of the composition. Stability of the emulsion may be deteriorated when the amount of blending of the water-swellable clay mineral is less than 0.1% by mass, while feeling of use may be impaired due to too large viscosity of the emulsion when the amount is more than 10.0% by mass.

An example of the quaternary ammonium salt type cationic surfactant used for the water-in-oil emulsion cosmetic according to the invention is represented by formula (16) as below. 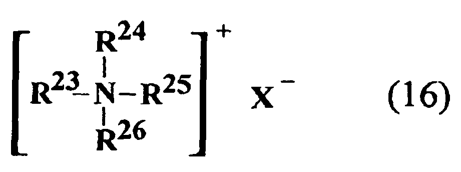 where, R²³ is an alkyl or a benzyl group having 10 to 22 of carbon atoms; R²⁴ is a methyl group or an alkyl group having 10 to 22 of carbon atoms; each of R²⁵ and R²⁶ are an alkyl group or hydroxyalkyl group having 1 to 3 of carbon atoms; and X is a halogen atom or a methylsulfate residue.

Specific examples of the quaternary ammonium salt type cationic surfactant include dodecyltrimethyl ammonium chloride, myristyltrimethyl ammonium chloride, cetyltrimethyl ammonium chloride, stearyltrimethyl ammonium chloride, aralkyltrimethyl ammonium chloride, behenyltrimethyl ammonium chloride, myristyldemethylethyl ammonium chloride, cetyldiemthyl ammonium chloride, steaiyldimethylethyl ammonium chloride, aralkyldimethylethyl ammonium chloride, behenyldimethylethyl ammonium chloride, myristyldiethylmethyl ammonium chloride, cetyldoethylmethyl ammonium chloride, stearyldiethylmethyl ammonium chloride, aralkyldiethylmethyl ammonium chloride, nehenyldiethylmethyl ammonium chloride, benzyldimethylmyristyl ammonium chloride, benzyldimethylcetyl ammonium chloride, benzyldimethylstearyl ammonium chloride, benzyldimethylbehenyl ammonium chloride, benzylmethylethylcetyl ammonium chloride, benzylmethylethylstearyl ammonium chloride, distearyldimethylammonium chloride, dibehenyldihydroxyethyl ammonium chloride, and corresponding bromides as well as dipalmitylpropyl ammonium methylsulfate. One or more of the quaternary ammonium salt type cationic surfactant may be selected for use in the water-in-oil emulsion cosmetic according to the invention.

The amount of blending of the quaternary ammonium salt type cationic surfactant in the water-in-oil emulsion cosmetic according to the invention is not particularly restricted, and may be appropriately adjusted depending on the object of use. The amount of blending is preferably 40 to 140 milliequivalent (abbreviated as meq), more preferably 60 to 120 meq, relative to 100 g of the water-swellable clay mineral. Stability of the emulsion may be deteriorated when the amount of blending of the quaternary ammonium salt type cationic surfactant is less than 40 meq, while the effect for improving the stability of the emulsion cannot be improved any more when the amount is more than 140 meq.

The water-swellable clay mineral which is an essential ingredient and quaternary ammonium salt type cationic surfactant in the preparation of water-in-oil emulsion cosmetic according to the invention may be separately added as an oil phase component or aqueous phase component, or may be added in the oil phase as a organo-modified clay mineral after they have been allowed to react in an appropriate solvent.

An example of a commercially available water-swellable clay mineral that has been reacted with the quaternary ammonium salt type cationic surfactant is Benton (trade name, manufactured by National Red Co.)

When organo-modified clay mineral prepared by allowing the water-swellable clay mineral to react with the quaternary ammonium salt type cationic cosmetic in advance is used in the water-in-oil emulsion surfactant according to the invention, the ratio of blending of the copolymer to the organo-modified clay mineral is 0.01:1 to 20:1, preferably 0.2:1 to 10:1, in mass ratio. Sufficient stability with time cannot be attained when the ratio of blending of the copolymer to the organo-modified clay mineral is out of the range described above.

Components used in conventional cosmetic and pharmaceuticals may be blended in the water-in-oil emulsion cosmetic according to the invention in addition to the components above in the range not impairing the effect of the invention.

A powder may be favorably used together with the essential ingredients in the water-in-oil emulsion cosmetic according to the invention. When a water-in-oil emulsion cosmetic was prepared by blending a large amount of powders using a conventionally used emulsifying agent, feeling of use as cosmetic was severely deteriorated due to coagulation of powders having poor dispersibility. On the contrary, dispersibility of the powder is improved in the water-in-oil emulsion cosmetic according to the invention by virtue of blending of the essential ingredients as described above.

Examples of the powder used in the invention include inorganic white pigments such as talc, kaolin, sericite, muscovite, titanium oxide and iron oxide; inorganic red pigments such as iron oxide red and iron titanate; inorganic yellow pigments such as iron oxide yellow and yellow earth; inorganic purple pigments such as mango violet and cobalt violet; inorganic green pigments such as chromium oxide, chromium hydroxide and cobalt titanate; inorganic blue pigments such as ultramarine and prussian blue; pearl pigments such as titanium oxide-coated mica, titanium oxide-coated bismuth oxide, bismuth oxychloride, titanium oxide-coated talc, fish scale foil and colored titanium oxide-coated mica; metal powder pigments such as aluminum powder and copper powder; inorganic powder such as synthetic mica, bronze mica, red mica, black mica, lithia mica, vermiculite, magnesium carbonate, calcium carbonate, diatom earth, magnesium silicate, calcium silicate, aluminum silicate, barium silicate, strontium silicate, metal salts of tungustic acid, α-iron oxide, iron oxide hydrate, silica and hyroxyapatite; and organic powders such as nylon powder, polyethylene powder, benzoguanamine powder, fine crystalline cellulose powder and silicone powder. A composite powder prepared by coating an organic powder with an inorganic powder, and various powders subjected to hydrophobic treatment with metal soaps, silicone and fatty acid esters may be also used.

One or more of the powder may be blended in the water-in-oil emulsion cosmetic according to the invention. The amount of blending of the powder in the composition is preferably 0.1 to 70% by mass, more preferably 0.5 to 60% by mass. The effect of blending of the powder cannot be sufficiently manifested when the amount of blending is too small, while the powder is not sufficiently dispersed to form coagulation when the amount is too large.

A nonionic surfactant may be favorably blended in the water-in-oil emulsion cosmetic according to the invention as well as the essential ingredients. While the nonionic surfactant used in the invention is not particularly restricted, a surfactant with an HLB value of 2 to 16 is preferably used, and a surfactant with a HLB value of 3 to 12 is more preferably used. Examples of the nonionic surfactant used in the invention include ethyleneoxide adduct type surfactant including ether-base surfactants such as oleylether adduct with 2 to 30 mole of polyoxyethylene (abbreviated as POE(2 to 30) hereinafter), POE(2 to 35) stearylether, POE(2 to 30) laurylether, POE(1 to 20) alkylphenylether, POE(6 to 18) behenylether, POE(5 to 25) 2-decylpentadecylether, POE(3 to 30) 2-decyltetradecylether and POE(8 to 16) 2-octyldecylether; ester-base surfactant such as POE(4 to 60) hardened caster oil, POE(3 to 14) fatty acid monoester, POE(5 to 20) sorbitan fatty acid ester; ether-ester-base surfactants such as POE(2 to 30) glycerylmonoisostearate, POE(10 to 60) glyceryltriisostearate, POE(7 to 50) hardened caster oil monoisostearate and POE(12 to 60) hardened caster oil triisostearate; and glycerin fatty acid ester type surfactant including polyglycerin fatty acid esters such as decagluceryl tetraoleate, hexaglyceryl triisostearate, tetraglyceryl diisostearate and diglyceryl diisostearate; and glycerin fatty acid esters such as glyceryl monostearate, glyceryl monoisostearate and glyceryl monooleate.

One or more nonionic surfactant may be blended in the water-in-oil emulsion cosmetic according to the invention. The amount of blending of the nonionic surfactant is preferably 0.1 to 10.0% by mass, more preferably 0.2 to 5.0% by mass, in the composition. The cosmetic may become sticky when the amount of blending of the nonionic surfactant is too large.

Preferably, volatile silicone may be blended with the water-in-oil emulsion cosmetic according to the invention as well as the essential ingredients. Examples of volatile silicone used in the invention include linear polysiloxane such as dimethyl polysiloxane, methylphenyl polysiloxane and methyl hydrogen polysiloxane; and cyclic polysiloxane such as octamethyl cyclotetrasilopxane, decamethyl cyclopentasiloxane, dodecamethyl cyclohexasiloxane and tetramethyl tetrahydrogen cyclosiloxane.

One or more of the silicone oil may be blended in the water-in-oil emulsion cosmetic according to the invention. The amount of blending of the silicone oil in the composition is preferably 0.1 to 90% by mass, more preferably 1.0 to 70% by mass. The cosmetic may be sticky when the amount of blending of the silicone oil is too small, while emulsification becomes poor when the amount of blending is too large.

A UV absorbing agent or UV scattering agent may be favorably blended with the water-in-oil emulsion cosmetic according to the invention as well as the essential ingredients. Examples of the UV absorbing agent used in the invention include benzoic acid-base UV absorbing agent such as paraaminobenzoic acid (abbreviated as PABA hereinafter), PABA monoglycerin ester, N,N-dipropoxy PABA ethyl ester, N.N-diethoxy PABA ethyl ester, N,N-dimethyl PABA ethyl ester, N,N-dimethyl PABA butyl ester and N,N-dimethyl PABA methyl ester; anthranilic acid-base UV absorbing agent such as homomenthyl-N-acetyl anthoranylate; salicylic acid-base UV absorbing agent such as amyl salicylate, menthyl salicylate, homomenthyl salicylate, octyl salicylate, phenyl salicylate, benzyl salicylate and p-isopropanol phenylsalicylate; cinnamic acid-base UV absorbing agent such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-eyano-β-phenyl cinnamate, 2-ethylhexyl-α-cyano-β-phenyl cinnamate, glyceryl mono-2-ethylhexanoyl-diparamethoxy cinnamate and trimethoxycinnamic acid methylbis(trimethylsiloxane)silylisopentyl; and 3-(4'-methylbenzylidene)-d,1 -camphor, 3-benzylidene-d,1-camphor, urocanic acid, urocanic acid ethyl ester, 2-phenyl-5-methyl benzoxazol, 2,2'-hydroxy-5-methylphenyl benzotriazole, 2-(2'-hydroxy-5'-t-octylphenyl)benzotriazole, 2-(2'-hydroxy-5'-methylphenyl)benzotriazole, dibenzalazine, dianisoyl methane, 4-methoxy-4'-t-butyl dibenzoyl methane, 5-(3,3-dimethyl-2-norbonylidene)-3-pentane-2-on and 2-ethylhexyl 2-cyano-3,3-diphenyl acrylate.

Examples of the UV scattering agent used in the invention include inorganic powders such as titanium and zinc oxide; and surface-coated inorganic powders prepared by coating the surface of the inorganic powder with fatty acid soap such as aluminum stearate and zinc palmitate, with fatty acid such as stearic acid, myristic acid and palmitic acid, and with fatty acid esters such as dextrin palmitate.

One or more of the UV absorbing agent and/or UV scattering agent may be selected for blending with the water-in-oil emulsion cosmetic according to the invention. The amount of blending of the UV absorbing agent and/or UV scattering agent is preferably 0.1 to 50.0% by mass, more preferably 1.0 to 40.0% by mass in the composition. A sufficient effect for protecting from UV light cannot be obtained when the amount of blending of the UV scattering agent is too small, while emulsification becomes insufficient when the amount is too large.

While the application fields of the water-in-oil emulsion cosmetic according to the invention are not particularly restricted so long as the cosmetic are the water-in-oil emulsion cosmetic containing the essential ingredients of the invention, for example, they may be applied for lotions, milky liquids, creams, foundations, lipsticks, cleansing foams, shampoos, hair rinses, lip creams, eyeliners, hair sprays, mousses, sunscreen or suntan lotion creams, mascara, treatment of hair or nails, and creams, body makeup agents.

While the invention is described in more detail with reference to examples of the invention, the invention is by no means restricted to these examples.

The structures of the monomers used in the production of the copolymers of the invention are shown below.
Monomer A1: 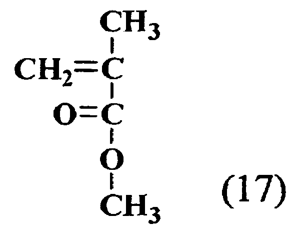 Monomer A2: 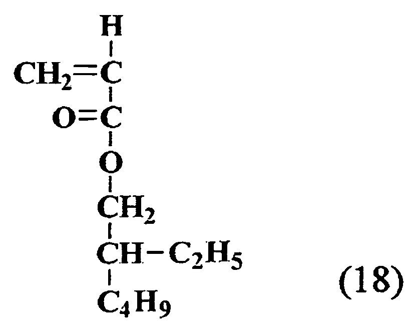 Monomer B1: 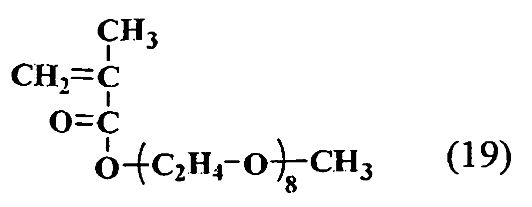 Monomer C1: 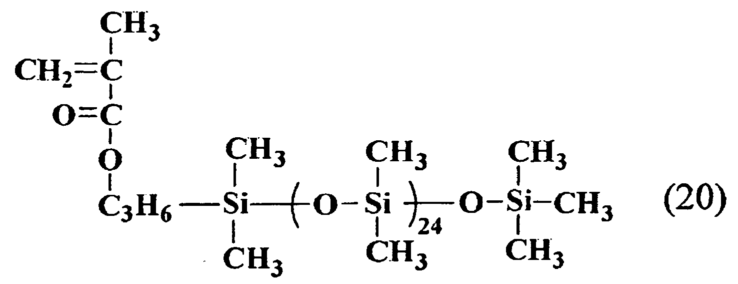 Monomer C2:

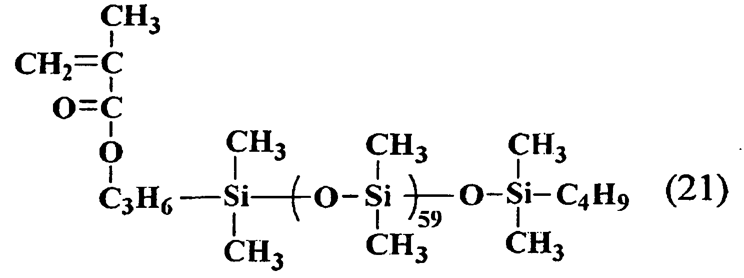

Then, the method for synthesizing the copolymer of the invention will be described below.

### Copolymer 1-1

Charged in a glass flask equipped with a stirrer, thermometer and reflux condenser are 35 parts by mass of methyl methacrylate (monomer A1) represented by formula (17), 5 parts by mass of polyoxyethylene ether methacrylate (monomer B1) represented by formula (19), 60 parts by mass of methacryloxypropyl polydimethylsiloxane (monomer C1) represented by formula (20), 120 parts by mass of isopropanol, and 4 parts by mass of dimethyl-2,2'-azobis(2-methylpropionate), and the mixture was allowed to react by heating at 80°C for 10 hours in a nitrogen stream. Then, volatile components were removed by evaporation in vacuum to obtain copolymer 1-1.

The inventors have prepared various copolymers according to the synthetic method above, and the copolymers were blended with various cosmetics to evaluate them as follows.

### 1. Lip makeup cosmetic

### Blending of copolymer

The inventors of the invention prepared various copolymers according to the production example above, and lipsticks in which each copolymer was blended as a film-forming component were compared with lipsticks in which conventional film-forming components were blended. The monomer proportion of the copolymer used in each test, blended composition of the lipsticks, and evaluation thereof are listed in Table 1 below. The evaluation criteria were as follows.

### (1) Makeup retaining performance

Tests for practical uses were conducted by 10 special panelists with respect to retaining performance of the lipstick in each test. The evaluation criteria are as follows:
A: more than 8 panelists recognized that retaining performance is good;
B: 6 or more and less than 8 of panelists recognized that retaining performance is good;
C: 3 or more and less than 6 panelists recognized that retaining performance is good; and
D: less than 3 panelists recognized that retaining performance is good.

### (2) Film-like feeling

Tests for practical uses were conducted by 10 special panelists with respect to film-like feeling of the lipstick in each test. The evaluation criteria are as follows:
A: more than 8 panelists recognized that there is no film-like feeling;
B: 6 or more and less than 8 panelists recognized that there is no film-like feeling;
C: 3 or more and less than 6 panelists recognized that there is no film-like feeling;
D: less than 3 panelists recognized that there is no film-like feeling.

### (3) Luster

Tests for practical uses were conducted by 10 special panelists with respect to luster of the lipstick in each test. The evaluation criteria are as follows:
A: more than 8 panelists recognized that luster is good;
B: 6 or more and less than 8 panelists recognized that luster is good;
C: 3 or more and less than 6 panelists recognized that luster is good;
D: less than 3 panelists recognized that luster is good.

### (4) Solubility in formulation

Solubility of the copolymer in formulation was evaluated with respect to luster of the lipstick in each test. The evaluation criteria are as follows:
A: solubility was good; and
D: solubility was poor to make preparation impossible.

**Table 1**

| | | | | Test Example | | | | | |
|---|---|---|---|---|---|---|---|---|---|
| | | | | 1-1 | 1-2 | 1-3 | 1-4 | 1-5 | 1-6 |
| | Monomer A1 | Monomer B1 | Monomer C1 | | | | | | |
| Copolymer 1-1 | 35.0 | 5.0 | 60.0 | 10.0 | - | - | - | - | - |
| Copolymer 1-2 | 100.0 | - | - | - | 10.0 | - | - | - | - |
| Copolymer 1-3 | 50.0 | 50.0 | - | - | - | 10.0 | - | - | - |
| Copolymer 1-4 | 50.0 | - | 50.0 | - | - | - | 10.0 | - | - |
| Trimethylsiloxy Silicate | | | | - | - | - | - | 10.0 | - |
| Non-aqueous Polymer Emulsion^{*1} | | | | - | - | - | - | - | 10.0 |
| Microcrystalline Wax | | | | 1.0 | 1.0 | 1.0 | 1.0 | 1.0 | 1.0 |
| Paraffin | | | | 11.0 | 11.0 | 11.0 | 11.0 | 11.0 | 11.0 |
| Candelilla wax | | | | 3.0 | 3.0 | 3.0 | 3.0 | 3.0 | 3.0 |
| Decamethyl Cyclopentasiloxane | | | | Balance | Balance | Balance | Balance | Balance | Balance |
| Polyoxyethylene-modified Silicone | | | | 3.0 | 3.0 | 3.0 | 3.0 | 3.0 | 3.0 |
| Methylphenyl polysiloxane | | | | 3.0 | 3.0 | 3.0 | 3.0 | 3.0 | 3.0 |
| Tri(hydrogenated rosin-isostearic acid)glyceryl | | | | 5.0 | 5.0 | 5.0 | 5.0 | 5.0 | 5.0 |
| Silica*² | | | | 1.0 | 1.0 | 1.0 | 1.0 | 1.0 | 1.0 |
| Calcium Hydrogen Phosphate | | | | 1.0 | 1.0 | 1.0 | 1.0 | 1.0 | 1.0 |
| Pigment | | | | 5.0 | 5.0 | 5.0 | 5.0 | 5.0 | 5.0 |
| Perfume | | | | q.s. | q.s. | q.s. | q.s. | q.s. | q.s. |
| (1) Makeup retaining performance | | | | A | - | - | A | C | B |
| (2) Film-like Feeling | | | | A | - | - | C | D | B |
| (3) Luster | | | | A | - | - | B | C | B |
| (4) Solubility in formulation | | | | A | D | D | A | A | A |

| | | | | | | | | | |
|---|---|---|---|---|---|---|---|---|---|
| * 1 Added were 15% of methyl methacrylate monomer, 25% of ethyl acrylate monomer, 0.1% of a polymerization initiator, 5% of a dispersion stabilizer (dimethylpolysiloxane graft copolymer, molecular weight 150,000) and 54.9% of a dispersion medium (decamethyl cyclopentasiloxane), and the mixture was allowed to polymerize by stirring for 10 hours at 20°C. Then, after removing the monomer in vacuum, a non-aqueous polymer emulsion (milky white, average particle diameter of dispersed polymer: 1 µm) was obtained by cooling to 25°C. | | | | | | | | | |
| *2 Aerosil R972 (manufactured by Degussa) | | | | | | | | | |

Table 1 shows that the lipstick in Test Example 1-1, in which copolymer 1-1 was blended as the film-forming component, showed a quite excellent makeup retaining effect with a remarkable improvement of film-like feeling.

On the contrary, the composition in Test Example 1-2, in which copolymer 1-2 comprising monomer A only was blended, and the composition in Test Example 1-3, in which copolymer 1-3 comprising monomers A and B was blended, could not be formed into preparations due to poor solubility in formulation. The composition in Test Example 1-4, in which copolymer 1-4 comprising monomers A and C was blended, were poor in film-like feeling on the lip. The composition in Test Example 1-5, in which trimethylsiloxy silicate known as a generally used film-forming component was blended as the film-forming component, was poor in retaining performance, film-like feeling and luster. The composition in Test Example 1-6, in which a non-aqueous polymer emulsion was blended, was not sufficient in retaining performance, film-like feeling and luster, although these properties had been improved to some extent.

### Monomer proportion of copolymer

Subsequently, the inventors prepared various copolymers having different contents of monomer A according to the production method described above in order to investigate favorable monomer proportion of the copolymer, and evaluated the lipstick in which each copolymer was blended. The blend composition of the lipstick in each test example, and results of evaluation thereof are shown in table 2. The evaluation criteria are the same as in the foregoing test.

**Table 2**

| | | | | Test Example | | | | | | |
|---|---|---|---|---|---|---|---|---|---|---|
| | | | | 1-7 | 1-8 | 1-9 | 1-10 | 1-11 | 1-12 | 1-13 |
| | Monomer A1 | Monomer B1 | Monomer C1 | | | | | | | |
| Copolymer 1-5 | - | 5.0 | 95.0 | 10.0 | - | - | - | - | - | - |
| Copolymer 1-6 | 10.0 | 5.0 | 85.0 | - | 10.0 | - | - | - | - | - |
| Copolymer 1-7 | 15.0 | 5.0 | 80.0 | - | - | 10.0 | - | - | - | - |
| Copolymer 1-8 | 20.0 | 5.0 | 75.0 | - | - | - | 10.0 | - | - | - |
| Copolymer 1-9 | 25.0 | 5.0 | 70.0 | - | - | - | - | 10.0 | - | - |
| Copolymer 1-10 | 30.0 | 5.0 | 65.0 | - | - | - | - | - | 10.0 | - |
| Copolymer 1-11 | 40.0 | 5.0 | 55.0 | - | - | - | - | - | - | 10.0 |
| Microcrystalline Wax | | | | 1.0 | 1.0 | 1.0 | 1.0 | 1.0 | 1.0 | 1.0 |
| Paraffin | | | | 11.0 | 11.0 | 11.0 | 11.0 | 11.0 | 11.0 | 11.0 |
| Candelilla wax | | | | 3.0 | 3.0 | 3.0 | 3.0 | 3.0 | 3.0 | 3.0 |
| Decamethyl Cyclopentasiloxane | | | | Balance | Balance | Balance | Balance | Balance | Balance | Balance |
| Polyoxyethylene-modified Silicone | | | | 3.0 | 3.0 | 3.0 | 3.0 | 3.0 | 3.0 | 3.0 |
| Methylphenyl polysiloxane | | | | 3.0 | 3.0 | 3.0 | 3.0 | 3.0 | 3.0 | 3.0 |
| Tri(hydrogenated rosin-isostearic cid)glyceryl | | | | 5.0 | 5.0 | 5.0 | 5.0 | 5.0 | 5.0 | 5.0 |
| Silica*¹ | | | | 1.0 | 1.0 | 1.0 | 1.0 | 1.0 | 1.0 | 1.0 |
| Calcium Hydrogen Phosphate | | | | 1.0 | 1.0 | 1.0 | 1.0 | 1.0 | 1.0 | 1.0 |
| Pigment | | | | 5.0 | 5.0 | 5.0 | 5.0 | 5.0 | 5.0 | 5.0 |
| Perfume | | | | q.s. | q.s. | q.s. | q.s. | q.s. | q.s. | q.s. |
| (1) Makeup retaining performance | | | | D | D | C | A | A | A | A |
| (2) Film-like Feeling | | | | A | A | A | A | A | A | A |
| (3) Luster | | | | B | B | B | A | A | A | A |
| (4) Solubility in formulation | | | | A | A | A | A | A | A | A |

| | | | | | | | | | | |
|---|---|---|---|---|---|---|---|---|---|---|
| *1: Aerosil R972 (manufactured by Degussa) | | | | | | | | | | |

Table 2 shows the composition in Test Example 1-7, in which copolymer 1-5 containing no monomer A at all was used, was quite poor in makeup retaining performance. On the other hand, while it was shown that retaining performance tends to be improved as the proportion of monomer A increases in the copolymer, the composition in Test Examples 1-8 and 1-9, in which copolymer 1-6 and 1-7 having a proportion of monomer A of less than 20% by mass, respectively, were used, could be hardly recognized to be sufficient in makeup retaining performance. On the contrary, the compositions in Test Examples 1-10 to 1-13, in which copolymer 1-8 to 1-11 having a proportion of monomer A of 20% by mass or more, respectively, were used, were shown to be quite excellent in both retaining performance and film-like feeling.

These results indicate that the proportion of monomer A in the copolymer should be 20% by mass or more in the lip makeup cosmetic according to the invention.

### Concentration of copolymer in lip makeup cosmetic

Subsequently, the inventors prepared lipsticks having different amounts of blending of the copolymer in order to investigate the favorable concentration of the copolymer to be blended in the lip makeup cosmetic, and evaluated the cosmetic. The blending compositions of the lipsticks in each test examples, and the results of evaluation are listed in Table 3. The evaluation criteria are the same as in the foregoing tests.

**Table 3**

| | Test Example | | | | | | | |
|---|---|---|---|---|---|---|---|---|
| | 1-13 | 1-14 | 1-15 | 1-1 | 1-16 | 1-17 | 1-18 | 1-19 |
| Copolymer 1-1 | - | 0.1 | 1.0 | 10.0 | 15.0 | 20.0 | 25.0 | 30.0 |
| Microcrystalline Wax | 1.0 | 1.0 | 1.0 | 1.0 | 1.0 | 1.0 | 1.0 | 1.0 |
| Paraffin | 11.0 | 11.0 | 11.0 | 11.0 | 11.0 | 11.0 | 11.0 | 11.0 |
| Candelilla wax | 3.0 | 3.0 | 3.0 | 3.0 | 3.0 | 3.0 | 3.0 | 3.0 |
| Decamethyl Cyclopentasiloxane | Balance | Balance | Balance | Balance | Balance | Balance | Balance | Balance |
| Polyoxyethylene-modified Silicone | 3.0 | 3.0 | 3.0 | 3.0 | 3.0 | 3.0 | 3.0 | 3.0 |
| Methylphenyl polysiloxane | 3.0 | 3.0 | 3.0 | 3.0 | 3.0 | 3.0 | 3.0 | 3.0 |
| Tri(hydrogenated rosin-isostearic acid) glyceryl | 5.0 | 5.0 | 5.0 | 5.0 | 5.0 | 5.0 | 5.0 | 5.0 |
| Silica*¹ | 1.0 | 1.0 | 1.0 | 1.0 | 1.0 | 1.0 | 1.0 | 1.0 |
| Calcium Hydrogen Phosphate | 1.0 | 1.0 | 1.0 | 1.0 | 1.0 | 1.0 | 1.0 | 1.0 |
| Pigment | 5.0 | 5.0 | 5.0 | 5.0 | 5.0 | 5.0 | 5.0 | 5.0 |
| Perfume | q.s. | q.s | q.s | q.s | q.s | q.s | q.s | q.s |
| (1) Makeup retaining performance | D | D | B | A | A | A | A | A |
| (2) Film-like Feeling | A | A | A | A | A | A | B | C |
| (3) Luster | B | B | B | A | A | A | A | A |
| (4) Solubility in formulation | A | A | A | A | A | A | A | A |

| | | | | | | | | |
|---|---|---|---|---|---|---|---|---|
| * 1: Aerosil R972 (manufactured by Degussa) | | | | | | | | |

Table 3 shows that the cosmetic in Test Example 1-15, in which copolymer 1-1 was blended in an amount of about 1% by mass, exhibited an improving effect in makeup retaining, film-like feeling and luster. On the contrary, the cosmetic in Test Example 1-19, in which copolymer 1-1 was blended in an amount of about 30% by mass, tend to be poor in film-like feeling. These results show that it may be preferable to blend 1 to 25% by mass of the copolymer in the lip makeup cosmetic according to the invention.

While examples of the lip makeup cosmetic and other examples are described hereinafter, the invention is by no means restricted to these examples.

### Copolymer 1-12

Charged in a glass flask equipped with a stirrer, thermometer and reflux condenser were 35 parts by mass of methyl methacrylate (monomer A1) represented by formula (17), 15 parts by mass of polyoxyethylene ether methacrylate (monomer B1) represented by formula (19), 50 parts by mass of methacryloxypropyl polydimethylsiloxne (monomer C1) represented by formula (20), 120 parts by mass of isopropanol, and 4 parts by mass of dimethyl-2,2'-azobis(2-methyl propionate), and the mixture was allowed to react for 10 hours by heating at 80°C in a nitrogen stream. Then, the volatile component was removed by evaporation to obtain copolymer 1-12.

### Copolymer 1-13

Charged in a glass flask equipped with a stirrer, thermometer and reflux condenser were 35 parts by mass of methyl methacrylate (monomer A1) represented by formula (17), 15 parts by mass of 2-ethylhexyl acrylate (monomer A2), 5 parts by mass of polyoxyethylene ether methacrylate (monomer B1) represented by formula (19), 45 parts by mass of methacryloxypropyl polydimethylsiloxane (monomer C2) represented by formula (21), 120 parts by mass of isopropanol, and 4 parts by mass of dimethyl-2,2'-azobis(2-methyl propionate), and the mixture was allowed to react for 10 hours by heating at 80°C in a nitrogen stream. Then, the volatile component was removed by evaporation to obtain copolymer 1-13.

### Example 1-1

| Lipstick | % by Mass |
|---|---|
| Microcrystalline Wax | 1.0 |
| Paraffin | 11.0 |
| Candellila Wax | 3.0 |
| Decamethyl Cyclopentasiloxane (y=5 in Formula (5)) | Balance |
| Dimethyl Polysiloxane (X=2 in Formula (4) | 20.0 |
| Alkyl-modified silicone (R⁹=C₈H₁₇ in Formula (6)) | 10.0 |
| Copolymer 1-12 | 10.0 |
| Polyoxyethylene-modified silicone | 3.0 |
| Methylphenyl Polysiloxane | 5.0 |
| Fluorine-modified methylphenyl silicone (R¹¹=Ph, R¹²=CH₃, R¹³=C₈F₁₇, b=2, q=2, r=2, s=3 in Formula 8)) | 10.0 |
| Allcoxy-modified silicone (R¹⁴=C₁₈H₃₇, t=5, u=3 in Formula (9)) | 2.0 |
| 2-Ethylhexyl Paramethoxycinnamate | 1.0 |
| Silica (Aerosil R972; manufactured by Degussa) | 1.0 |
| Pigment | 5.0 |
| Perfume | q.s. |

### (Production Method)

After mixing and dissolving all the components at 95°C, the liquid was flowed into a vessel to obtain a lipstick after solidifying by cooling.

The lipstick in Example 1-1 was excellent in makeup retaining performance without any film-like feeling while being excellent in luster.

### Example 1-2

| Lipstick | % by Mass |
|---|---|
| Microcrystalline Wax | 0.5 |
| Candellila Wax | 1.0 |
| Synthetic Wax (FNP-0090, manufactured by Nippon Seiro) | 8.0 |
| Decamethyl Cyclopentasiloxane | Balance |
| Dimethyl Polysiloxane (X=2 in Formula (4)) | 20.0 |
| Alkyl-modified Silicone (R⁹=C₈H₁₇ in Formula (6)) | 10.0 |
| Copolymer 1-1 | 10.0 |
| Polyoxyethylene-modified Silicone | 3.0 |
| Methylphenyl Polysiloxane | 5.0 |
| Fluorine-modified Methylphenyl Silicone (R¹¹=Ph, R¹²=CH₃, R¹³=C₈F₁₇, b=2, q=2, r=2, s=3 in Formula (8)) | 10.0 |
| Alkoxy-modified Silicone (R¹⁴=C₁₈H₃₇, t=5, u=3 in Formula (9)) | 2.0 |
| 2-Ethylhexyl Paramethoxycinnamate | 1.0 |
| Silica (Aerosil R972: manufactured by Degussa) | 1.0 |
| Pigment | 5.0 |
| Perfume | q.s. |

### (Production Method)

After mixing and dissolving all the components at 95°C, the liquid was flowed into a vessel to obtain a lipstick after solidifying by cooling.

The lipstick in Example 1-2 was excellent in makeup retaining performance without any film-like feeling while being excellent in luster.

### Example 1-3

| Lipstick | % by Mass |
|---|---|
| Microcrystalline Wax | 2.0 |
| Paraffin | 1.0 |
| Polyethylene Wax (Average Molecular Weight 500) | 10.0 |
| Carnauba Wax | 1.0 |
| Decamethyl Cyclopentasiloxane | Balance |
| Dimethyl Polysiloxane (X=2 in Formula (4)) | 30.0 |
| Copolymer 1-13 | 10.0 |
| Polyoxyethylene-modified Silicone | 3.0 |
| Methylphenyl Polysiloxane | 5.0 |
| Fluorine-modified Dimethyl Silicone (R¹⁰=C₈F₁₇, a=11, o=11, p=75 in Formula (7)) | 5.0 |
| Alkoxy-modified Silicone (R¹⁴=C₁₈H₃₇, t=5, u=3 in Formula (9)) | 2.0 |
| Alkyl-modified Silicone (R¹⁵=C₁₈H₃₇, v=5, w=3 in Formula (10)) | 3.0 |
| Tri(Hydrogenated Rosin-Isostearic Acid)Glyceryl | 5.0 |
| Silica (Aerosil R972: Manufactured by Degussa) | 1.0 |
| Pigment | 5.0 |
| Perfume | q.s. |

### (Production Method)

After mixing and dissolving all the components at 95°C, the liquid was flowed into a vessel to obtain a lipstick after solidifying by cooling.

The lipstick in Example 1-3 was excellent in makeup retaining performance without any film-like feeling while being excellent in luster.

### Example 1-4

| Emulsified Rouge | % by Mass |
|---|---|
| Microcrystalline Wax | 1.0 |
| Paraffin | 12.0 |
| Candellila Wax | 2.0 |
| Decamethyl Cyclopentasiloxane | Balance |
| Polymer 1-13 | 10.0 |
| Polyoxyethylene-modified Silicone | 3.0 |
| Methylphenyl Polysiloxane | 5.0 |
| Tri(Hydrogenated Rosin-Isostearic Acid)Glyceryl | 5.0 |
| Silica (Aerosil R972: Manufactured by Degussa) | 1.0 |
| Pigment | 5.0 |
| Perfume | q.s. |
| Laponite | 0.4 |
| Glycerin | 0.5 |
| Water | 0.1 |

### (Production Method)

After mixing and dissolving the components except aqueous phase parts (laponite, glycerin and water) at 95°C, the liquid was emulsified by adding the aqueous phase parts with stirring, and the emulsion was flowed into a vessel to obtain an emulsified rouge after solidifying by cooling.

The rouge in Example 1-4 was excellent in retaining performance without any film-like feeling while being excellent in luster.

### Example 1-5

| Liquid Rouge | % by Mass |
|---|---|
| Microcrystalline Wax | 0.2 |
| Paraffin | 2.0 |
| Decamethyl Cyclopentasiloxane | Balance |
| Copolymer 1-13 | 15.0 |
| Polyoxyethylene-modified Silicone | 3.0 |
| Methylphenyl Polysiloxane | 5.0 |
| Fluorine-modified Methylphenyl Silicone (R¹¹=Ph, R¹²=CH₃, R¹³=C₈F₁₇, b=3, q=6, r=20, s=100 in Formula (8)) | 20.0 |
| Silica (Aerosil R972: manufactured by Degussa) | 2.0 |
| Pigment | 5.0 |
| Perfume | q.s. |
| Laponite | 0.4 |
| Glycerin | 0.5 |
| Water | 0.1 |

### (Production Method)

After mixing and dissolving the components except aqueous phase parts (laponite, glycerin and water) at 95°C, the liquid was emulsified by adding the aqueous phase parts with stirring, and the emulsion was flowed into a vessel to obtain an emulsified rouge after solidifying by cooling.

The rouge in Example 1-5 was excellent in retaining performance without any film-like feeling while being excellent in luster.

### 2. Eyelashes Makeup Cosmetic

### Blending of copolymer

The inventors prepared each copolymer according to the foregoing production examples, and a W/O mascara in which the copolymer of the invention was blended as a film-forming component was compared with a W/O mascara in which a conventional film-forming component was blended. The monomer proportion of the copolymer used in each test and the composition of blending of the W/O mascara, and the results of evaluation thereof are shown in Table 4 below. The evaluation criteria are as follows.

### (1) Curling effect

The curling effect of the mascara in each example in practical uses was evaluated by 10 special panelists. The evaluation criteria are as follows:
A: 8 or more panelists evaluated the curling effect to be good;
B: 6 or more and less than 8 panelists evaluated the curling effect to be good;
C: 3 or more and less than 6 panelists evaluated the curling effect to be good; and
D: less than 3 panelists evaluated the curling effect to be good.

### (2) Makeup retaining performance

Retaining performance of the mascara in each example in practical uses was evaluated by 10 special panelists. The evaluation criteria are as follows:
A: 8 or more panelists evaluated makeup retaining performance to be good;
B: 6 or more and less than 8 panelists evaluated retaining performance to be good;
C: 3 or more and less than 6 panelists evaluated retaining performance to be good; and
D: less than 3 panelists evaluated retaining performance to be good.

### (3) Film-like feeling

Film-like feeling of the mascara in each example in practical uses was evaluated by 10 special panelists. The evaluation criteria are as follows:
A: 8 or more panelists recognized no film-like feeling;
B: 6 or more and less than 8 panelists recognized no film-like feeling;
C: 3 or more and less than 6 panelists recognized no film-like feeling; and
D: less than 3 panelists evaluated recognized no film-like feeling.

### (4) Temporal stability (after 1 month)

The mascara in each example was stored at room temperature for 1 month, and appearance and properties were visually observed:
A: there were no changes in appearance and properties;
B: there were changes in appearance and properties such as slight separation or precipitation;
C: there were changes in appearance and properties such as appreciable separation or precipitation; and
D: there were changes in appearance and properties such as apparent separation or precipitation.

**Table 4**

| W/O mascara | | | | | | | | | |
|---|---|---|---|---|---|---|---|---|---|
| | | | | Test Example | | | | | |
| | | | | 2-1 | 2-2 | 2-3 | 2-4 | 2-5 | 2-6 |
| | Monomer A1 | Monomer B1 | Monomer C1 | | | | | | |
| Copolymer 2-1 | 35.0 | 5.0 | 60.0 | 10.0 | - | - | - | - | - |
| Copolymer 2-2 | 100.0 | - | - | - | 10.0 | - | - | - | - |
| Copolymer 2-3 | 50.0 | 50.0 | - | - | - | 10.0 | - | - | - |
| Copolymer 2-4 | 50.0 | - | 50.0 | - | - | - | 10.0 | - | - |
| Hydroxyethyl Cellulose | | | | - | - | - | - | 10.0 | - |
| Trimethylsiloxy Silicate | | | | - | - | - | - | - | 10.0 |
| Light Isoparaffin | | | | 7.0 | 7.0 | 7.0 | 7.0 | 7.0 | 7.0 |
| Dimethyl Polysiloxane | | | | 2.0 | 2.0 | 2.0 | 2.0 | 2.0 | 2.0 |
| Decamethyl Cyclopentasiloxane | | | | 10.0 | 10.0 | 10.0 | 10.0 | 10.0 | 10.0 |
| Methyl Polysiloxane Emulsion | | | | q.s. | q.s. | q.s. | q.s. | q.s. | q.s. |
| 1,3-Butyleneglycol | | | | 4.0 | 4.0 | 4.0 | 4.0 | 4.0 | 4.0 |
| Polyethyleneglycol Dioleate | | | | 2.0 | 2.0 | 2.0 | 2.0 | 2.0 | 2.0 |
| Diglyceryl Diisostearate | | | | 2.0 | 2.0 | 2.0 | 2.0 | 2.0 | 2.0 |
| Sodium Hydrogen Carbonate | | | | 0.2 | 0.2 | 0.2 | 0.2 | 0.2 | 0.2 |
| DL-α-tocopherol Acetate | | | | 0.1 | 0.1 | 0.1 | 0.1 | 0.1 | 0.1 |
| Paraoxybenzoic Acid Ester | | | | q.s. | q.s. | q.s. | q.s. | q.s. | q.s. |
| Sodium Dehydroacetate | | | | q.s. | q.s. | q.s. | q.s. | q.s. | q.s. |
| Iron Oxide Black | | | | 7.0 | 7.0 | 7.0 | 7.0 | 7.0 | 7.0 |
| Seaweed Extract | | | | 0.1 | 0.1 | 0.1 | 0.1 | 0.1 | 0.1 |
| Bentonite | | | | 1.0 | 1.0 | 1.0 | 1.0 | 1.0 | 1.0 |
| Dimethylstearyl Ammonium Hectorite | | | | 6.0 | 6.0 | 6.0 | 6.0 | 6.0 | 6.0 |
| Polyvinyl Acetate Emulsion | | | | 30.0 | 30.0 | 30.0 | 30.0 | 30.0 | 30.0 |
| Purified Water | | | | Balance | Balance | Balance | Balance | Balance | Balance |
| (1) Curling Effect | | | | A | A | D | C | C | B |
| (2) Makeup retaining performance | | | | A | B | D | B | D | A |
| (3) Film-like Feeling | | | | A | D | B | B | C | D |
| (4) Temporal Stability (after 1 month) | | | | A | D | D | A | A | A |

Table 4 shows that the W/O mascara in Test Example 2-1, in which copolymer 2-1 was blended as the film-forming component, showed a quite excellent curling effect and makeup retaining effect with a remarkable improvement of film-like feeling.

On the contrary, the composition in Test Example 2-2, in which copolymer 2-2 comprising monomer A only was blended, was poor in film-like feeling, temporal stability. And the composition in Test Example 2-3, in which copolymer 2-3 comprising monomers A and B was blended, was poor in the curling effect, makeup retaining performance and temporal stability. The composition in Test Example 2-4, in which copolymer 2-4 comprising monomers A and C was blended, could not be obtained sufficient curling effect. The composition in Test Example 2-5, in which hydroxyethyl cellulose known as a generally used film-forming component was blended, was poor in retaining performance, curling effect and film-like feeling. The composition in Test Example 2-6, in which trimethylsiloxy silicate was blended, could be hardly recognized to be sufficient in film-like feeling.

Subsequently, the inventors prepared O/W mascara and oil-based mascara using monomer 2-1, and compared with the mascara blending general film-forming component. The blend composition of the mascara in each test example, and results of evaluation thereof are shown in table 5 and 6. The evaluation criteria are the same as in the foregoing test.

**Table 5**

| O/W Mascara | | | |
|---|---|---|---|
| | Test Example | | |
| | 2-7 | 2-8 | 2-9 |
| Copolymer 2-1 | 5.0 | - | - |
| Hydroxyethyl Cellulose | - | 5.0 | - |
| Trimethylsiloxy Silicate | - | - | 5.0 |
| Light Isoparaffin | 6.0 | 6.0 | 6.0 |
| Dimethyl Polysiloxane | 1.0 | 1.0 | 1.0 |
| Decamethyl Cyclopentasiloxane | 5.0 | 5.0 | 5.0 |
| Methyl Polysiloxane Emulsion | q.s. | q.s. | q.s. |
| Isopropanol | 3.0 | 3.0 | 3.0 |
| 1,3-Butyleneglycol | 6.0 | 6.0 | 6.0 |
| Polyoxyethylene Hardened Caster Oil | 1.0 | 1.0 | 1.0 |
| Sucrose Fatty Acid Ester | 0.6 | 0.6 | 0.6 |
| Diglyceryl Diisostearate | 1.0 | 1.0 | 1.0 |
| Sodium Hydrogen Carbonate | 0.01 | 0.01 | 0.01 |
| DL-α-Tocopherol Acetate | 0.1 | 0.1 | 0.1 |
| Sodium Acetylated Hyaluronate | 0.1 | 0.1 | 0.1 |
| Paraoxybenzoic Acid Ester | q.s. | q.s. | q.s. |
| Phenoxyethanol | 0.3 | 0.3 | 0.3 |
| Iron Oxide Black | 8.0 | 8.0 | 8.0 |
| Bentonite | 1.0 | 1.0 | 1.0 |
| Dimethylstearyl Ammonium Hectorite | 4.0 | 4.0 | 4.0 |
| Polyvinyl alcohol | 4.0 | 4.0 | 4.0 |
| Alkyl Acrylate Copolymer Emulsion | 12.0 | 12.0 | 12.0 |
| Polyvinyl Acetate Emulsion | 12.0 | 12.0 | 12.0 |
| Nylon Fiber | 6.0 | 6.0 | 6.0 |
| Purified Water | Balance | Balance | Balance |
| Silicic Acid Anhydride | 0.5 | 0.5 | 0.5 |
| Titanium Oxide | 1.0 | 1.0 | 1.0 |
| Perfume | q.s. | q.s. | q.s. |
| (1) Curling Effect | A | C | B |
| (2) Makeup Retaining performance | A | D | A |
| (3) Film-like Feeling | A | B | C |
| (4) Temporal Stability (after 1 month) | A | A | A |

**Table 6**

| Oil-based Mascara | | | |
|---|---|---|---|
| | Test Example | | |
| | 2-10 | 2-11 | 2-12 |
| Copolymer 2-1 | 15.0 | - | - |
| Hydroxyethyl Cellulose | - | 15.0 | - |
| Trimethylsiloxy Silicate | - | - | 15.0 |
| Light Isoparaffin | Balance | Balance | Balance |
| Decamethyl Cyclopentasiloxane | 20.0 | 20.0 | 20.0 |
| Microcrystalline Wax | 17.0 | 17.0 | 17.0 |
| Iron Oxide Black | 5.0 | 5.0 | 5.0 |
| Dextrin Fatty Acid Ester | 11.0 | 11.0 | 11.0 |
| (1) Curling Effect | A | C | A |
| (2) Makeup Retaining Performance | A | D | B |
| (3) Film-like Feeling | A | C | C |
| (4) Temporal Stability (after 1 month) | A | A | A |

Table 5 and 6 shows that the O/W mascara in Test Example 2-7 and the oil-based mascara n Test Example 2-10, in which copolymer 2-1 was blended as the film-forming component, showed a quite excellent curling effect and makeup retaining effect with a remarkable improvement of film-like feeling.

### Monomer proportion of copolymer

Subsequently, the inventors prepared various copolymers having different contents of monomer A according to the production method described above in order to investigate favorable monomer proportion of the copolymer, and evaluated the mascara in which each copolymer was blended. The blend composition of the mascara in each test example, and results of evaluation thereof are shown in table 7. The evaluation criteria are the same as in the foregoing test.

**Table 7**

| | | | | Test Example | | | | | | |
|---|---|---|---|---|---|---|---|---|---|---|
| | | | | 2-13 | 2-14 | 2-15 | 2-16 | 2-17 | 2-18 | 2-19 |
| | Monomer A1 | Monomer B1 | Monomer C1 | | | | | | | |
| Copolymer 2-5 | - | 5.0 | 95.0 | 10.0 | - | - | - | - | - | - |
| Copolymer 2-6 | 10.0 | 5.0 | 85.0 | - | 10.0 | - | - | - | - | - |
| Copolymer 2-7 | 15.0 | 5.0 | 80.0 | - | - | 10.0 | - | - | - | - |
| Copolymer 2-8 | 20.0 | 5.0 | 75.0 | - | - | - | 10.0 | - | - | - |
| Copolymer 2-9 | 25.0 | 5.0 | 70.0 | - | - | - | - | 10.0 | - | - |
| Copolymer 2-10 | 30.0 | 5.0 | 65.0 | - | - | - | - | - | 10.0 | - |
| Copolymer 2-11 | 40.0 | 5.0 | 55.0 | - | - | - | - | - | - | 10.0 |
| Light Isoparaffin | | | | 7.0 | 7.0 | 7.0 | 7.0 | 7.0 | 7.0 | 7.0 |
| Dimethyl Polysiloxane | | | | 2.0 | 2.0 | 2.0 | 2.0 | 2.0 | 2.0 | 2.0 |
| Decamethyl Cyclopentasiloxane | | | | 10.0 | 10.0 | 10.0 | 10.0 | 10.0 | 10.0 | 10.0 |
| Methyl Polysiloxane Emulsion | | | | q.s. | q.s. | q.s. | q.s. | q.s. | q.s. | q.s. |
| 1,3-butyleneglycol | | | | 4.0 | 4.0 | 4.0 | 4.0 | 4.0 | 4.0 | 4.0 |
| Polyethyleneglycol Dioleate | | | | 2.0 | 2.0 | 2.0 | 2.0 | 2.0 | 2.0 | 2.0 |
| Diglyceryl Diisostearate | | | | 2.0 | 2.0 | 2.0 | 2.0 | 2.0 | 2.0 | 2.0 |
| Sodium Hydrogen Carbonate | | | | 0.2 | 0.2 | 0.2 | 0.2 | 0.2 | 0.2 | 0.2 |
| DL-α-toeopherol Acetate | | | | 0.1 | 0.1 | 0.1 | 0.1 | 0.1 | 0.1 | 0.1 |
| Paraoxybenzoic Acid Ester | | | | q.s. | q.s. | q.s. | q.s. | q.s. | q.s. | q.s. |
| Sodium Dehydroacetate | | | | q.s. | q.s. | q.s. | q.s. | q.s. | q.s. | q.s. |
| Iron Oxide Black | | | | 7.0 | 7.0 | 7.0 | 7.0 | 7.0 | 7.0 | 7.0 |
| Seaweed Extract | | | | 0.1 | 0.1 | 0.1 | 0.1 | 0.1 | 0.1 | 0.1 |
| Bentonite | | | | 1.0 | 1.0 | 1.0 | 1.0 | 1.0 | 1.0 | 1.0 |
| Dimethylstearyl Ammonium Hectorite | | | | 6.0 | 6.0 | 6.0 | 6.0 | 6.0 | 6.0 | 6.0 |
| Polyvinyl Acetate Emulsion | | | | 30.0 | 30.0 | 30.0 | 30.0 | 30.0 | 30.0 | 30.0 |
| Purified Water | | | | Balance | Balance | Balance | Balance | Balance | Balance | Balance |
| (1) Curling Effect | | | | D | D | C | A | A | A | A |
| (2) Makeup Retaining Performance | | | | D | C | B | A | A | A | A |
| (3) Film-like Feeling | | | | A | A | A | A | A | A | A |
| (4) Temporal Stability (after 1 month) | | | | A | A | A | A | A | A | A |

Table 7 shows the composition in Test Example 2-13, in which copolymer 2-5 comprising no monomer A at all was used, was quite poor in curling effect and makeup retaining performance. On the other hand, while it was shown that curling effect and retaining performance tends to be improved as the proportion of monomer A increases in the copolymer, the composition in Test Examples 2-14 and 2-15, in which copolymer 2-6 and 2-7 having a proportion of monomer A of less than 20% by mass, respectively, were used, could be hardly recognized to be sufficient in curling effect and makeup retaining performance. On the contrary, the compositions in Test Examples 2-16 to 2-19, in which copolymer 2-8 to 2-11 having a proportion of monomer A of 20% by mass or more, respectively, were used, were shown to be excellent in curling effect and makeup retaining performance as well as film-like feeling.

These results indicate that the proportion of monomer A in the copolymer should be 20% by mass or more in the eyelashes makeup cosmetic according to the invention.

### Concentration of copolymer in eyelashes makeup cosmetic

Subsequently, the inventors prepared mascaras having different amounts of blending of the copolymer in order to investigate the favorable concentration of the copolymer to be blended in the eyelashes makeup cosmetic, and evaluated the cosmetic. The blending compositions of the mascara in each test examples, and the results of evaluation are listed in Table 8. The evaluation criteria are the same as in the foregoing tests.

**Table 8**

| | Test Example | | | | | | | |
|---|---|---|---|---|---|---|---|---|
| | 2-20 | 2-21 | 2-22 | 2-23 | 2-1 | 2-24 | 2-25 | 2-26 |
| Copolymer 2-1 | - | 0.1 | 1.0 | 3.0 | 10.0 | 25.0 | 30.0 | 40.0 |
| Light Isoparaffin | 7.0 | 7.0 | 7.0 | 7.0 | 7.0 | 7.0 | 7.0 | 7.0 |
| Dimethyl Polysiloxane | 2.0 | 2.0 | 2.0 | 2.0 | 2.0 | 2.0 | 2.0 | 2.0 |
| Decamethyl Cyclopentasiloxane | 10.0 | 10.0 | 10.0 | 10.0 | 10.0 | 10.0 | 10.0 | 10.0 |
| Methyl Polysiloxane Emulsion | q.s. | q.s. | q.s. | q.s. | q.s. | q.s. | q.s. | q.s. |
| 1,3-butyleneglycol | 4.0 | 4.0 | 4.0 | 4.0 | 4.0 | 4.0 | 4.0 | 4.0 |
| Polyethyleneglycol Dioleate | 2.0 | 2.0 | 2.0 | 2.0 | 2.0 | 2.0 | 2.0 | 2.0 |
| Diglyceryl Diisostearate | 2.0 | 2.0 | 2.0 | 2.0 | 2.0 | 2.0 | 2.0 | 2.0 |
| Sodium Hydrogen Carbonate | 0.2 | 0.2 | 0.2 | 0.2 | 0.2 | 0.2 | 0.2 | 0.2 |
| DL-α-tocopherol Acetate | 0.1 | 0.1 | 0.1 | 0.1 | 0.1 | 0.1 | 0.1 | 0.1 |
| Paraoxybenzoic Acid Ester | q.s. | q.s. | q.s. | q.s. | q.s. | q.s. | q.s. | q.s. |
| Sodium Dehydroacetate | q.s. | q.s. | q.s. | q.s. | q.s. | q.s. | q.s. | q.s. |
| Iron Oxide Black | 7.0 | 7.0 | 7.0 | 7.0 | 7.0 | 7.0 | 7.0 | 7.0 |
| Seaweed Extract | 0.1 | 0.1 | 0.1 | 0.1 | 0.1 | 0.1 | 0.1 | 0.1 |
| Bentonite | 1.0 | 1.0 | 1.0 | 1.0 | 1.0 | 1.0 | 1.0 | 1.0 |
| Dimethylstearyl Ammonium Hectorite | 6.0 | 6.0 | 6.0 | 6.0 | 6.0 | 6.0 | 6.0 | 6.0 |
| Polyvinyl Acetate Emulsion | 30.0 | 30.0 | 30.0 | 30.0 | 30.0 | 30.0 | 30.0 | 30.0 |
| Purified Water | Balance | Balance | Balance | Balance | Balance | Balance | Balance | Balance |
| (1) Curling Effect | D | C | B | A | A | A | A | A |
| (2) Makeup Retaining Performance | D | C | A | A | A | A | A | A |
| (3) Film-like Feeling | C | B | A | A | A | A | B | C |
| (4) Temporal Stability (after 1 month) | A | A | A | A | A | A | A | A |

Table 8 shows that the composition in Test Example 2-21, in which copolymer 2-1 was blended in an amount of about 1% by mass, exhibited excellent curling effect and makeup retaining with improving effect in film-like feeling. Also, Test Example 2-25, in which copolymer 2-1 was blended in an amount of about 30% by mass, exhibited excellent effect all of curling effect, makeup retaining performance, and film-like feeling. On the contrary, the composition in Test Example 2-20, in which copolymer 2-1 was blended in an amount of about 0.1 % by mass, tend to be poor in makeup retaining and film-like feeling. Test Example 2-26, in which copolymer 2-1 was blended in an amount of about 40% by mass, tend to be poor in film-like feeling.

These results show that it may be preferable to blend 1 to 30% by mass of the copolymer in the eyelashes makeup cosmetic according to the invention.

While examples of the eyelashes makeup cosmetic and other examples are describe hereinafter, the invention is by no means restricted to these examples.

### Copolymer 2-12

Charged in a glass flask equipped with a stirrer, thermometer and reflux condenser were 35 parts by mass of methyl methacrylate (monomer A1) represented by formula (17), parts by mass ofpolyoxyethylene ether methacrylate (monomer B1) represented by formula (19), 50 parts by mass of methacryloxypropyl polydimethylsiloxne (monomer C1) represented by formula (20), 120 parts by mass of isopropanol, and 4 parts by mass of dimethyl-2,2'-azobis(2-methyl propionate), and the mixture was allowed to react for 10 hou by heating at 80°C in a nitrogen stream. Then, the volatile component was removed by evaporation to obtain copolymer 2-12.

### Copolymer 2-13

Charged in a glass flask equipped with a stirrer, thermometer and reflux condenser were 35 parts by mass of methyl methacrylate (monomer A1) represented by formula (17), 1 parts by mass of 2-ethylhexyl acrylate (monomer A2), 5 parts by mass of polyoxyethylene ether methacrylate (monomer B1) represented by formula (19), 45 parts by mass of methacryloxypropyl polydimethylsiloxane (monomer C2) represented by formula (21), 120 parts by mass of isopropanol, and 4 parts by mass of dimethyl-2,2'-azobis(2-methyl propionate), and the mixture was allowed to react for 10 hours by heating at 80°C in a nitrogen stream. Then, the volatile component was removed by evaporation to obtain copolymer 2-13.

### Example 2-1

| W/O Mascara | % by Mass |
|---|---|
| Light Isoparaffin | 8.0 |
| Dimethyl Polysiloxane | 3.0 |
| Decamethyl Cyclopentasiloxane | 10.0 |
| Copolymer 2-12 | 10.0 |
| Methyl Polysiloxane Emulsion | q.s. |
| 1,3-Butyleneglycol | 4.0 |
| Polyethyleneglycol Dioleate | 2.0 |
| Diglyceryl Diisostearate | 2.0 |
| Sodium Hydrogen Carbonate | 0.1 |
| Sodium Metaphosphate | q.s. |
| DL-α-tocopherol Acetate | 0.1 |
| Paraoxybenzoic Acid Ester | q.s. |
| Sodium Dehydroacetate | q.s. |
| Iron Oxide Black | 7.0 |
| Seaweed Extract | 0.1 |
| Bentonite | 1.0 |
| Dimethylstearyl Ammonium Hectorite | 5.0 |
| Polyvinyl Acetate Emulsion | 20.0 |
| Heavy Liquid Paraffin | 4.0 |
| Nylon Fiber (1 to 2 mm) | 3.0 |
| Pure Water | Balance |

The W/O mascara in Example 2-1 above is excellent in makeup retaining performance with remarkably improved film-like feeling.

### Example 2-2

| Oil-based Mascara | % by Mass |
|---|---|
| Light Isoparaffin | Balance |
| Decamethyl Cyclopentasiloxane | 20.0 |
| Microcrystalline Wax | 17.0 |
| Copolymer 2-13 | 15.0 |
| Iron Oxide Black | 2.0 |
| Hollow Resin Powder | 5.0 |
| Dextrin Fatty Acid Ester | 11.0 |

The oil-based mascara in Example 2-2 above is excellent in makeup retaining performance with remarkably improved film-like feeling.

### 3. Skin Makeup Cosmetics

### Blending of Copolymer

The inventors prepared each copolymer according to the production methods above, and oil-based foundations in which the copolymer was blended as a film-forming component were evaluated. The monomer proportion of the copolymer used in each example and blend composition of the oil-based foundation, and results of evaluation are shown in Table 9. Evaluation criteria are as follows.

### (1) Makeup retaining performance

Retaining performance of the oil-based foundation in each example in practical uses was evaluated by 10 special panelists. The evaluation criteria are as follows:
A: 8 or more panelists recognized retaining performance to be good;
B: 6 or more and less than 8 panelists recognized retaining performance to be good;
C: 3 or more and less than 6 panelists recognized retaining performance to be good; and
D: less than 3 panelists recognized retaining performance to be good.

### (2) Film-like feeling

Film-like feeling of the oil-based foundation in each example in practical uses was evaluated by 10 special panelists. The evaluation criteria are as follows:
A: 8 or more panelists recognized no film-like feeling;
B: 6 or more and less than 8 panelists recognized no film-like feeling;
C: 3 or more and less than 6 panelists recognized no film-like feeling; and
D: less than 3 panelists recognized no film-like feeling.

### (3) Solubility in formulation

Solubility in formulation was evaluated for each copolymer in the example. The evaluation criteria are as follows:
A: solubility was good; and
D: solubility was poor to make preparation impossible.

**Table 9**

| | | | | Test Example | | | | | |
|---|---|---|---|---|---|---|---|---|---|
| | | | | 3-1 | 3-2 | 3-3 | 3-4 | 3-5 | 3-6 |
| | Monomer A1 | Monomer B1 | Monomer C1 | | | | | | |
| Copolymer 1-1 | 35.0 | 5.0 | 60.0 | 5.0 | - | - | - | 10.0 | - |
| Copolymer 1-2 | 100.0 | - | - | - | 5.0 | - | - | - | - |
| Copolymer 1-3 | 50.0 | 50.0 | - | - | - | 5.0 | - | - | - |
| Copolymer 1-4 | 50.0 | - | 50.0 | - | - | - | 5.0 | - | - |
| Siliconated Pullulane (molecular weight 700,000, average degree of substitution 2.0) | | | | 5.0 | 5.0 | 5.0 | 5.0 | - | 10.0 |
| Decamethyl Cyclopentasiloxane | | | | Balance | Balance | Balance | Balance | Balance | Balance |
| Isostearic Acid | | | | 1.0 | 1.0 | 1.0 | 1.0 | 1.0 | 1.0 |
| Ethanol | | | | 10 | 10 | 10 | 10 | 10 | 10 |
| 2-Ethylhexyl Paramethoxycinnamate | | | | 5 | 5 | 5 | 5 | 5 | 5 |
| Titanium Oxide | | | | 5 | 5 | 5 | 5 | 5 | 5 |
| Dextrin Fatty Acid-treated Talc | | | | 3 | 3 | 3 | 3 | 3 | 3 |
| Dextrin Fatty Acid-treated Titanium Dioxide | | | | 15 | 15 | 15 | 15 | 15 | 15 |
| Dextrin Fatty Acid-treated Iron Oxide Yellow | | | | 3 | 3 | 3 | 3 | 3 | 3 |
| Dextrin Fatty Acid-treated Iron Oxide Black | | | | 0.5 | 0.5 | 0.5 | 0.5 | 0.5 | 0.5 |
| Perfume | | | | q.s. | q.s. | q.s.. | q.s. | q.s. | q.s.. |
| (1) Makeup retaining performance | | | | A | - | - | B | B | A |
| (2) Film-like Feeling | | | | A | - | - | C | C | D |
| (3) Solubility in formulation | | | | A | D | D | A | A | A |

Table 9 shows that the oil-based foundation in Test Example 3-1, in which copolymer 3-1 was blended as the film-forming component, showed a quite excellent makeup retaining effect with a remarkable improvement of film-like feeling.

On the contrary, the composition in Test Example 3-2, in which copolymer 3-2 comprising monomer A only was blended with siliconated pullulane, and the composition in Test Example 3-3, in which copolymer 3-3 comprising monomers A and B was blended with siliconated pullulane, could not be formed into preparations due to poor solubility in formulation. The composition in Test Example 3-4, in which copolymer 3-4 comprising monomers A and C was blended, was poor in film-like feeling.

The composition in Test Example 3-5, which is used only the copolymer 3-1, and the Test Example 3-6, which is used siliconated pullulane as film-forming component, were not sufficient in film-like feeling. These results show that it may be preferable to blend two of film forming component, the copolymer 3-1 and siliconated pullulane in the skin makeup cosmetic according to the invention.

### Monomer proportion of copolymer

Subsequently, the inventors prepared various copolymers having different contents of monomer A according to the production method described above in order to investigate favorable monomer proportions of the copolymer, and evaluated the oil-based foundation in which each copolymer was blended. The blend composition of the oil-based foundation in each test example, and results of evaluation thereof are shown in table 10. The evaluation criteria are the same as in the foregoing test.

**Table 10**

| | | | | Test Example | | | | | | |
|---|---|---|---|---|---|---|---|---|---|---|
| | | | | 3-7 | 3-8 | 3-9 | 3-10 | 3-11 | 3-12 | 3-13 |
| | Monomer A1 | Monomer B1 | Monomer C1 | | | | | | | |
| Copolymer 3-5 | - | 5.0 | 95.0 | 10.0 | - | - | - | - | - | - |
| Copolymer 3-6 | 10.0 | 5.0 | 85.0 | - | 10.0 | - | - | - | - | - |
| Copolymer 3-7 | 15.0 | 5.0 | 80.0 | - | - | 10.0 | - | - | - | - |
| Copolymer 3-8 | 20.0 | 5.0 | 75.0 | - | - | - | 10.0 | - | - | - |
| Copolymer 3-9 | 25.0 | 5.0 | 70.0 | - | - | - | - | 10.0 | - | - |
| Copolymer 3-10 | 30.0 | 5.0 | 65.0 | - | - | - | - | - | 10.0 | - |
| Copolymer 3-11 | 40.0 | 5.0 | 55.0 | - | - | - | - | - | - | 10.0 |
| Siliconated Pullulane (molecular weight 700,000, average degree of substitution 2.0) | | | | 5.0 | 5.0 | 5.0 | 5.0 | 5.0 | 5.0 | 5.0 |
| Decamethyl Cyclopentasiloxane | | | | Balance | Balance | Balance | Balance | Balance | Balance | Balance |
| Isostearic Acid | | | | 1.0 | 1.0 | 1.0 | 1.0 | 1.0 | 1.0 | 1.0 |
| Ethanol | | | | 10 | 10 | 10 | 10 | 10 | 10 | 10 |
| 2-Ethylhexyl Paramethoxycmnamate | | | | 5 | 5 | 5 | 5 | 5 | 5 | 5 |
| Titanium Oxide | | | | 5 | 5 | 5 | 5 | 5 | 5 | 5 |
| Dextrin Fatty Acid-treated Talc | | | | 3 | 3 | 3 | 3 | 3 | 3 | 3 |
| Dextrin Fatty Acid-treated Titanium Dioxide | | | | 15 | 15 | 15 | 15 | 15 | 15 | 15 |
| Dextrin Fatty Acid-treated Iron Oxide Yellow | | | | 3 | 3 | 3 | 3 | 3 | 3 | 3 |
| Dextrin Fatty Acid-treated Iron Oxide Black | | | | 0.5 | 0.5 | 0.5 | 0.5 | 0.5 | 0.5 | 0.5 |
| Perfume | | | | q.s. | q.s. | q.s. | q.s. | q.s. | q.s. | q.s. |
| (1) Makeup retaining performance | | | | D | D | C | A | A | A | A |
| (2) Film-like Feeling | | | | A | A | A | A | A | A | A |
| (3) Solubility in formulation | | | | A | A | A | A | A | A | A |

Table 10 shows the composition in Test Example 3-7, in which copolymer 3-5 comprising no monomer A at all was used, was quite poor in makeup retaining performance. On the other hand, while it was shown that makeup retaining performance tends to be improved as the proportion of monomer A increases in the copolymer, the composition in Test Examples 3-8 and 3-9, in which copolymer 3-6 and 3-7 having a proportion of monomer A of less than 20% by mass, respectively, were used, could be hardly recognized to be sufficient in makeup retaining performance. On the contrary, the compositions in Test Examples 3-10 to 3-13, in which copolymer 3-8 to 3-11 having a proportion of monomer A of 20% by mass or more, respectively, were used, were shown to be excellent in both of makeup retaining performance and film-like feeling.

These results indicate that the proportion of monomer A in the copolymer should be 20% by mass or more in the skin makeup cosmetic according to the invention.

### Ratio of amounts of the copolymer: siliconated polysaccharide in skin makeup cosmetics

Subsequently, the inventors prepared oil-based foundation having different ratio of amounts of the copolymer to siliconated polysaccharide in order to investigate the favorable the ratio thereof in the skin makeup cosmetics, and evaluated the cosmetics. The blending compositions of the oil-based foundation in each test examples, and the results of evaluation are listed in Table 11. The evaluation criteria are the same as in the foregoing tests.

**Table 11**

| | Test Example | | | | | | | | | |
|---|---|---|---|---|---|---|---|---|---|---|
| | 3-14 | 3-15 | 3-16 | 3-17 | 3-18 | 3-19 | 3-20 | 3-21 | 3-22 | 3-23 |
| Copolymer 3-1 | 0..1 | 0.5 | 1.0 | 2.5 | 4.0 | 6.0 | 7.5 | 9.0 | 9.5 | 9.9 |
| Siliconated Pullulane | | | | | | | | | | |
| (molecular weight 700,000, average degree of | 9.9 | 9.5 | 9.0 | 7.5 | 6.0 | 4.0 | 2.5 | 1.0 | 0.5 | 0.1 |
| substitution 2.0) | | | | | | | | | | |
| Decamethyl Cyclopentasiloxane | Balance | Balance | Balance | Balance | Balance | Balance | Balance | Balance | Balance | Balance |
| Isostearic Acid | 1.0 | 1.0 | 1.0 | 1.0 | 1.0 | 1.0 | 1.0 | 1.0 | 1.0 | 1.0 |
| Ethanol | 10 | 10 | 10 | 10 | 10 | 10 | 10 | 10 | 10 | 10 |
| 2-Ethylhexyl Paramethoxycinnamate | 5 | 5 | 5 | 5 | 5 | 5 | 5 | 5 | 5 | 5 |
| Titanium Oxide | 5 | 5 | 5 | 5 | 5 | 5 | 5 | 5 | 5 | 5 |
| Dextrin Fatty Acid-treated Talc | 3 | 3 | 3 | 3 | 3 | 3 | 3 | 3 | 3 | 3 |
| Dextrin Fatty Acid-treated Titanium Dioxide | 15 | 15 | 15 | 15 | 15 | 15 | 15 | 15 | 15 | 15 |
| Dextrin Fatty Acid-treated Iron Oxide Yellow | 3 | 3 | 3 | 3 | 3 | 3 | 3 | 3 | 3 | 3 |
| Dextrin Fatty Acid-treated Iron Oxide Black | 0.5 | 0.5 | 0.5 | 0.5 | 0.5 | 0.5 | 0.5 | 0.5 | 0.5 | 0.5 |
| Perfume | q.s. | q.s. | q.s. | q.s. | q.s. | q.s. | q.s. | q.s. | q.s. | q.s. |
| Ratio of Copolymer: Siliconated Pullulane | 1:99 | 1:19 | 1:9 | 1:3 | 4:6 | 6:4 | 3:1 | 1:9 | 19:1 | 99:1 |
| (1) Makeup Retaining Performance | A | A | A | A | A | A | A | A | B | C |
| (2) Film-like Feeling | D | B | A | A | A | A | A | A | A | A |
| (3) Solubility in formulation | A | A | A | A | A | A | A | A | A | A |

Table 11 shows that the cosmetic in Test Example 3-15 to 3-21, in which a ratio of the copolymer 3-1 to siliconated pullulane is 5: 95 to 95:5, exhibited excellent makeup retaining with improving effect in film-like feeling. On the contrary, the cosmetic in Test Example 3-14, in which a ratio of the copolymer 3-1 to siliconated pullulane is 1: 99 tend to be poor in film-like feeling. Also, Test Example 3-23, in which the ratio is 99: 1 tend to be poor in makeup retaining. These results show that it may be preferable that a ratio of the copolymer 3-1 to siliconated pullulane is 5: 95 to 95:5 in the skin makeup cosmetic according to the invention.

While examples of the skin makeup cosmetic and other examples are described hereinafter, the invention is by no means restricted to these examples.

### Copolymer 3-12

Charged in a glass flask equipped with a stirrer, thermometer and reflux condenser were 35 parts by mass of methyl methacrylate (monomer A1) represented by formula (17), 15 parts by mass of polyoxyethylene ether methacrylate (monomer B1) represented by formula (19), 50 parts by mass of methacryloxypropyl polydimethylsiloxne (monomer C1) represented by formula (20), 120 parts by mass of isopropanol, and 4 parts by mass of dimethyl-2,2'-azobis(2-methyl propionate), and the mixture was allowed to react for 10 hours by heating at 80°C in a nitrogen stream. Then, the volatile component was removed by evaporation to obtain copolymer 3-12.

### Example 3-1

| Eye Shadow | % by Mass |
|---|---|
| Copolymer 3-12 | 1.5 |
| Siliconated Pullulane | 1.5 |
| Cerecin | 3 |
| Decamethyl Cyclopentasiloxane | Balance |
| Polyoxyethylene-Methyl Polysiloxane Copolymer | 3 |
| Methylphenyl Polysiloxane | 5 |
| Sorbitan Sesquiisostearate | 1 |
| Mica Titanium | 3 |
| Calmine-coated Mica Titanium | 5 |
| Iron Oxide Red-coated Mica Titanium | 6 |
| Mica | 15 |
| D-• •tocopherol | 0.02 |
| Dimethyldistearyl Ammonium Hectorite | 2.5 |
| Alkyl Polyacrylate | 10 |
| Spherical Nylon Powder | 5 |

### (Production Method)

After dissolving all the components at 90°C the solution was flowed into a vessel to obtain an eye shadow by solidification by cooling.

The eye shadow in Example 3-1 was excellent in retaining performance with no film-like feeling.

### 4 Water-in-oil emulsion cosmetic

### Blending of copolymer

The inventors prepared each copolymer according to the foregoing production methods, and the water-in-oil foundation comprising the copolymer as an emulsifying agent was evaluated. The monomer proportion of the copolymer used in each test and the composition of blending of the water-in-oil foundation, and the results of evaluation are shown in Table 12 below. The evaluation criteria are as follows.

### (1) Temporal stability (after 1 month)

After preserving the water-in-oil foundation in each test for 1 month at room temperature, configuration of the emulsion was visually observed.
A: Emulsion particles were uniform with quite good emulsion state;
B: Emulsion particles were uniform with good emulsion state;
C: Emulsion particles were irregular with slight separation of the aqueous phase from the oil phase; and
D: The aqueous phase was completely separated from the oil phase.

### (2) Temperature stability (50°C)

After preserving the water-in-oil foundation in each test for 1 month at 50°C, configuration of the emulsion was visually observed.
A: Emulsion particles were uniform with quite good emulsion state;
B: Emulsion particles were uniform with good emulsion state;
C: Emulsion particles were irregular with slight separation of the aqueous phase from the oil phase; and
D: The aqueous phase was completely separated from the oil phase.

### (3) Dispersion stability of powder

After preserving the water-in-oil foundation in each test for 1 month at room temperature, dispersion state of the powder was visually observed.
A: the powder particles were uniformly dispersed;
B: the powder particles were almost uniformly dispersed;
C: the powder particles were partially coagulated, and coarse coagulation of the particles was locally confirmed; and
D: the powder particles were remarkably coagulated, and much coarse coagulation of the particles was confirmed.

**Table 12**

| | | | | Test Example | | | | |
|---|---|---|---|---|---|---|---|---|
| | | | | 4-1 | 4-2 | 4-3 | 4-4 | 4-5 |
| | Monomer A1 | Monomer B1 | Monomer C1 | | | | | |
| Copolymer 4-1 | 35.0 | 15.0 | 50.0 | 2.0 | - | - | - | - |
| Copolymer 4-2 | 100.0 | - | - | - | 2.0 | - | - | - |
| Copolymer 4-3 | 85.0 | 15.0 | - | - | - | 2.0 | - | - |
| Copolymer 4-4 | 50.0 | - | 50.0 | - | - | - | 2.0 | - |
| Organo-modified Clay Mineral*¹ | | | | 1.5 | 1.5 | 1.5 | 1.5 | 2.0 |
| Celicite | | | | 5.4 | 5.4 | 5.4 | 5.4 | 5.4 |
| Kaolin | | | | 4.0 | 4.0 | 4.0 | 4.0 | 4.0 |
| Titanium Oxide | | | | 9.3 | 9.3 | 9.3 | 9.3 | 9.3 |
| Iron Oxide Red | | | | 0.4 | 0.4 | 0.4 | 0.4 | 0.4 |
| Iron Oxide Yellow | | | | 0.8 | 0.8 | 0.8 | 0.8 | 0.8 |
| Iron Oxide Black | | | | 0.1 | 0.1 | 0.1 | 0.1 | 0.1 |
| Liquid Paraffin | | | | 5.0 | 5.0 | 5.0 | 5.0 | 5.0 |
| Decamethyl Cyclopentasiloxane | | | | 12.0 | 12.0 | 12.0 | 12.0 | 12.0 |
| Ion-exchange Water | | | | Balance | Balance | Balance | Balance | Balance |
| 1,3-Butyleneglycol | | | | 5.0 | 5.0 | 5.0 | 5.0 | 5.0 |
| Antiseptics | | | | q.s. | q.s. | q.s. | q.s. | q.s. |
| (1) Temporal Stability (after 1 month) | | | | A | C | C | C | C |
| (2) Temperature Stability (50°C) | | | | A | C | C | C | C |

| | | | | | | | | |
|---|---|---|---|---|---|---|---|---|
| *1 An organo-modified clay mineral obtained by allowing smectone with distearyldimethyl ammonium chloride in water at 65:35 ratio. | | | | | | | | |

Table 12 shows that the water-in-oil foundation in Test Example 4-1, in which copolymer 4-1 showed a quite excellent temporal stability and temperature stability of the water-in-oil emulsion.

On the contrary, the composition in Test Example 4-2, in which copolymer 4-2 comprising monomer A only was blended, the composition in Test Example 4-3, in which copolymer 4-3 comprising monomers A and B was blended, and the composition in Test Example 4-4, in which copolymer 4-4 comprising monomers A and C was blended, respectively, were poor in the stability of the emulsion.

Also, the composition in Test Example 4-5, in which was blended only organo-modified clay mineral (smectone - distearyldimethyl ammonium chloride) was not obtained sufficient stability of the emulsion. These results show that it may be preferable to blend both of the copolymer 4-1 and organo-modified clay mineral in the water-in-oil cosmetic according to the invention.

### Blending of large amount of powder

Subsequently, the inventors prepared water-in-oil foundation blended with 30.0 % of powder in order to investigate the dispersion stability of powder in the water-in-oil emulsion bases blended with large amount of powder, and evaluated the cosmetic. The blending compositions of the water-in-oil foundation in each test examples, and the results of evaluation are listed in Table 13. The evaluation criteria are the same as in the foregoing tests.

**Table 13**

| | Test Example | | |
|---|---|---|---|
| | 4-6 | 4-7 | 4-8 |
| Copolymer 4-1 | 2.0 | - | - |
| Diglyceryl Diisostearate (HLB2) | - | 2.0 | - |
| POE(3) Hardened Caster Oil (HLB3) | - | - | 2.0 |
| Organo-modified Clay Mineral*¹ | 1.5 | 1.5 | 1.5 |
| Celicite | 8.4 | 8.4 | 8.4 |
| Kaolin | 7.0 | 7.0 | 7.0 |
| Titanium Oxide | 13.3 | 13.3 | 13.3 |
| Iron Oxide Red | 0.4 | 0.4 | 0.4 |
| Iron Oxide Yellow | 0.8 | 0.8 | 0.8 |
| Iron Oxide Black | 0.1 | 0.1 | 0.1 |
| Liquid Paraffin | 5.0 | 5.0 | 5.0 |
| Decamethyl Cyclopentasiloxane | 12.0 | 12.0 | 12.0 |
| Ion-exchange Water | Balance | Balance | Balance |
| 1,3-Butyleneglycol | 5.0 | 5.0 | 5.0 |
| Antiseptics | q.s. | q.s. | q.s. |
| (1) Temporal Stability (after 1 month) | A | C | C |
| (2) Temperature Stability (50°C) | A | C | C |
| (3) Dispersion Stability of Powder | A | C | C |

| | | | |
|---|---|---|---|
| *1 A organo-modified clay mineral obtained by allowing smectone with distearyldimethyl ammonium chloride in water at 65:35 ratio. | | | |

Table 13 shows that the water-in-oil foundation of Test Example 4-7 and 4-8, in which general water-in-oil emulsifying agents were used, were poor in dispersion stability of powder in order to generate condensation of large amount of powder which coexist in the water-in-oil base.

On the contrary, water-in-oil foundation of Test Example 4-6, in which copolymer 4-1 was blended as water-in-oil emulsifying agent, although showed a quite excellent dispersion stability of powder, which was blended large amount of powder.

### Monomer proportion of copolymer

Subsequently, the inventors prepared various copolymers having different contents of monomer A according to the production method described above in order to investigate favorable monomer proportion of the copolymer, and evaluated the water-in-oil foundation in which each copolymer was blended. The blend composition of the water-in-oil foundation in each test example, and results of evaluation thereof are shown in table 14. The evaluation criteria are the same as in the foregoing test.

**Table 14**

| | | | | Test Example | | | | | | |
|---|---|---|---|---|---|---|---|---|---|---|
| | | | | 4-9 | 4-10 | 4-11 | 4-12 | 4-13 | 4-14 | 4-15 |
| | Monomer A1 | Monomer B1 | Monomer C1 | | | | | | | |
| Copolymer 4-5 | - | 15.0 | 85.0 | 2.0 | - | - | - | - | - | - |
| Copolymer 4-6 | 10.0 | 15.0 | 75.0 | - | 2.0 | - | - | - | - | - |
| Copolymer 4-7 | 15.0 | 15.0 | 70.0 | - | - | 2.0 | - | - | - | - |
| Copolymer 4-8 | 20.0 | 15.0 | 65.0 | - | - | - | 2.0 | - | - | - |
| Copolymer 4-9 | 25.0 | 15.0 | 60.0 | - | - | - | - | 2.0 | - | - |
| Copolymer 4-10 | 30.0 | 15.0 | 55.0 | - | - | - | - | - | 2.0 | - |
| Copolymer 4-11 | 40.0 | 15.0 | 45.0 | - | - | - | - | - | - | 2.0 |
| Organo-modified Clay Mineral*¹ | | | | 1.5 | 1.5 | 1.5 | 1.5 | 1.5 | 1.5 | 1.5 |
| Celicite | | | | 5.4 | 5.4 | 5.4 | 5.4 | 5.4 | 5.4 | 5.4 |
| Kaolin | | | | 4.0 | 4.0 | 4.0 | 4.0 | 4.0 | 4.0 | 4.0 |
| Titanium Oxide | | | | 9.3 | 9.3 | 9.3 | 9.3 | 9.3 | 9.3 | 9.3 |
| Iron Oxide Red | | | | 0.4 | 0.4 | 0.4 | 0.4 | 0.4 | 0.4 | 0.4 |
| Iron Oxide Yellow | | | | 0.8 | 0.8 | 0.8 | 0.8 | 0.8 | 0.8 | 0.8 |
| Iron Oxide Black | | | | 0.1 | 0.1 | 0.1 | 0.1 | 0.1 | 0.1 | 0.1 |
| Liquid Paraffin | | | | 5.0 | 5.0 | 5.0 | 5.0 | 5.0 | 5.0 | 5.0 |
| Decamethyl Cyclopentasiloxane | | | | 12.0 | 12.0 | 12.0 | 12.0 | 12.0 | 12.0 | 12.0 |
| Ion-exchange Water | | | | Balance | Balance | Balance | Balance | Balance | Balance | Balance |
| 1,3-Butyleneglycol | | | | 5.0 | 5.0 | 5.0 | 5.0 | 5.0 | 5.0 | 5.0 |
| Antiseptics | | | | q.s. | q.s. | q.s. | q.s. | q.s. | q.s. | q.s. |
| (1) Temporal Stability (after 1 month) | | | | C | B | B | A | A | A | A |
| (2) Temperature Stability (50°C) | | | | D | C | B | A | A | A | A |
| (3) Dispersion Stability of Powder | | | | D | C | C | A | A | A | A |

| | | | | | | | | | | |
|---|---|---|---|---|---|---|---|---|---|---|
| * 1 An organo-modified clay mineral obtained by allowing smectone with distearyldimethyl ammonium chloride in water at 65:35 ratio. | | | | | | | | | | |

Table 14 shows the composition in Test Example 4-9, in which copolymer 4-5 comprising no monomer A at all was used, was quite poor in the stability of emulsion and dispersion stability of powder. On the other hand, while it was shown that the stability of emulsion and dispersion stability of powder tends to be improved as the proportion of monomer A increases in the copolymer, the composition in Test Examples 4-10 and 4-11, in which copolymer 4-6 and 4-7 having a proportion of monomer A of less than 20% by mass, respectively, were used, could be hardly recognized to be sufficient in the stability thereof. On the contrary, the compositions in Test Examples 4-12 to 4-15, in which copolymer 4-8 to 4-11 having a proportion of monomer A of 20% by mass or more, respectively, were used, were shown to be excellent in both the stability of emulsion and dispersion stability of powder.

These results indicate that the proportion of monomer A in the copolymer should be 20% by mass or more in the water-in-oil cosmetic according to the invention.

### Ratio of amounts of the copolymer: organo -modified clay mineral

Subsequently, the inventors prepared water-in-oil foundation having different ratio of amounts of the copolymer to organo-modified clay mineral in order to investigate the favorable the ratio thereof in the water-in-oil cosmetic, and evaluated the cosmetic. The blending compositions of the water-in-oil foundation in each test examples, and the results of evaluation are listed in Table 15. The evaluation criteria are the same as in the foregoing tests.

**Table 15**

| | Test Example | | | | | | | |
|---|---|---|---|---|---|---|---|---|
| | 4-16 | 4-17 | 4-18 | 4-19 | 4-20 | 4-21 | 4-22 | 4-23 |
| Copolymer 3-1 | 0.01 | 0.8 | 1.5 | 3.0 | 7.5 | 5.0 | 6.0 | 4.0 |
| Organo-modified Clay Mineral*¹ | 1.5 | 1.5 | 1.5 | 1.5 | 1.5 | 0.5 | 0.3 | 0.1 |
| Celicite | 5.4 | 5.4 | 5.4 | 5.4 | 5.4 | 5.4 | 5.4 | 5.4 |
| Kaolin | 4.0 | 4.0 | 4.0 | 4.0 | 4.0 | 4.0 | 4.0 | 4.0 |
| Titanium dioxide | 9.3 | 9.3 | 9.3 | 9.3 | 9.3 | 9.3 | 9.3 | 9.3 |
| Iron Oxide Red | 0.4 | 0.4 | 0.4 | 0.4 | 0.4 | 0.4 | 0.4 | 0.4 |
| Iron Oxide Yellow | 0.8 | 0.8 | 0.8 | 0.8 | 0.8 | 0.8 | 0.8 | 0.8 |
| Iron Oxide Black | 0.1 | 0.1 | 0.1 | 0.1 | 0.1 | 0.1 | 0.1 | 0.1 |
| Liquid Paraffin | 5.0 | 5.0 | 5.0 | 5.0 | 5.0 | 5.0 | 5.0 | 5.0 |
| Decamethyl Cyclopentasiloxane | 12.0 | 12.0 | 12.0 | 12.0 | 12.0 | 12.0 | 12.0 | 12.0 |
| Ion-exchange Water | Balance | Balance | Balance | Balance | Balance | Balance | Balance | Balance |
| 1,3-Butyleneglycol | 5.0 | 5.0 | 5.0 | 5.0 | 5.0 | 5.0 | 5.0 | 5.0 |
| Antiseptics | q.s. | q.s. | q.s. | q.s. | q.s. | q.s. | q.s. | q.s. |
| Copolymer / Siliconated Pullulane | 0.007 | 0.53 | 1.0 | 2.0 | 5.0 | 10.0 | 20.0 | 40.0 |
| (1) Temporal Stability (after 1 month) | C | A | A | A | A | A | A | B |
| (2) Temperature Stability (50°C) | C | A | A | A | A | A | A | B |
| (3) Dispersion Stability of Powder | C | A | A | A | A | A | A | B |
| *1 An organo-modified clay mineral obtained by allowing smectone with distearyldimethyl ammonium chloride in water at 65:35 ratio. | | | | | | | | |

Table15 shows that the composition in Test Example 4-17 to 4-22, in which ratio of the copolymer 4-1 to organo-modified clay mineral is 0.53:1 to 20:1, exhibited excellent stability of emulsion and dispersion stability of powder. On the contrary, the cosmetic in Test Example 4-16, in which the ratio is 0.007: 1 tend to be poor in the stability of emulsion and dispersion stability of powder. Also, Test Example 4-23, in which the ratio is 40: 1 tend to be poor in the stabilizing effect. These results show that it may be preferable that a ratio of the copolymer 4-1 to organo-modified clay mineral is 0.01: 1 to 20:1 in the water-in-oil cosmetic according to the invention.
While examples of the water-in-oil cosmetic and other examples are described hereinafter, the invention is by no means restricted to these examples.

### Copolymer 4-12

Charged in a glass flask equipped with a stirrer, thermometer and reflux condenser were 35 parts by mass of methyl methacrylate (monomer A1) represented by formula (17), 5 parts by mass of polyoxyethylene ether methacrylate (monomer B1) represented by formula (19), 60 parts by mass of methacryloxypropyl polydimethylsiloxne (monomer C1) represented by formula (20), 120 parts by mass of isopropanol, and 4 parts by mass of dimethyl-2,2'-azobis(2-methyl propionate), and the mixture was allowed to react for 10 hours by heating at 80°C in a nitrogen stream. Then, the volatile component was removed by evaporation to obtain copolymer 4-12,

### Example 4-1

| Water-in-oil Sunscreen | % by Mass |
|---|---|
| (A) | |
| Copolymer 4-12 | 3.0 |
| Bee Gum (Water-swellable Clay Mineral Manufactured by Bander Built Co., USA) | 2.0 |
| Benzyldimethylstearyl Ammonium Chloride | 1.0 |
| Isostearyl Alcohol | 2.0 |
| Isoparaffin | 5.0 |
| Di-2-ethylhexyl Succinate | 10.0 |
| Pentaerythrityl Tetraoctanoate | 5.0 |
| Glyceryl Tri-2-ethylhexanoate | 5.0 |
| Hydrophobic Titanium Oxide Fine Particle | 10.0 |
| Hydrophobic Zinc Oxide Fine Particle | 6.0 |
| Octylmethoxy Cinnamate | 7.0 |
| 2,4-bis-[[4-(2-ethylhexyloxy)-2-hydroxy]-phenyl]-6-(4-methoxyphanyl)-1,3,5-triazine | 2.0 |
| Perfume | q.s. |

| (B) | |
|---|---|
| Glycerin | 5.0 |
| Dipropyleneglycol | 5.0 |
| Purified Water | Balance |

### (Production Method)

Bee gum was dispersed in ethanol, and benzyldimethylstearyl ammonium chloride was added. After heating at 50°C, the suspension was dispersed with a disper. Remaining component (A) was added at 70°C to the organo-modified clay mineral obtained by removing ethanol by sufficient drying. After sufficiently mixing with the disper, the components of phase (B) were added at 70°C to phase (A) while stirring with the disper to sufficiently mix. The water-in-oil sunscreen was obtained by cooling the mixture to 30°C.

The water-in-oil sunscreen in Example 4-1 showed good extendibility with quite excellent stability of the emulsion with time.

## Claims

1. A cosmetic comprising one or more of copolymer which comprises monomer (A) represented by formula (1), monomer (B) represented by formula (2) and monomer (C) represented by formula (3) as constituting monomers, wherein the content of monomer (A) is 20% by mass or more, the content of monomer (B) is 0.1 to 50% by mass, the content of monomer (C) is 30 to 80% by mass, each relative to the total amount of the constituting monomers. 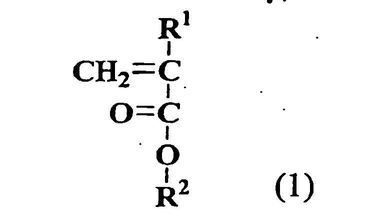 where, R¹ is hydrogen or a hydrocarbon group having 1 to 3 of carbon atoms, and R² is hydrogen or a hydrocarbon group having 1 to 24 of carbon atoms. 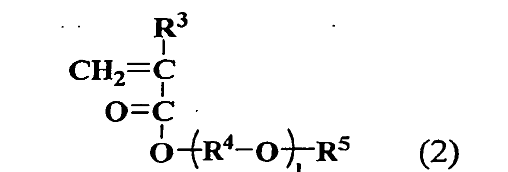 where, R³ is hydrogen or a hydrocarbon group having 1 to 3 of carbon atoms, R⁴ is a divalent hydrocarbon group having 1 to 4 of carbon atoms, R⁵ is a hydrocarbon group having 1 to 24 of carbon atoms, and 1 is an integer of 1 to 50. 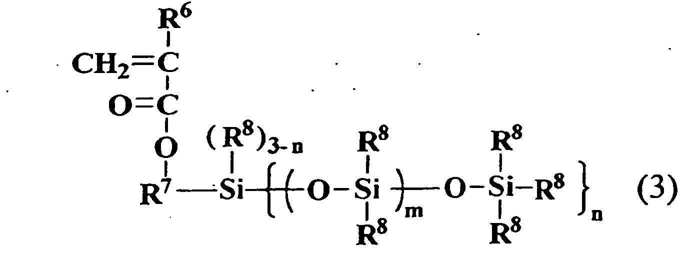 where, R⁶ is hydrogen or a hydrocarbon group having 1 to 3 of carbon atoms, R⁷ is a divalent hydrocarbon group having 1 to 4 of carbon atoms, R⁸ is a hydrocarbon group 1 to 6 of carbon atoms, m is an integers of 0 to 500, and n is an integers of 1 to 3.

2. The cosmetic according to claim 1, wherein the cosmetic is a lip makeup cosmetic.

3. The lip makeup cosmetic according to claim2, wherein said lip makeup cosmetic further comprises one or more of volatile oil components selected from a linear silicone oil represented by formula (4), a cyclic silicone oil represented by formula (5), an alkyl-modified silicone oil represented by formula (6), and isoparaffin. 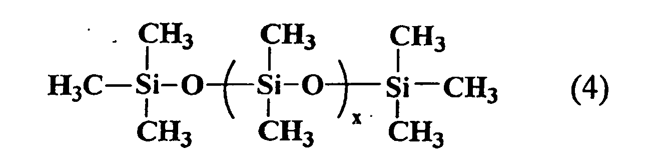 where, x is an integer of 0 to 3. 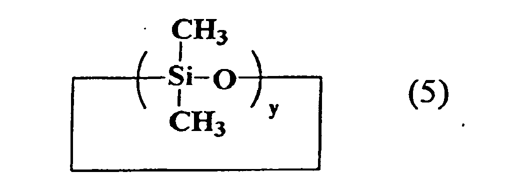 where, y is an integer of 3 to 6. 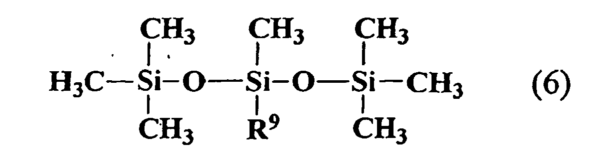 where, R⁹ is a hydrocarbon group having 2 to 8 of carbon atoms.

4. The lip makeup cosmetic according to claim2, wherein said lip makeup cosmetic further comprises one or more of non-volatile component selected from a fluorine-modified dimethylsilicone represented by formula (7), a fluorine-modified phenylsilicone represented by formula (8), an alkoxy-modified silicone represented by formula (9), an alkyl-modified silicone represented by formula (10), tri(hydrogenated rosin-isostearic acid)glyceryl, and 2-ethylhexyl paramethoxycinnamate. 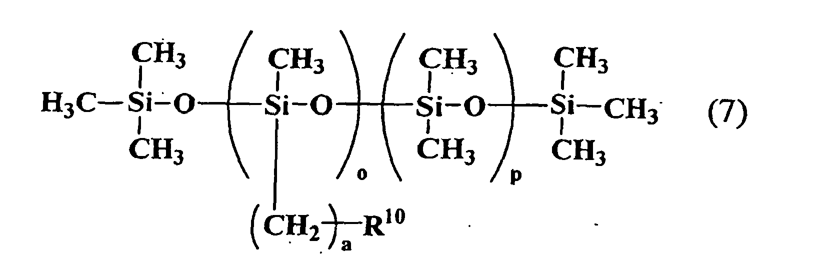 where, o and p are average values, o is an integer of 1 to 150, p is an integer of 0 to 150, the sum of o and p is 4 or more, a is an integer of 0 to 10, and R¹⁰ is a perfluoroalkyl group having 1 to 12 of carbon atoms. 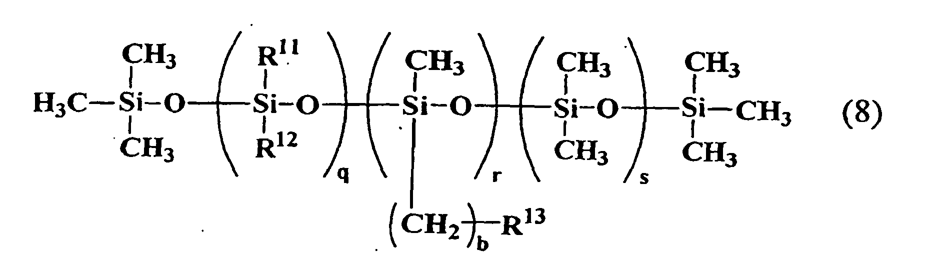 where, q, r and s are average values, q is an integer of 1 to 150, r is an integer of 1 to 150, s is an integer of 0 to 150, the sum of q, r and s is 4 or more, b is an integer of 0 to 10, R¹¹ and R¹² is a methyl or phenyl group with at least one of R¹¹ and R¹² is the phenyl group, and R¹³ is a perfluoroalkyl group having 1 to 12 of carbon atoms. 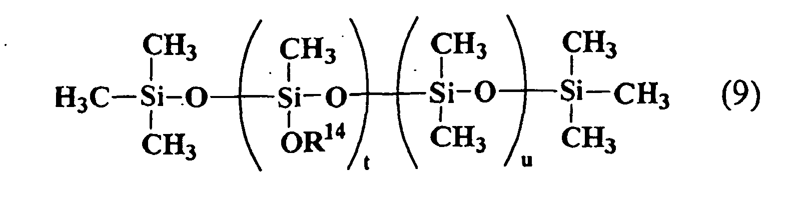 where, OR¹⁴ is an alkoxy group having 2 to 30 of carbon atoms, t and u are average values, t is an integer of 1 to 500, u is an integer of 0 to 500, and the sum of t and u is 4 or more. 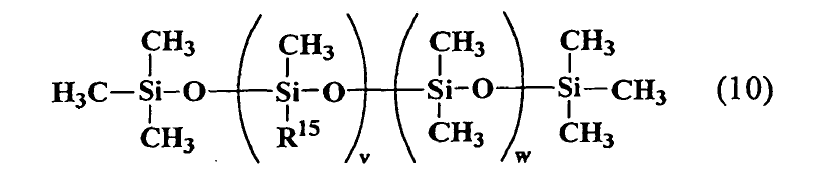 where, R¹⁵ is an alkyl group having 10 to 30 of carbon atoms, v and w are average values, v is an integer of 1 to 500, w is an integer of 0 to 500, and the sum of v and w is 4 or more.

5. The lip makeup cosmetic according to claim2, wherein said lip makeup cosmetic further comprises water or a humectant.

6. The cosmetic according to claim 1, wherein said cosmetic is an eyelashes makeup cosmetic.

7. The eyelashes makeup cosmetic according to claim6, wherein said eyelashes makeup cosmetic is a water-in-oil eyelashes makeup cosmetic comprising the copolymer and an oil ingredient in the outer phase and water in the inner phase.

8. The water-in-oil eyelashes makeup cosmetic according to claim7, wherein said water-in-oil eyelashes makeup cosmetic comprises 1 to 30% of the copolymer and volatile silicone oil and/or hydrocarbon oil in the outer phase, and water and a film-forming emulsion resin in the inner phase.

9. The eyelashes makeup cosmetic according to claim6, wherein said eyelashes makeup cosmetic is an oil-in-water eyelashes makeup cosmetic comprising the copolymer and an oil ingredient in the inner phase, and water in the outer phase.

10. The oil-in-water eyelashes makeup cosmetic according to claim9, wherein said oil-in-water eyelashes cosmetic comprises 1 to 30% of the copolymer and a volatile silicone oil and/or hydrocarbon oil in the inner phase, and water and a film-forming emulsion resin in the outer phase.

11. The eyelashes makeup cosmetic according to claim6, wherein said eyelashes makeup is an oil-based eyelashes makeup cosmetic comprises the copolymer and a wax.

12. The oil-based eyelashes makeup cosmetic according to claim11, wherein said oil-based eyelashes makeup cosmetic comprises 1 to 30% of the polymer, a wax, a volatile silicone oil and/or hydrocarbon oil and a viscosity improving agent.

13. The oil-based eyelashes makeup cosmetic according to claimll, wherein said oil-based eyelashes makeup cosmetic further comprises hollow particle.

14. The cosmetic according to claim 1, wherein said cosmetic is a skin makeup cosmetic.

15. The skin makeup cosmetic according to claiml4, wherein said skin makeup cosmetic further comprises siliconated polysaccharide represented by formula (11). 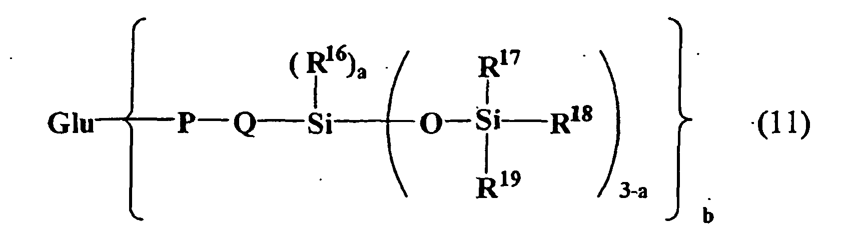 where, Glu is a sugar residue of the polysaccharide, P is a divalent binding group, Q represents a divalent aliphatic group, R¹⁶ is a hydrocarbon group having 1 to 8 of carbon atoms, and R¹⁷, R¹⁸ and R¹⁹ is hydrocarbon groups having 1 to 8 of carbon atoms or siloxy groups resented by -OSiR²⁰R²¹R²², wherein R²⁰, R²¹ and R²² are hydrocarbon group having 1 to 8 of carbon atoms, a is an integer of 0 to 2, and b is a positive integer.

16. The skin makeup cosmetic according to claim 15, wherein said siliconated polysaccharide is siliconated pullulane represented by formula (12). 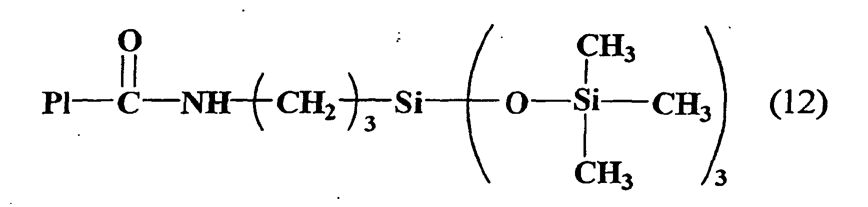 where, P1 is a glucose residue of pullulane.

17. The cosmetic according to claim 1, wherein the cosmetic is a water-in-oil emulsion cosmetic.

18. The water-in-oil emulsion cosmetic according to claim17, wherein said water-in-oil emulsion cosmetic further comprises a water-swelling clay mineral and quaternary ammonium cation surfactant.

19. The water-in-oil emulsion cosmetic according to claim 17, wherein said water-in-oil emulsion cosmetic further comprises powder.

20. The water-in-oil emulsion cosmetic according to claiml7, wherein said water-in-oil emulsion cosmetic further comprises a nonionic surfactant.

21. The water-in-oil emulsion cosmetic according to claim 17, wherein said water-in-oil emulsion cosmetic further comprises a volatile silicone.

22. The water-in-oil emulsion cosmetic according to claim 17, wherein said water-in-oil emulsion cosmetic further comprises a UV absorbing agent and/or a UV scattering agent.

## Patentansprüche

1. Kosmetikum, das ein oder mehrere Copolymere, die ein Monomer (A) der Formel (1), ein Monomer (B) der Formel (2) und ein Monomer (C) der Formel (3) als konstituierende Monomere umfassen, wobei der Gehalt an dem Monomer (A) 20 Masse-% oder mehr, der Gehalt an dem Monomer (B) 0,1 bis 50 Masse-%, der Gehalt an dem Monomer (C) 30 bis 80 Masse-%, jeweils bezogen auf die Gesamtmenge der konstituierenden Monomere, beträgt, umfasst. 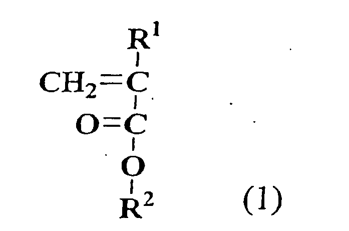 worin R¹ für Wasserstoff oder eine Kohlenwasserstoffgruppe mit 1 bis 3 Kohlenstoffatomen steht und R² für Wasserstoff oder eine Kohlenwasserstoffgruppe mit 1 bis 24 Kohlenstoffatomen steht. 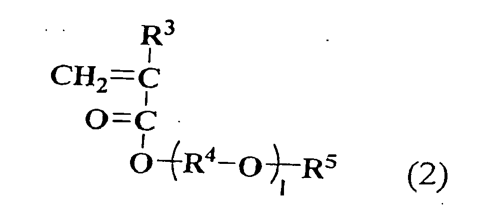 worin R³ für Wasserstoff oder eine Kohlenwasserstoffgruppe mit 1 bis 3 Kohlenstoffatomen steht, R⁴ für eine zweiwertige Kohlenwasserstoffgruppe mit 1 bis 4 Kohlenstoffatomen steht, R⁵ für eine Kohlenwasserstoffgruppe mit 1 bis 24 Kohlenstoffatomen steht und 1 eine ganze Zahl von 1 bis 50 ist. 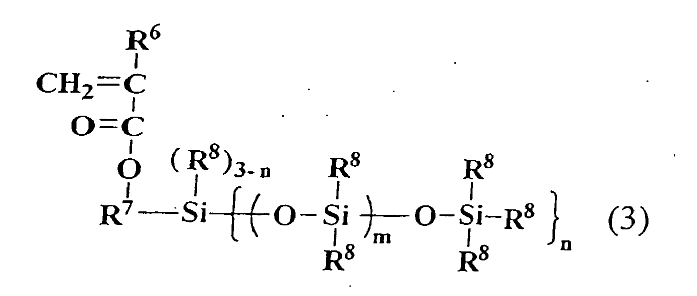 worin R⁶ für Wasserstoff oder eine Kohlenwasserstoffgruppe mit 1 bis 3 Kohlenstoffatomen steht, R⁷ für eine zweiwertige Kohlenwasserstoffgruppe mit 1 bis 4 Kohlenstoffatomen steht, R⁸ für eine Kohlenwasserstoffgruppe mit 1 bis 6 Kohlenstoffatomen steht, m eine ganze Zahl von 0 bis 500 ist und n eine ganze Zahl von 1 bis 3 ist.

2. Kosmetikum nach Anspruch 1, wobei das Kosmetikum ein Lippenmakeup-Kosmetikum ist.

3. Lippenmakeup-Kosmetikum nach Anspruch 2, wobei das Lippenmakeup-Kosmetikum ferner eine oder mehrere flüchtige Ölkomponenten umfasst, die aus einem linearen Siliconöl der Formel (4), einem cyclischen Siliconöl der Formel (5), einem alkylmodifizierten Siliconöl der Formel (6) und einem Isoparaffin ausgewählt sind. 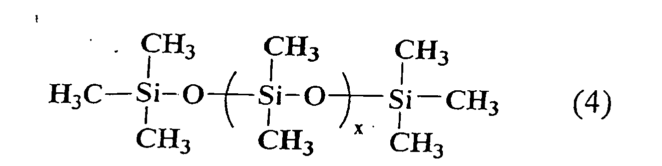 worin x eine ganze Zahl von 0 bis 3 ist. 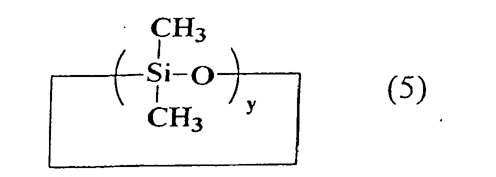 worin y eine ganze Zahl von 3 bis 6 ist. 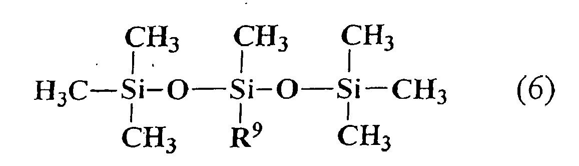 worin R⁹ eine Kohlenwasserstoffgruppe mit 2 bis 8 Kohlenstoffatomen ist.

4. Lippenmakeup-Kosmetikum nach Anspruch 2, wobei das Lippenmakeup-Kosmetikum ferner eine oder mehrere nichtflüchtige Komponenten umfasst, die aus einem fluormodifizierten Dimethylsilicon der Formel (7), einem fluormodifizierten Phenylsilicon der Formel (8), einem alkoxymodifizierten Silicon der Formel (9), einem alkylmodifizierten Silicon der Formel (10), Tri(hydriertes-Kolophonium-isostearinsäure)glycerin und 2-Ethylhexyl-paramethoxycinnamat ausgewählt sind. 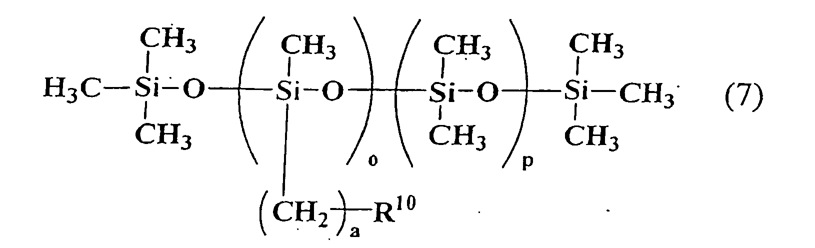 worin o und p Durchschnittswerte sind, o eine ganze Zahl von 1 bis 150 ist, p eine ganze Zahl von 0 bis 150 ist, die Summe von o und p 4 oder mehr beträgt, a eine ganze Zahl von 0 bis 10 ist und R¹⁰ für eine Perfluoralkylgruppe mit 1 bis 12 Kohlenstoffatomen steht. 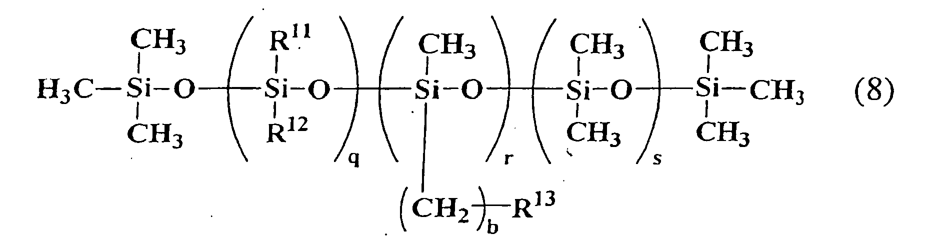 worin q, r und s Durchschnittswerte sind, q eine ganze Zahl von 1 bis 150 ist, r eine ganze Zahl von 1 bis 150 ist, s eine ganze Zahl von 0 bis 150 ist, die Summe von q, r und s 4 oder mehr beträgt, b eine ganze Zahl von 0 bis 10 ist, R¹¹ und R¹² für eine Methyl- oder Phenylgruppe stehen, wobei mindestens ein Rest von R¹¹ und R¹² die Phenylgruppe ist, und R¹³ für eine Perfluoralkylgruppe mit 1 bis 12 Kohlenstoffatomen steht. 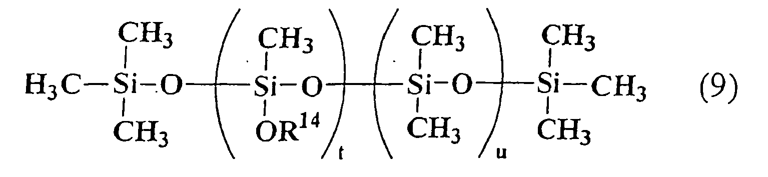 worin OR¹⁴ für eine Alkoxygruppe mit 2 bis 30 Kohlenstoffatomen steht, t und u Durchschnittswerte sind, t eine ganze Zahl von 1 bis 500 ist, u eine ganze Zahl von 0 bis 500 ist und die Summe von t und u 4 oder mehr beträgt. 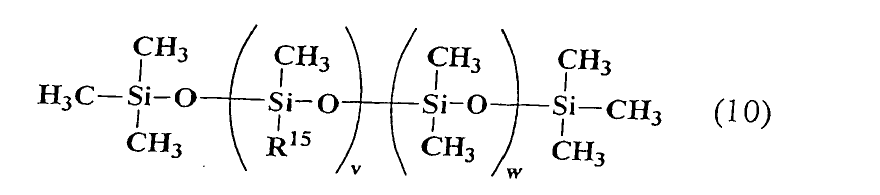 worin R¹⁵ für eine Alkylgruppe mit 10 bis 30 Kohlenstoffatomen steht, v und w Durchschnittswerte sind, v eine ganze Zahl von 1 bis 500 ist, w eine ganze Zahl von 0 bis 500 ist und die Summe von v und w 4 oder mehr beträgt.

5. Lippenmakeup-Kosmetikum nach Anspruch 2, wobei das Lippenmakeup-Kosmetikum ferner Wasser oder ein Feuchthaltemittel umfasst.

6. Kosmetikum nach Anspruch 1, wobei das Kosmetikum ein Wimpernmakeup-Kosmetikum ist.

7. Wimpernmakeup-Kosmetikum nach Anspruch 6, wobei das Wimpernmakeup-Kosmetikum ein Wasser-in-Öl-Wimpernmakeup-Kosmetikum ist, das das Copolymer und einen Ölbestandteil in der äußeren Phase und Wasser in der inneren Phase umfasst.

8. Wasser-in-Öl-Wimpernmakeup-Kosmetikum nach Anspruch 7, wobei das Wasser-in-Öl-Wimpernmakeup-Kosmetikum 1 bis 30 Gew.-% an dem Copolymer und das flüchtige Siliconöl und/oder das Kohlenwasserstofföl in der äußeren Phase und Wasser und ein filmbildendes Emulsionsharz in der inneren Phase umfasst.

9. Wimpernmakeup-Kosmetikum nach Anspruch 6, wobei das Wimpernmakeup-Kosmetikum ein Öl-in-Wasser-Wimpernmakeup-Kosmetikum ist, das das Copolymer und einen Ölbestandteil in der inneren Phase und Wasser in der äußeren Phase umfasst.

10. Öl-in-Wasser-Wimpernmakeup-Kosmetikum nach Anspruch 9, wobei das Öl-in-Wasser-Wimpernmakeup-Kosmetikum 1 bis 30 Gew.-% an dem Copolymer und das flüchtige Siliconöl und/oder das Kohlenwasserstofföl in der inneren Phase und Wasser und ein filmbildendes Emulsionsharz in der äußeren Phase umfasst.

11. Wimpernmakeup-Kosmetikum nach Anspruch 6, wobei das Wimpernmakeup ein Wimpernmakeup-Kosmetikum auf Ölbasis ist, das das Copolymer und ein Wachs umfasst.

12. Wimpernmakeup-Kosmetikum auf Ölbasis nach Anspruch 11, wobei das Wimpernmakeup-Kosmetikum auf Ölbasis 1 bis 30 Gew.% an dem Polymer, ein Wachs, ein flüchtiges Siliconöl und/ oder ein Kohlenwasserstofföl und ein die Viskosität verbesserndes Mittel umfasst.

13. Wimpernmakeup-Kosmetikum auf Ölbasis nach Anspruch 11, wobei das Wimpernmakeup-Kosmetikum auf Ölbasis ferner hohle Teilchen umfasst.

14. Kosmetikum nach Anspruch 1, wobei das Kosmetikum ein Hautmakeup-Kosmetikum ist.

15. Hautmakeup-Kosmetikum nach Anspruch 14, wobei das Hautmakeup-Kosmetikum ferner ein siliconisiertes Polysaccharid der Formel (11) umfasst. 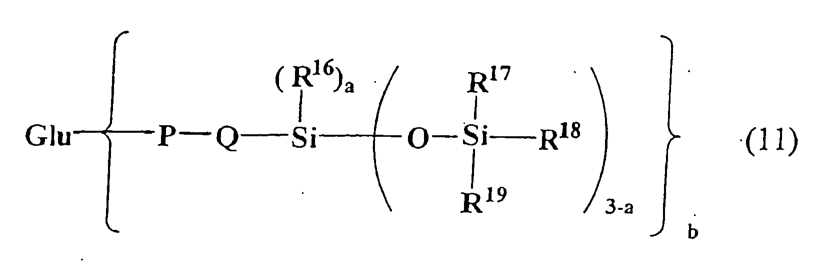 worin Glu für einen Zuckerrest des Polysaccharids steht, P für eine zweiwertige Bindungsgruppe steht, Q für eine zweiwertige aliphatische Gruppe steht, R¹⁶ für eine Kohlenwasserstoffgruppe mit 1 bis 8 Kohlenstoffatomen steht und R¹⁷, R¹⁸ und R¹⁹ für Kohlenwasserstoffgruppen mit 1 bis 8 Kohlenstoffatomen oder durch -OSiR²⁰R²¹R²² dargestellte Siloxygruppen, worin R²⁰, R²¹ und R²² Kohlenwasserstoffgruppen mit 1 bis 8 Kohlenstoffatomen sind, stehen, a eine ganze Zahl von 0 bis 2 ist und b eine positive ganze Zahl ist.

16. Hautmakeup-Kosmetikum nach Anspruch 15, wobei das siliconisierte Polysaccharid siliconisiertes Pullulan der Formel (12) ist. 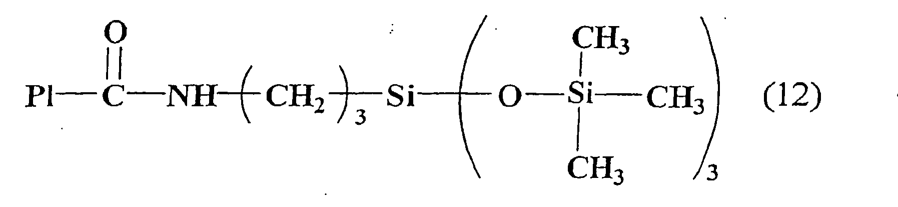 worin Pl für einen Glucoserest von Pullulan steht.

17. Kosmetikum nach Anspruch 1, wobei das Kosmetikum ein Kosmetikum einer Wasser-in-Öl-Emulsion ist.

18. Kosmetikum einer Wasser-in-Öl-Emulsion nach Anspruch 17, wobei das Kosmetikum einer Wasser-in-Öl-Emulsion ferner ein wasserquellbares Tonmineral und ein grenzflächenaktives Mittel eines quaternären Ammoniumkations umfasst.

19. Kosmetikum einer Wasser-in-Öl-Emulsion nach Anspruch 17, wobei das Kosmetikum einer Wasser-in-Öl-Emulsion ferner ein Pulver umfasst.

20. Kosmetikum einer Wasser-in-Öl-Emulsion nach Anspruch 17, wobei das Kosmetikum einer Wasser-in-Öl-Emulsion ferner ein nichtionisches grenzflächenaktives Mittel umfasst.

21. Kosmetikum einer Wasser-in-Öl-Emulsion nach Anspruch 17, wobei das Kosmetikum einer Wasser-in-Öl-Emulsion ferner ein flüchtiges Silicon umfasst.

22. Kosmetikum einer Wasser-in-Öl-Emulsion nach Anspruch 17, wobei das Kosmetikum einer Wasser-in-Öl-Emulsion ferner ein UV-Absorptionsmittel und/oder ein UV-Streuungsmittel umfasst.

## Revendications

1. Composition cosmétique comprenant un ou plusieurs copolymères comprenant un monomère (A) représenté par la formule (1), un monomère (B) représenté par la formule (2) et un monomère (C) représenté par la formule (3) comme monomères de constitution, dans laquelle la teneur en monomère (A) est de 20 % en masse ou plus, la teneur en monomère (B) est de 0,1 à 50 % en masse, la teneur en monomère (C) est de 30 à 80 % en masse, chaque, par rapport à la quantité totale des monomères de constitution ; 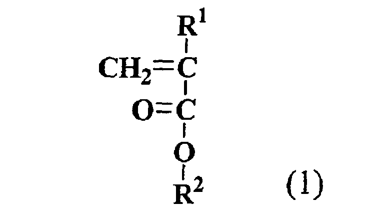 où R¹ est un hydrogène ou un groupe hydrocarboné ayant de 1 à 3 atomes de carbone, et R² est un hydrogène ou un groupe hydrocarboné ayant de 1 à 24 atomes de carbone ; 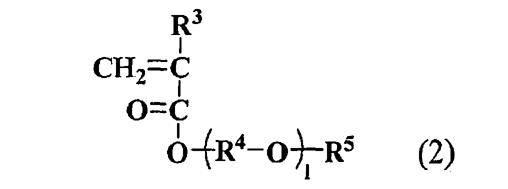 où R³ est un hydrogène ou un groupe hydrocarboné ayant de 1 à 3 atomes de carbone, R⁴ est un groupe hydrocarboné divalent ayant de 1 à 4 atomes de carbone, R⁵ est un groupe hydrocarboné ayant de 1 à 24 atomes de carbone, et 1 est un entier de 1 à 50 ; 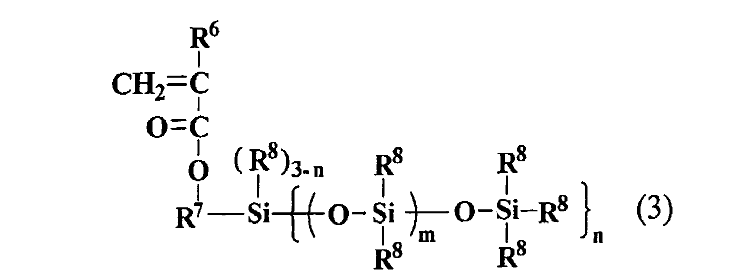 où R⁶ est un hydrogène ou un groupe hydrocarboné ayant de 1 à 3 atomes de carbone, R⁷ est un groupe hydrocarboné divalent ayant de 1 à 4 atomes de carbone, R⁸ est un groupe hydrocarboné ayant de 1 à 6 atomes de carbone, m est un entier de 0 à 500 et n est un nombre entier de 1 à 3.

2. Composition cosmétique selon la revendication 1, dans laquelle la composition cosmétique est composition cosmétique pour rouge à lèvres.

3. Composition cosmétique pour rouge à lèvres selon la revendication 2, dans laquelle ladite composition cosmétique pour rouge à lèvres comprend en outre un ou plusieurs composants huileux volatiles choisis parmi une huile silicone linéaire représentée par la formule (4), une huile silicone cyclique représentée par la formule (5), une huile silicone à modification alkyle représentée par la formule (6), et l'isoparaffine ; 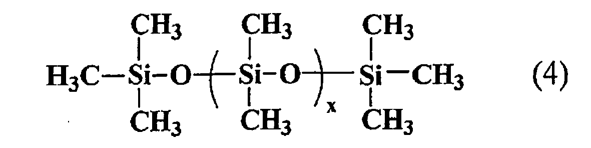 où x est un nombre entier de 0 à 3 ; 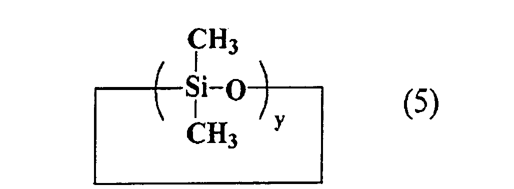 où y est un nombre entier de 3 à 6 ; 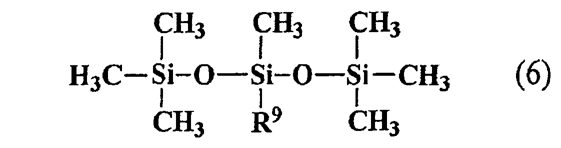 où R⁹ est un groupe hydrocarbure ayant de 2 à 8 atomes de carbone.

4. Composition cosmétique pour rouge à lèvres selon la revendication 2, dans laquelle ladite composition cosmétique pour rouge à lèvres comprend en outre un ou plusieurs composants non volatiles choisis parmi une diméthylsilicone à modification fluor représentée par la formule (7), une phénylsilicone à modification fluor représentée par la formule (8), une silicone à modification alcoxy représentée par la formule (9), une silicone à modification alkyle représentée par la formule (10), le tri(colophane hydrogéné-acide isostéarique)glycéryle, et le paraméthoxycinnamate de 2-éthylhexyle 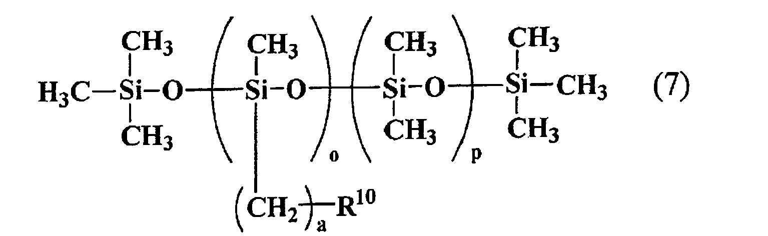 où o et p sont des valeurs moyennes, o est un nombre entier de 1 à 150, p est un nombre entier de 0 à 150, la somme de o et p vaut 4 ou plus, a est un nombre entier de 0 à 10, et R¹⁰ est un groupe perfluoroalkyle ayant de 1 à 12 atomes de carbone ; 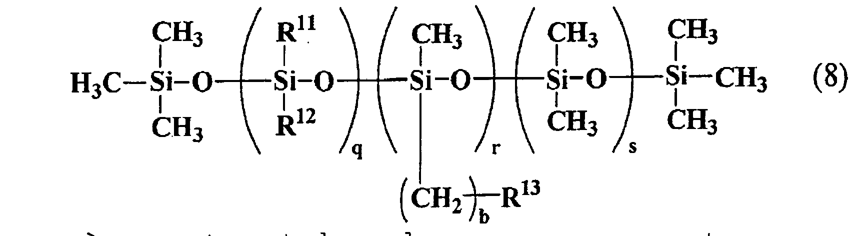 où q, r et sont des valeurs moyennes, q est un nombre entier de 1 à 150, r est un nombre entier de 1 à 150, s est un nombre entier de 0 à 150, la somme de q, r et s vaut 4 ou plus, b est un nombre entier de 0 à 10, R¹¹ et R¹² est un groupe méthyle ou phényle, un de R¹¹ et R¹² étant un groupe phényle, et R¹³ est un groupe perfluoroalkyle ayant de 1 à 12 atomes de carbone ; 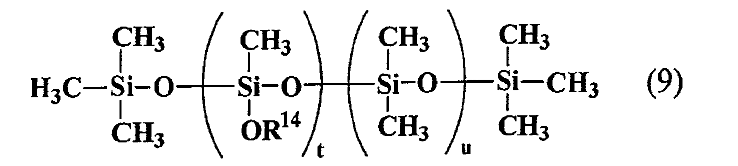 où OR¹⁴ est un groupe alcoxy ayant de 2 à 30 atomes de carbone, t et u sont des valeurs moyennes, t est un nombre entier de 1 à 500, u est un nombre entier de 0 à 500, et la somme de t et u vaut 4 ou plus ; 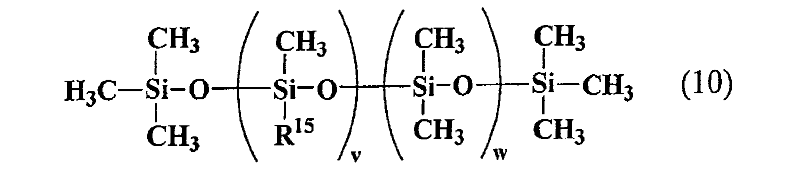 où R¹⁵ est un groupe alkyle ayant de 10 à 30 atomes de carbone, v et w sont des valeurs moyennes, v est un nombre entier de 1 à 500, w est un nombre entier de 0 à 500, et la somme de v et w vaut 4 ou plus.

5. Composition cosmétique pour rouge à lèvres selon la revendication 2, dans laquelle ladite composition cosmétique pour rouge à lèvres comprend en outre de l'eau ou un humectant.

6. Composition cosmétique selon la revendication 1, dans laquelle ladite composition cosmétique est une composition cosmétique de maquillage des cils.

7. Composition cosmétique de maquillage des cils selon la revendication 6, dans laquelle ladite composition cosmétique de maquillage des cils est une composition cosmétique de maquillage des cils de type eau-dans-l'huile comprenant le copolymère et un ingrédient huileux dans la phase externe et l'eau dans la phase interne.

8. Composition cosmétique de maquillage des cils de type eau-dans-l'huile selon la revendication 7, dans laquelle ladite composition cosmétique de maquillage des cils de type eau-dans-l'huile comprend de 1 à 30 % de copolymère et une huile silicone volatile et/ou une huile hydrocarbonée dans la phase externe, et de l'eau et une résine d'émulsion filmogène dans la phase interne.

9. Composition cosmétique de maquillage des cils selon la revendication 6, dans laquelle ladite composition cosmétique de maquillage des cils est une composition cosmétique de maquillage des cils de type huile-dans-l'eau comprenant le copolymère et un ingrédient huileux dans la phase interne et l'eau dans la phase externe.

10. Composition cosmétique de maquillage des cils de type huile-dans-l'eau selon la revendication 9, dans laquelle ladite composition cosmétique pour cils de type huile-dans-l'eau comprend de 1 à 30 % de copolymère et une huile silicone volatile et/ou une huile hydrocarbonée dans la phase interne, et de l'eau et une résine d'émulsion filmogène dans la phase externe.

11. Composition cosmétique de maquillage des cils selon la revendication 6, dans laquelle ladite composition de maquillage des cils est une composition cosmétique pour cils à base d'huile comprenant le copolymère et une cire.

12. Composition cosmétique de maquillage des cils à base d'huile selon la revendication 11, dans laquelle ladite composition cosmétique de maquillage des cils à base d'huile comprend de 1 à 30 % de copolymère, une cire, une huile silicone volatile et/ou une huile hydrocarbonée et un agent améliorant la viscosité.

13. Composition cosmétique de maquillage des cils à base d'huile selon la revendication 11, dans laquelle ladite composition cosmétique de maquillage des cils à base d'huile comprend en outre des particules creuses.

14. Composition cosmétique selon la revendication 1, dans laquelle ladite composition cosmétique est une composition cosmétique de maquillage de la peau.

15. Composition cosmétique de maquillage de la peau selon la revendication 14, dans laquelle ladite composition cosmétique de maquillage de la peau comprend en outre un polysaccharide siliconé représenté par la formule (11) ; 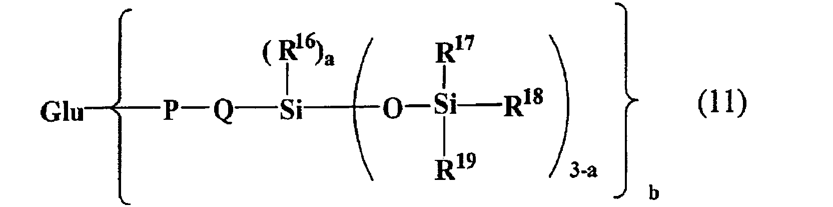 où Glu est un résidu sucre du polysaccharide, P est un groupe liant divalent, Q représente un groupe aliphatique divalent, R¹⁶ est un groupe hydrocarboné ayant de 1 à 8 atomes de carbone, et R¹⁷, R¹⁸ et R¹⁹ sont des groupes hydrocarbonés ayant de 1 à 8 atomes de carbone ou des groupes siloxy représentés par - OSiR²⁰R²¹R²², où R²⁰, R²¹ et R²² sont des groupes hydrocarbonés ayant de 1 à 8 atomes de carbone, a est un nombre entier de 0 à 2, et b est un nombre entier positif.

16. Composition cosmétique de maquillage de la peau selon la revendication 15, dans laquelle ledit polysaccharide siliconé est un pullulane siliconé représenté par la formule (12) 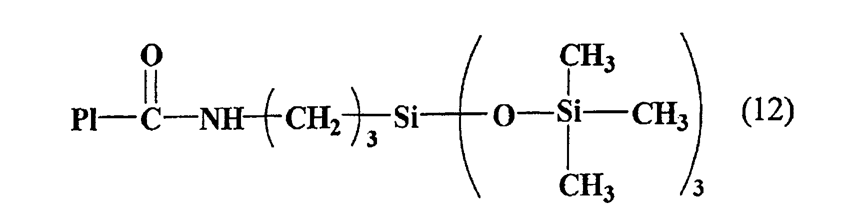 où Pl est un résidu glucose du pullulane.

17. Composition cosmétique selon la revendication 1, dans laquelle la composition cosmétique est une émulsion cosmétique eau-dans-l'huile.

18. Emulsion cosmétique eau-dans-l'huile selon la revendication 17, dans laquelle ladite émulsion cosmétique eau-dans-l'huile comprend en outre une argile minérale gonflant dans l'eau et un tensioactif cationique de type ammonium quaternaire.

19. Emulsion cosmétique eau dans l'huile selon la revendication 17, dans laquelle ladite émulsion cosmétique eau-dans-l'huile comprend en outre une poudre.

20. Emulsion cosmétique eau-dans-l'huile selon la revendication 17, dans laquelle ladite émulsion cosmétique eau-dans-l'huile comprend en outre un tensioactif non ionique.

21. Emulsion cosmétique eau-dans-l'huile selon la revendication 17, dans laquelle ladite émulsion cosmétique eau-dans-l'huile comprend en outre une silicone volatile.

22. Emulsion cosmétique eau-dans-l'huile selon la revendication 17, dans laquelle ladite émulsion cosmétique eau-dans-l'huile comprend en outre un agent d'absorption d'UV et/ou un agent de diffusion d'UV.
